# EUROPEAN PATENT APPLICATION

(11) **EP 3 346 001 A1**
(43) Date of publication of application: **11.07.2018**
(21) Application number: 17150579.5
(22) Date of filing: 06.01.2017
(51) Int. Cl.: C12N 5/0783, A61K 35/17, C07K 16/30

(54) **MONOSPECIFIC REGULATORY T CELL POPULATION WITH CYTOTOXICITY FOR B CELLS**

(71) Applicant: TXCell, 06560 Valbonne (FR)
(72) Inventor: ZHOU, Li, West Roxbury, MA 02132 (US); MEYER, François, 2900 Porrentruy (CH)
(74) Representative: Icosa

(57) **Abstract**

The present invention relates to a monospecific population of Treg cells, wherein the Treg cells comprise a chimeric receptor, wherein said chimeric receptor recognizes a B cell surface marker on a B cell, and wherein said Treg cells are cytotoxic for B cells. The present invention also relates to methods for obtaining said monospecific Treg cell population and to the use of said monospecific Treg cell population, in particular for treating an autoimmune disease.

SEQUENCE LISTING

<110> TXCELL
      ZHOU LI

<120> MONOSPECIFIC REGULATORY T CELL POPULATION WITH CYTOTOXICITY FOR B CELLS

<130> CV - 732/EP

<160> 87

<170> BiSSAP 1.3.6

<210> 1
<211> 4
<212> PRT
<213> Artificial Sequence

<220>
<223> Linker

<400> 1
Gly Gly Gly Ser
1

<210> 2
<211> 20
<212> PRT
<213> Artificial Sequence

<220>
<223> Linker

<400> 2
Gly Gly Gly Gly Ser Gly Gly Gly Gly Ser Gly Gly Gly Gly Ser Gly
1               5                   10                  15
Gly Gly Gly Ser
            20

<210> 3
<211> 15
<212> PRT
<213> Artificial Sequence

<220>
<223> Linker

<400> 3
Gly Gly Gly Gly Ser Gly Gly Gly Gly Ser Gly Gly Gly Gly Ser
1               5                   10                  15

<210> 4
<211> 10
<212> PRT
<213> Artificial Sequence

<220>
<223> Linker

<400> 4
Gly Gly Gly Gly Ser Gly Gly Gly Gly Ser
1               5                   10

<210> 5
<211> 5
<212> PRT
<213> Artificial Sequence

<220>
<223> Linker

<400> 5
Gly Gly Gly Gly Ser
1               5

<210> 6
<211> 16
<212> PRT
<213> Artificial Sequence

<220>
<223> Linker

<400> 6
Gly Gly Gly Ser Gly Gly Gly Ser Gly Gly Gly Ser Gly Gly Gly Ser
1               5                   10                  15

<210> 7
<211> 16
<212> PRT
<213> Artificial Sequence

<220>
<223> Linker

<400> 7
Ala Gly Ser Ser Ser Ser Gly Gly Ser Thr Thr Gly Gly Ser Thr Thr
1               5                   10                  15

<210> 8
<211> 16
<212> PRT
<213> Artificial Sequence

<220>
<223> Linker

<400> 8
Gly Thr Thr Ala Ala Ser Gly Ser Ser Gly Gly Ser Ser Ser Gly Ala
1               5                   10                  15

<210> 9

<211> 16
<212> PRT
<213> Artificial Sequence

<220>
<223> Linker

<400> 9
Ser Ser Ala Thr Ala Thr Ala Gly Thr Gly Ser Ser Thr Gly Ser Thr
1               5                   10                  15

<210> 10
<211> 16
<212> PRT
<213> Artificial Sequence

<220>
<223> Linker

<400> 10
Thr Ser Gly Ser Thr Gly Thr Ala Ala Ser Ser Thr Ser Thr Ser Thr
1               5                   10                  15

<210> 11
<211> 30
<212> DNA
<213> Artificial Sequence

<220>
<223> Linker

<400> 11
ggtggcggag gttctggagg tggaggttcc                                        30

<210> 12
<211> 7
<212> PRT
<213> Artificial Sequence

<220>
<223> Hinge KIR2DS2

<400> 12
Lys Ile Arg Arg Asp Ser Ser
1               5

<210> 13
<211> 45
<212> PRT
<213> Artificial Sequence

<220>
<223> CD8 hinge

<400> 13
Thr Thr Thr Pro Ala Pro Arg Pro Pro Thr Pro Ala Pro Thr Ile Ala
1               5                   10                  15
Ser Gln Pro Leu Ser Leu Arg Pro Glu Ala Cys Arg Pro Ala Ala Gly
            20                  25                  30
Gly Ala Val His Thr Arg Gly Leu Asp Phe Ala Cys Asp
        35                  40                  45

<210> 14
<211> 135
<212> DNA
<213> Artificial Sequence

<220>
<223> CD8 hinge

<400> 14
accacgacgc cagcgccgcg accaccaaca ccggcgccca ccatcgcgtc gcagcccctg      60

tccctgcgcc cagaggcgtg ccggccagcg gcggggggcg cagtgcacac gagggggctg     120

gacttcgcct gtgat                                                      135

<210> 15
<211> 230
<212> PRT
<213> Artificial Sequence

<220>
<223> IgG4 hinge

<400> 15
Glu Ser Lys Tyr Gly Pro Pro Cys Pro Pro Cys Pro Ala Pro Glu Phe
1               5                   10                  15
Leu Gly Gly Pro Ser Val Phe Leu Phe Pro Pro Lys Pro Lys Asp Thr
            20                  25                  30
Leu Met Ile Ser Arg Thr Pro Glu Val Thr Cys Val Val Val Asp Val
        35                  40                  45
Ser Gln Glu Asp Pro Glu Val Gln Phe Asn Trp Tyr Val Asp Gly Val
    50                  55                  60
Glu Val His Asn Ala Lys Thr Lys Pro Arg Glu Glu Gln Phe Asn Ser
65                  70                  75                  80
Thr Tyr Arg Val Val Ser Val Leu Thr Val Leu His Gln Asp Trp Leu
                85                  90                  95
Asn Gly Lys Glu Tyr Lys Cys Lys Val Ser Asn Lys Gly Leu Pro Ser
            100                 105                 110
Ser Ile Glu Lys Thr Ile Ser Lys Ala Lys Gly Gln Pro Arg Glu Pro
        115                 120                 125
Gln Val Tyr Thr Leu Pro Pro Ser Gln Glu Glu Met Thr Lys Asn Gln
    130                 135                 140
Val Ser Leu Thr Cys Leu Val Lys Gly Phe Tyr Pro Ser Asp Ile Ala
145                 150                 155                 160
Val Glu Trp Glu Ser Asn Gly Gln Pro Glu Asn Asn Tyr Lys Thr Thr
                165                 170                 175
Pro Pro Val Leu Asp Ser Asp Gly Ser Phe Phe Leu Tyr Ser Arg Leu
            180                 185                 190
Thr Val Asp Lys Ser Arg Trp Gln Glu Gly Asn Val Phe Ser Cys Ser
        195                 200                 205
Val Met His Glu Ala Leu His Asn His Tyr Thr Gln Lys Ser Leu Ser

210                 215                 220
Leu Ser Leu Gly Lys Met
225                 230

<210> 16
<211> 690
<212> DNA
<213> Artificial Sequence

<220>
<223> IgG4 hinge

<400> 16
gagagcaagt acggccctcc ctgcccccct tgccctgccc ccgagttcct gggcggaccc      60

agcgtgttcc tgttcccccc caagcccaag gacaccctga tgatcagccg gacccccgag     120

gtgacctgtg tggtggtgga cgtgtcccag gaggaccccg aggtccagtt caactggtac     180

gtggacggcg tggaggtgca caacgccaag accaagcccc gggaggagca gttcaatagc     240

acctaccggg tggtgtccgt gctgaccgtg ctgcaccagg actggctgaa cggcaaggaa     300

tacaagtgta aggtgtccaa caagggcctg cccagcagca tcgagaaaac catcagcaag     360

gccaagggcc agcctcggga gccccaggtg tacaccctgc cccctagcca agaggagatg     420

accaagaacc aggtgtccct gacctgcctg gtgaagggct tctaccccag cgacatcgcc     480

gtggagtggg agagcaacgg ccagcccgag aacaactaca agaccacccc ccctgtgctg     540

gacagcgacg gcagcttctt cctgtacagc cggctgaccg tggacaagag ccggtggcag     600

gagggcaacg tctttagctg ctccgtgatg cacgaggccc tgcacaacca ctacacccag     660

aagagcctga gcctgtccct gggcaagatg                                      690

<210> 17
<211> 282
<212> PRT
<213> Artificial Sequence

<220>
<223> IgD hinge

<400> 17
Arg Trp Pro Glu Ser Pro Lys Ala Gln Ala Ser Ser Val Pro Thr Ala
1               5                   10                  15
Gln Pro Gln Ala Glu Gly Ser Leu Ala Lys Ala Thr Thr Ala Pro Ala
            20                  25                  30
Thr Thr Arg Asn Thr Gly Arg Gly Gly Glu Glu Lys Lys Lys Glu Lys
        35                  40                  45
Glu Lys Glu Glu Gln Glu Glu Arg Glu Thr Lys Thr Pro Glu Cys Pro
    50                  55                  60
Ser His Thr Gln Pro Leu Gly Val Tyr Leu Leu Thr Pro Ala Val Gln
65                  70                  75                  80
Asp Leu Trp Leu Arg Asp Lys Ala Thr Phe Thr Cys Phe Val Val Gly
                85                  90                  95
Ser Asp Leu Lys Asp Ala His Leu Thr Trp Glu Val Ala Gly Lys Val
            100                 105                 110

Pro Thr Gly Gly Val Glu Glu Gly Leu Leu Glu Arg His Ser Asn Gly
        115                 120                 125
Ser Gln Ser Gln His Ser Arg Leu Thr Leu Pro Arg Ser Leu Trp Asn
    130                 135                 140
Ala Gly Thr Ser Val Thr Cys Thr Leu Asn His Pro Ser Leu Pro Pro
145                 150                 155                 160
Gln Arg Leu Met Ala Leu Arg Glu Pro Ala Ala Gln Ala Pro Val Lys
                165                 170                 175
Leu Ser Leu Asn Leu Leu Ala Ser Ser Asp Pro Pro Glu Ala Ala Ser
            180                 185                 190
Trp Leu Leu Cys Glu Val Ser Gly Phe Ser Pro Pro Asn Ile Leu Leu
        195                 200                 205
Met Trp Leu Glu Asp Gln Arg Glu Val Asn Thr Ser Gly Phe Ala Pro
    210                 215                 220
Ala Arg Pro Pro Pro Gln Pro Gly Ser Thr Thr Phe Trp Ala Trp Ser
225                 230                 235                 240
Val Leu Arg Val Pro Ala Pro Pro Ser Pro Gln Pro Ala Thr Tyr Thr
                245                 250                 255
Cys Val Val Ser His Glu Asp Ser Arg Thr Leu Leu Asn Ala Ser Arg
            260                 265                 270
Ser Leu Glu Val Ser Tyr Val Thr Asp His
        275                 280

<210> 18
<211> 847
<212> DNA
<213> Artificial Sequence

<220>
<223> IgD hinge

<400> 18
aggtggcccg aaagtcccaa ggcccaggca tctagtgttc ctactgcaca gccccaggca      60

gaaggcagcc tagccaaagc tactactgca cctgccacta cgcgcaatac tggccgtggc     120

ggggaggaga agaaaaagga gaaagagaaa gaagaacagg aagagaggga gaccaagacc     180

cctgaatgtc catcccatac ccagccgctg ggcgtctatc tcttgactcc cgcagtacag     240

gacttgtggc ttagagataa ggccaccttt acatgtttcg tcgtgggctc tgacctgaag     300

gatgcccatt tgacttggga ggttgccgga aaggtaccca cagggggggt tgaggaaggg      360

ttgctggagc gccattccaa tggctctcag agccagcact caagactcac ccttccgaga     420

tccctgtgga acgccgggac ctctgtcaca tgtactctaa atcatcctag cctgccccca     480

cagcgtctga tggcccttag agagccagcc gcccaggcac cagttaagct tagcctgaat     540

ctgctcgcca gtagtgatcc cccagaggcc gccagctggc tcttatgcga agtgtccggc     600

tttagcccgc ccaacatctt gctcatgtgg ctggaggacc agcgagaagt gaacaccagc     660

ggcttcgctc cagcccggcc cccaccccag ccgggttcta ccacattctg ggcctggagt     720

gtcttaaggg tcccagcacc acctagcccc cagccagcca catacacctg tgttgtgtcc     780

catgaagata gcaggaccct gctaaatgct tctaggagtc tggaggtttc ctacgtgact     840

gaccatt                                                               847

<210> 19
<211> 39
<212> PRT
<213> Artificial Sequence

<220>
<223> CD28 hinge

<400> 19
Ile Glu Val Met Tyr Pro Pro Pro Tyr Leu Asp Asn Glu Lys Ser Asn
1               5                   10                  15
Gly Thr Ile Ile His Val Lys Gly Lys His Leu Cys Pro Ser Pro Leu
            20                  25                  30
Phe Pro Gly Pro Ser Lys Pro
        35

<210> 20
<211> 117
<212> DNA
<213> Artificial Sequence

<220>
<223> CD28 hinge

<400> 20
attgaagtta tgtatcctcc tccttaccta gacaatgaga agagcaatgg aaccattatc      60

catgtgaaag ggaaacacct ttgtccaagt cccctatttc ccggaccttc taagccc        117

<210> 21
<211> 24
<212> PRT
<213> Artificial Sequence

<220>
<223> CD8 transmembrane

<400> 21
Ile Tyr Ile Trp Ala Pro Leu Ala Gly Thr Cys Gly Val Leu Leu Leu
1               5                   10                  15
Ser Leu Val Ile Thr Leu Tyr Cys
            20

<210> 22
<211> 72
<212> DNA
<213> Artificial Sequence

<220>
<223> CD8 transmembrane

<400> 22
atctacatct gggcgccctt ggccgggact tgtggggtcc ttctcctgtc actggttatc      60

accctttact gc                                                          72

<210> 23
<211> 27
<212> PRT
<213> Artificial Sequence

<220>
<223> CD28 transmembrane

<400> 23
Phe Trp Val Leu Val Val Val Gly Gly Val Leu Ala Cys Tyr Ser Leu
1               5                   10                  15
Leu Val Thr Val Ala Phe Ile Ile Phe Trp Val
            20                  25

<210> 24
<211> 81
<212> DNA
<213> Artificial Sequence

<220>
<223> CD28 transmembrane

<400> 24
ttttgggtgc tggtggtggt tggtggagtc ctggcttgct atagcttgct agtaacagtg      60

gcctttatta ttttctgggt g                                                81

<210> 25
<211> 112
<212> PRT
<213> Artificial Sequence

<220>
<223> CD3 zeta

<400> 25
Arg Val Lys Phe Ser Arg Ser Ala Asp Ala Pro Ala Tyr Lys Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Arg Arg Lys Asn Pro Gln Glu Gly Leu Tyr Asn Glu Leu Gln Lys
    50                  55                  60
Asp Lys Met Ala Glu Ala Tyr Ser Glu Ile Gly Met Lys Gly Glu Arg
65                  70                  75                  80
Arg Arg Gly Lys Gly His Asp Gly Leu Tyr Gln Gly Leu Ser Thr Ala
                85                  90                  95
Thr Lys Asp Thr Tyr Asp Ala Leu His Met Gln Ala Leu Pro Pro Arg
            100                 105                 110

<210> 26
<211> 112
<212> PRT
<213> Artificial Sequence

<220>
<223> CD3 zeta

<400> 26
Arg Val Lys Phe Ser Arg Ser Ala Asp Ala Pro Ala Tyr Gln Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Arg Arg Lys Asn Pro Gln Glu Gly Leu Tyr Asn Glu Leu Gln Lys
    50                  55                  60
Asp Lys Met Ala Glu Ala Tyr Ser Glu Ile Gly Met Lys Gly Glu Arg
65                  70                  75                  80
Arg Arg Gly Lys Gly His Asp Gly Leu Tyr Gln Gly Leu Ser Thr Ala
                85                  90                  95
Thr Lys Asp Thr Tyr Asp Ala Leu His Met Gln Ala Leu Pro Pro Arg
            100                 105                 110

<210> 27
<211> 336
<212> DNA
<213> Artificial Sequence

<220>
<223> CD3 zeta

<400> 27
agagtgaagt tcagcaggag cgcagacgcc cccgcgtaca agcagggcca gaaccagctc      60

tataacgagc tcaatctagg acgaagagag gagtacgatg ttttggacaa gagacgtggc     120

cgggaccctg agatgggggg aaagccgaga aggaagaacc ctcaggaagg cctgtacaat     180

gaactgcaga aagataagat ggcggaggcc tacagtgaga ttgggatgaa aggcgagcgc     240

cggaggggca aggggcacga tggcctttac cagggtctca gtacagccac caaggacacc     300

tacgacgccc ttcacatgca ggccctgccc cctcgc                               336

<210> 28
<211> 336
<212> DNA
<213> Artificial Sequence

<220>
<223> CD3 zeta

<400> 28
agagtgaagt tcagcaggag cgcagacgcc cccgcgtacc agcagggcca gaaccagctc      60

tataacgagc tcaatctagg acgaagagag gagtacgatg ttttggacaa gagacgtggc     120

cgggaccctg agatgggggg aaagccgaga aggaagaacc ctcaggaagg cctgtacaat     180

gaactgcaga aagataagat ggcggaggcc tacagtgaga ttgggatgaa aggcgagcgc     240

cggaggggca aggggcacga tggcctttac cagggtctca gtacagccac caaggacacc     300

tacgacgccc ttcacatgca ggccctgccc cctcgc                                336

<210> 29
<211> 42
<212> PRT
<213> Artificial Sequence

<220>
<223> 4-1BB intracellular

<400> 29
Lys Arg Gly Arg Lys Lys Leu Leu Tyr Ile Phe Lys Gln Pro Phe Met
1               5                   10                  15
Arg Pro Val Gln Thr Thr Gln Glu Glu Asp Gly Cys Ser Cys Arg Phe
            20                  25                  30
Pro Glu Glu Glu Glu Gly Gly Cys Glu Leu
        35                  40

<210> 30
<211> 48
<212> PRT
<213> Artificial Sequence

<220>
<223> CD27 intracellular

<400> 30
Gln Arg Arg Lys Tyr Arg Ser Asn Lys Gly Glu Ser Pro Val Glu Pro
1               5                   10                  15
Ala Glu Pro Cys Arg Tyr Ser Cys Pro Arg Glu Glu Glu Gly Ser Thr
            20                  25                  30
Ile Pro Ile Gln Glu Asp Tyr Arg Lys Pro Glu Pro Ala Cys Ser Pro
        35                  40                  45

<210> 31
<211> 41
<212> PRT
<213> Artificial Sequence

<220>
<223> CD28 intracellular

<400> 31
Arg Ser Lys Arg Ser Arg Leu Leu His Ser Asp Tyr Met Asn Met Thr
1               5                   10                  15
Pro Arg Arg Pro Gly Pro Thr Arg Lys His Tyr Gln Pro Tyr Ala Pro
            20                  25                  30
Pro Arg Asp Phe Ala Ala Tyr Arg Ser
        35                  40

<210> 32
<211> 126
<212> DNA
<213> Artificial Sequence

<220>
<223> 4-1BB intracellular

<400> 32
aaacggggca gaaagaaact cctgtatata ttcaaacaac catttatgag accagtacaa      60

actactcaag aggaagatgg ctgtagctgc cgatttccag aagaagaaga aggaggatgt     120

gaactg                                                                126


<210> 33
<211> 144
<212> DNA
<213> Artificial Sequence


<220>
<223> CD27 intracellular

<400> 33
caacgaagga aatatagatc aaacaaagga gaaagtcctg tggagcctgc agagccttgt      60

cgttacagct gccccaggga ggaggagggc agcaccatcc ccatccagga ggattaccga     120

aaaccggagc ctgcctgctc cccc                                            144


<210> 34
<211> 123
<212> DNA
<213> Artificial Sequence


<220>
<223> CD28 intracellular

<400> 34
aggagtaaga ggagcaggct cctgcacagt gactacatga acatgactcc ccgccgcccc      60

gggcccaccc gcaagcatta ccagccctat gccccaccac gcgacttcgc agcctatcgc     120

tcc                                                                   123


<210> 35
<211> 222
<212> PRT
<213> Artificial Sequence


<220>
<223> Chimeric receptor

<400> 35
Ala Ala Ala Ile Glu Val Met Tyr Pro Pro Pro Tyr Leu Asp Asn Glu
1               5                   10                  15
Lys Ser Asn Gly Thr Ile Ile His Val Lys Gly Lys His Leu Cys Pro
            20                  25                  30
Ser Pro Leu Phe Pro Gly Pro Ser Lys Pro Phe Trp Val Leu Val Val
        35                  40                  45
Val Gly Gly Val Leu Ala Cys Tyr Ser Leu Leu Val Thr Val Ala Phe
    50                  55                  60

Ile Ile Phe Trp Val Arg Ser Lys Arg Ser Arg Leu Leu His Ser Asp
65                  70                  75                  80
Tyr Met Asn Met Thr Pro Arg Arg Pro Gly Pro Thr Arg Lys His Tyr
                85                  90                  95
Gln Pro Tyr Ala Pro Pro Arg Asp Phe Ala Ala Tyr Arg Ser Arg Val
            100                 105                 110
Lys Phe Ser Arg Ser Ala Asp Ala Pro Ala Tyr Gln Gln Gly Gln Asn
        115                 120                 125
Gln Leu Tyr Asn Glu Leu Asn Leu Gly Arg Arg Glu Glu Tyr Asp Val
    130                 135                 140
Leu Asp Lys Arg Arg Gly Arg Asp Pro Glu Met Gly Gly Lys Pro Arg
145                 150                 155                 160
Arg Lys Asn Pro Gln Glu Gly Leu Tyr Asn Glu Leu Gln Lys Asp Lys
                165                 170                 175
Met Ala Glu Ala Tyr Ser Glu Ile Gly Met Lys Gly Glu Arg Arg Arg
            180                 185                 190
Gly Lys Gly His Asp Gly Leu Tyr Gln Gly Leu Ser Thr Ala Thr Lys
        195                 200                 205
Asp Thr Tyr Asp Ala Leu His Met Gln Ala Leu Pro Pro Arg
    210                 215                 220

<210> 36
<211> 726
<212> DNA
<213> Artificial Sequence

<220>
<223> CD19 scFv

<400> 36
gacatccaga tgacacagac tacatcctcc ctgtctgcct ctctgggaga cagagtcacc      60

atcagttgca gggcaagtca ggacattagt aaatatttaa attggtatca gcagaaacca     120

gatggaactg ttaaactcct gatctaccat acatcaagat tacactcagg agtcccatca     180

aggttcagtg gcagtgggtc tggaacagat tattctctca ccattagcaa cctggagcaa     240

gaagatattg ccacttactt ttgccaacag ggtaatacgc ttccgtacac gttcggaggg     300

gggaccaagc tggagatcac aggtggcggt ggctcgggcg gtggtgggtc gggtggcggc     360

ggatctgagg tgaaactgca ggagtcagga cctggcctgg tggcgccctc acagagcctg     420

tccgtcacat gcactgtctc aggggtctca ttacccgact atggtgtaag ctggattcgc     480

cagcctccac gaaagggtct ggagtggctg ggagtaatat ggggtagtga aaccacatac     540

tataattcag ctctcaaatc cagactgacc atcatcaagg acaactccaa gagccaagtt     600

ttcttaaaaa tgaacagtct gcaaactgat gacacagcca tttactactg tgccaaacat     660

tattactacg gtggtagcta tgctatggac tactggggcc aaggaacctc agtcaccgtc     720

tcctca                                                                726

<210> 37
<211> 242
<212> PRT
<213> Artificial Sequence

<220>
<223> CD19 scFv

<400> 37
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Gly Gly Gly Ser
            100                 105                 110
Gly Gly Gly Gly Ser Gly Gly Gly Gly Ser Glu Val Lys Leu Gln Glu
        115                 120                 125
Ser Gly Pro Gly Leu Val Ala Pro Ser Gln Ser Leu Ser Val Thr Cys
    130                 135                 140
Thr Val Ser Gly Val Ser Leu Pro Asp Tyr Gly Val Ser Trp Ile Arg
145                 150                 155                 160
Gln Pro Pro Arg Lys Gly Leu Glu Trp Leu Gly Val Ile Trp Gly Ser
                165                 170                 175
Glu Thr Thr Tyr Tyr Asn Ser Ala Leu Lys Ser Arg Leu Thr Ile Ile
            180                 185                 190
Lys Asp Asn Ser Lys Ser Gln Val Phe Leu Lys Met Asn Ser Leu Gln
        195                 200                 205
Thr Asp Asp Thr Ala Ile Tyr Tyr Cys Ala Lys His Tyr Tyr Tyr Gly
    210                 215                 220
Gly Ser Tyr Ala Met Asp Tyr Trp Gly Gln Gly Thr Ser Val Thr Val
225                 230                 235                 240
Ser Ser

<210> 38
<211> 245
<212> PRT
<213> Artificial Sequence

<220>
<223> CD19 scFv

<400> 38
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys

115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Ile
                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn
        195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser
                245

<210> 39
<211> 21
<212> PRT
<213> Artificial Sequence

<220>
<223> CD8 leader sequence

<400> 39
Met Ala Leu Pro Val Thr Ala Leu Leu Leu Pro Leu Ala Leu Leu Leu
1               5                   10                  15
His Ala Ala Arg Pro
            20

<210> 40
<211> 489
<212> PRT
<213> Artificial Sequence

<220>
<223> Chimeric receptor

<400> 40
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Ser Thr Ser Gly Ser Gly Lys Pro Gly Ser Gly Glu Gly Ser Thr
    130                 135                 140
Lys Gly Glu Val Lys Leu Gln Glu Ser Gly Pro Gly Leu Val Ala Pro
145                 150                 155                 160

Ser Gln Ser Leu Ser Val Thr Cys Thr Val Ser Gly Val Ser Leu Pro
                165                 170                 175
Asp Tyr Gly Val Ser Trp Ile Arg Gln Pro Pro Arg Lys Gly Leu Glu
            180                 185                 190
Trp Leu Gly Val Ile Trp Gly Ser Glu Thr Thr Tyr Tyr Asn Ser Ala
        195                 200                 205
Leu Lys Ser Arg Leu Thr Ile Ile Lys Asp Asn Ser Lys Ser Gln Val
    210                 215                 220
Phe Leu Lys Met Asn Ser Leu Gln Thr Asp Asp Thr Ala Ile Tyr Tyr
225                 230                 235                 240
Cys Ala Lys His Tyr Tyr Tyr Gly Gly Ser Tyr Ala Met Asp Tyr Trp
                245                 250                 255
Gly Gln Gly Thr Ser Val Thr Val Ser Ser Thr Thr Thr Pro Ala Pro
            260                 265                 270
Arg Pro Pro Thr Pro Ala Pro Thr Ile Ala Ser Gln Pro Leu Ser Leu
        275                 280                 285
Arg Pro Glu Ala Cys Arg Pro Ala Ala Gly Gly Ala Val His Thr Arg
    290                 295                 300
Gly Leu Asp Phe Ala Cys Asp Ile Tyr Ile Trp Ala Pro Leu Ala Gly
305                 310                 315                 320
Thr Cys Gly Val Leu Leu Leu Ser Leu Val Ile Thr Leu Tyr Cys Lys
                325                 330                 335
Arg Gly Arg Lys Lys Leu Leu Tyr Ile Phe Lys Gln Pro Phe Met Arg
            340                 345                 350
Pro Val Gln Thr Thr Gln Glu Glu Asp Gly Cys Ser Cys Arg Phe Pro
        355                 360                 365
Glu Glu Glu Glu Gly Gly Cys Glu Leu Arg Val Lys Phe Ser Arg Ser
    370                 375                 380
Ala Asp Ala Pro Ala Tyr Gln Gln Gly Gln Asn Gln Leu Tyr Asn Glu
385                 390                 395                 400
Leu Asn Leu Gly Arg Arg Glu Glu Tyr Asp Val Leu Asp Lys Arg Arg
                405                 410                 415
Gly Arg Asp Pro Glu Met Gly Gly Lys Pro Arg Arg Lys Asn Pro Gln
            420                 425                 430
Glu Gly Leu Tyr Asn Glu Leu Gln Lys Asp Lys Met Ala Glu Ala Tyr
        435                 440                 445
Ser Glu Ile Gly Met Lys Gly Glu Arg Arg Arg Gly Lys Gly His Asp
    450                 455                 460
Gly Leu Tyr Gln Gly Leu Ser Thr Ala Thr Lys Asp Thr Tyr Asp Ala
465                 470                 475                 480
Leu His Met Gln Ala Leu Pro Pro Arg
                485

<210> 41
<211> 468
<212> PRT
<213> Artificial Sequence

<220>
<223> Chimeric receptor

<400> 41
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys
        115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Ile
                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn
        195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser Thr Thr Thr Pro Ala Pro Arg Pro Pro Thr Pro
                245                 250                 255
Ala Pro Thr Ile Ala Ser Gln Pro Leu Ser Leu Arg Pro Glu Ala Cys
            260                 265                 270
Arg Pro Ala Ala Gly Gly Ala Val His Thr Arg Gly Leu Asp Phe Ala
        275                 280                 285
Cys Asp Ile Tyr Ile Trp Ala Pro Leu Ala Gly Thr Cys Gly Val Leu
    290                 295                 300
Leu Leu Ser Leu Val Ile Thr Leu Tyr Cys Lys Arg Gly Arg Lys Lys
305                 310                 315                 320
Leu Leu Tyr Ile Phe Lys Gln Pro Phe Met Arg Pro Val Gln Thr Thr
                325                 330                 335
Gln Glu Glu Asp Gly Cys Ser Cys Arg Phe Pro Glu Glu Glu Glu Gly
            340                 345                 350
Gly Cys Glu Leu Arg Val Lys Phe Ser Arg Ser Ala Asp Ala Pro Ala
        355                 360                 365
Tyr Gln Gln Gly Gln Asn Gln Leu Tyr Asn Glu Leu Asn Leu Gly Arg
    370                 375                 380
Arg Glu Glu Tyr Asp Val Leu Asp Lys Arg Arg Gly Arg Asp Pro Glu
385                 390                 395                 400
Met Gly Gly Lys Pro Arg Arg Lys Asn Pro Gln Glu Gly Leu Tyr Asn
                405                 410                 415
Glu Leu Gln Lys Asp Lys Met Ala Glu Ala Tyr Ser Glu Ile Gly Met
            420                 425                 430
Lys Gly Glu Arg Arg Arg Gly Lys Gly His Asp Gly Leu Tyr Gln Gly
        435                 440                 445
Leu Ser Thr Ala Thr Lys Asp Thr Tyr Asp Ala Leu His Met Gln Ala
    450                 455                 460
Leu Pro Pro Arg
465

<210> 42
<211> 502
<212> PRT
<213> Artificial Sequence

<220>
<223> Chimeric receptor

<400> 42
Met Ala Leu Pro Val Thr Ala Leu Leu Leu Pro Leu Ala Leu Leu Leu
1               5                   10                  15

His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Ser Thr Ser Gly Ser Gly Lys Pro Gly Ser Gly Glu Gly Ser Thr
    130                 135                 140
Lys Gly Glu Val Lys Leu Gln Glu Ser Gly Pro Gly Leu Val Ala Pro
145                 150                 155                 160
Ser Gln Ser Leu Ser Val Thr Cys Thr Val Ser Gly Val Ser Leu Pro
                165                 170                 175
Asp Tyr Gly Val Ser Trp Ile Arg Gln Pro Pro Arg Lys Gly Leu Glu
            180                 185                 190
Trp Leu Gly Val Ile Trp Gly Ser Glu Thr Thr Tyr Tyr Asn Ser Ala
        195                 200                 205
Leu Lys Ser Arg Leu Thr Ile Ile Lys Asp Asn Ser Lys Ser Gln Val
    210                 215                 220
Phe Leu Lys Met Asn Ser Leu Gln Thr Asp Asp Thr Ala Ile Tyr Tyr
225                 230                 235                 240
Cys Ala Lys His Tyr Tyr Tyr Gly Gly Ser Tyr Ala Met Asp Tyr Trp
                245                 250                 255
Gly Gln Gly Thr Ser Val Thr Val Ser Ser Asn Trp Ser His Pro Gln
            260                 265                 270
Phe Glu Lys Met His Thr Thr Thr Pro Ala Pro Arg Pro Pro Thr Pro
        275                 280                 285
Ala Pro Thr Ile Ala Ser Gln Pro Leu Ser Leu Arg Pro Glu Ala Cys
    290                 295                 300
Arg Pro Ala Ala Gly Gly Ala Val His Thr Arg Gly Leu Asp Phe Ala
305                 310                 315                 320
Cys Asp Ile Tyr Ile Trp Ala Pro Leu Ala Gly Thr Cys Gly Val Leu
                325                 330                 335
Leu Leu Ser Leu Val Ile Thr Leu Tyr Cys Lys Arg Gly Arg Lys Lys
            340                 345                 350
Leu Leu Tyr Ile Phe Lys Gln Pro Phe Met Arg Pro Val Gln Thr Thr
        355                 360                 365
Gln Glu Glu Asp Gly Cys Ser Cys Arg Phe Pro Glu Glu Glu Glu Gly
    370                 375                 380
Gly Cys Glu Leu Thr Arg Arg Val Lys Phe Ser Arg Ser Ala Asp Ala
385                 390                 395                 400
Pro Ala Tyr Gln Gln Gly Gln Asn Gln Leu Tyr Asn Glu Leu Asn Leu
                405                 410                 415
Gly Arg Arg Glu Glu Tyr Asp Val Leu Asp Lys Arg Arg Gly Arg Asp
            420                 425                 430
Pro Glu Met Gly Gly Lys Pro Arg Arg Lys Asn Pro Gln Glu Gly Leu
        435                 440                 445
Tyr Asn Glu Leu Gln Lys Asp Lys Met Ala Glu Ala Tyr Ser Glu Ile
    450                 455                 460
Gly Met Lys Gly Glu Arg Arg Arg Gly Lys Gly His Asp Gly Leu Tyr
465                 470                 475                 480
Gln Gly Leu Ser Thr Ala Thr Lys Asp Thr Tyr Asp Ala Leu His Met
                485                 490                 495
Gln Ala Leu Pro Pro Arg
            500

<210> 43
<211> 481
<212> PRT
<213> Artificial Sequence

<220>
<223> Chimeric receptor

<400> 43
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys
        115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Ile
                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn
        195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser Asn Trp Ser His Pro Gln Phe Glu Lys Met His
                245                 250                 255
Thr Thr Thr Pro Ala Pro Arg Pro Pro Thr Pro Ala Pro Thr Ile Ala
            260                 265                 270
Ser Gln Pro Leu Ser Leu Arg Pro Glu Ala Cys Arg Pro Ala Ala Gly
        275                 280                 285
Gly Ala Val His Thr Arg Gly Leu Asp Phe Ala Cys Asp Ile Tyr Ile
    290                 295                 300
Trp Ala Pro Leu Ala Gly Thr Cys Gly Val Leu Leu Leu Ser Leu Val
305                 310                 315                 320
Ile Thr Leu Tyr Cys Lys Arg Gly Arg Lys Lys Leu Leu Tyr Ile Phe
                325                 330                 335
Lys Gln Pro Phe Met Arg Pro Val Gln Thr Thr Gln Glu Glu Asp Gly
            340                 345                 350
Cys Ser Cys Arg Phe Pro Glu Glu Glu Glu Gly Gly Cys Glu Leu Thr
        355                 360                 365
Arg Arg Val Lys Phe Ser Arg Ser Ala Asp Ala Pro Ala Tyr Gln Gln
    370                 375                 380
Gly Gln Asn Gln Leu Tyr Asn Glu Leu Asn Leu Gly Arg Arg Glu Glu
385                 390                 395                 400
Tyr Asp Val Leu Asp Lys Arg Arg Gly Arg Asp Pro Glu Met Gly Gly
                405                 410                 415
Lys Pro Arg Arg Lys Asn Pro Gln Glu Gly Leu Tyr Asn Glu Leu Gln

                420                 425                 430
Lys Asp Lys Met Ala Glu Ala Tyr Ser Glu Ile Gly Met Lys Gly Glu
        435                 440                 445
Arg Arg Arg Gly Lys Gly His Asp Gly Leu Tyr Gln Gly Leu Ser Thr
    450                 455                 460
Ala Thr Lys Asp Thr Tyr Asp Ala Leu His Met Gln Ala Leu Pro Pro
465                 470                 475                 480
Arg

<210> 44
<211> 519
<212> PRT
<213> Artificial Sequence

<220>
<223> Chimeric receptor

<400> 44
Met Ala Leu Pro Val Thr Ala Leu Leu Leu Pro Leu Ala Leu Leu Leu
1               5                   10                  15
His Ala Ala Arg Pro Asp Ile Gln Leu Thr Gln Ser Pro Ala Ile Leu
            20                  25                  30
Ser Ala Ser Pro Gly Glu Lys Val Thr Met Thr Cys Arg Ala Ser Ser
        35                  40                  45
Ser Leu Ser Phe Met His Trp Tyr Gln Gln Lys Pro Gly Ser Ser Pro
    50                  55                  60
Lys Pro Trp Ile Tyr Ala Thr Ser Asn Leu Ala Ser Gly Val Pro Ala
65                  70                  75                  80
Arg Phe Ser Gly Ser Gly Ser Gly Thr Ser Tyr Ser Leu Thr Ile Ser
                85                  90                  95
Thr Val Glu Ala Glu Asp Ala Ala Ser Tyr Phe Cys His Gln Trp Ser
            100                 105                 110
Ser Asn Pro Leu Thr Phe Gly Ala Gly Thr Lys Leu Glu Ile Ser Ser
        115                 120                 125
Gly Gly Gly Gly Ser Gly Gly Gly Gly Ser Gly Asp Val Met Gly Val
    130                 135                 140
Asp Ser Gly Gly Gly Leu Val Gln Pro Gly Gly Ser Arg Lys Leu Ser
145                 150                 155                 160
Cys Ala Ala Pro Gly Phe Thr Phe Ser Ser Phe Gly Met His Trp Val
                165                 170                 175
Arg Gln Ala Pro Glu Lys Gly Leu Glu Trp Val Ala Tyr Ile Ser Ser
            180                 185                 190
Pro Ser Ser Thr Leu His Tyr Ala Asp Arg Val Lys Gly Arg Phe Thr
        195                 200                 205
Ile Ser Arg Asp Asn Pro Lys Asn Thr Leu Phe Leu Gln Met Lys Leu
    210                 215                 220
Pro Ser Leu Cys Tyr Gly Leu Leu Gly Pro Arg Asp His Val His Arg
225                 230                 235                 240
Leu Leu Lys His His His His His His Leu Glu Tyr Pro Tyr Asp Val
                245                 250                 255
Pro Asp Tyr Ala Thr Thr Asp Ala Ala His Pro Gly Arg Ser Val Val
            260                 265                 270
Pro Ala Leu Leu Pro Leu Leu Ala Gly Thr Leu Leu Leu Leu Glu Thr
        275                 280                 285
Ala Thr Ala Pro Met His Thr Thr Thr Pro Ala Pro Arg Pro Pro Thr
    290                 295                 300
Pro Ala Pro Thr Ile Ala Ser Gln Pro Leu Ser Leu Arg Pro Glu Ala
305                 310                 315                 320
Cys Arg Pro Ala Ala Gly Gly Ala Val His Thr Arg Gly Leu Asp Phe
                325                 330                 335
Ala Cys Asp Ile Tyr Ile Trp Ala Pro Leu Ala Gly Thr Cys Gly Val

340                 345                 350
Leu Leu Leu Ser Leu Val Ile Thr Leu Tyr Cys Lys Arg Gly Arg Lys
        355                 360                 365
Lys Leu Leu Tyr Ile Phe Lys Gln Pro Phe Met Arg Pro Val Gln Thr
    370                 375                 380
Thr Gln Glu Glu Asp Gly Cys Ser Cys Arg Phe Pro Glu Glu Glu Glu
385                 390                 395                 400
Gly Gly Cys Glu Leu Thr Arg Arg Val Lys Phe Ser Arg Ser Ala Asp
                405                 410                 415
Ala Pro Ala Tyr Gln Gln Gly Gln Asn Gln Leu Tyr Asn Glu Leu Asn
            420                 425                 430
Leu Gly Arg Arg Glu Glu Tyr Asp Val Leu Asp Lys Arg Arg Gly Arg
        435                 440                 445
Asp Pro Glu Met Gly Gly Lys Pro Arg Arg Lys Asn Pro Gln Glu Gly
    450                 455                 460
Leu Tyr Asn Glu Leu Gln Lys Asp Lys Met Ala Glu Ala Tyr Ser Glu
465                 470                 475                 480
Ile Gly Met Lys Gly Glu Arg Arg Arg Gly Lys Gly His Asp Gly Leu
                485                 490                 495
Tyr Gln Gly Leu Ser Thr Ala Thr Lys Asp Thr Tyr Asp Ala Leu His
            500                 505                 510
Met Gln Ala Leu Pro Pro Arg
        515

<210> 45
<211> 498
<212> PRT
<213> Artificial Sequence

<220>
<223> Chimeric receptor

<400> 45
Asp Ile Gln Leu Thr Gln Ser Pro Ala Ile Leu Ser Ala Ser Pro Gly
1               5                   10                  15
Glu Lys Val Thr Met Thr Cys Arg Ala Ser Ser Ser Leu Ser Phe Met
            20                  25                  30
His Trp Tyr Gln Gln Lys Pro Gly Ser Ser Pro Lys Pro Trp Ile Tyr
        35                  40                  45
Ala Thr Ser Asn Leu Ala Ser Gly Val Pro Ala Arg Phe Ser Gly Ser
    50                  55                  60
Gly Ser Gly Thr Ser Tyr Ser Leu Thr Ile Ser Thr Val Glu Ala Glu
65                  70                  75                  80
Asp Ala Ala Ser Tyr Phe Cys His Gln Trp Ser Ser Asn Pro Leu Thr
                85                  90                  95
Phe Gly Ala Gly Thr Lys Leu Glu Ile Ser Ser Gly Gly Gly Gly Ser
            100                 105                 110
Gly Gly Gly Gly Ser Gly Asp Val Met Gly Val Asp Ser Gly Gly Gly
        115                 120                 125
Leu Val Gln Pro Gly Gly Ser Arg Lys Leu Ser Cys Ala Ala Pro Gly
    130                 135                 140
Phe Thr Phe Ser Ser Phe Gly Met His Trp Val Arg Gln Ala Pro Glu
145                 150                 155                 160
Lys Gly Leu Glu Trp Val Ala Tyr Ile Ser Ser Pro Ser Ser Thr Leu
                165                 170                 175
His Tyr Ala Asp Arg Val Lys Gly Arg Phe Thr Ile Ser Arg Asp Asn
            180                 185                 190
Pro Lys Asn Thr Leu Phe Leu Gln Met Lys Leu Pro Ser Leu Cys Tyr
        195                 200                 205
Gly Leu Leu Gly Pro Arg Asp His Val His Arg Leu Leu Lys His His
    210                 215                 220
His His His His Leu Glu Tyr Pro Tyr Asp Val Pro Asp Tyr Ala Thr

225                 230                 235                 240
Thr Asp Ala Ala His Pro Gly Arg Ser Val Val Pro Ala Leu Leu Pro
                245                 250                 255
Leu Leu Ala Gly Thr Leu Leu Leu Leu Glu Thr Ala Thr Ala Pro Met
            260                 265                 270
His Thr Thr Thr Pro Ala Pro Arg Pro Pro Thr Pro Ala Pro Thr Ile
        275                 280                 285
Ala Ser Gln Pro Leu Ser Leu Arg Pro Glu Ala Cys Arg Pro Ala Ala
    290                 295                 300
Gly Gly Ala Val His Thr Arg Gly Leu Asp Phe Ala Cys Asp Ile Tyr
305                 310                 315                 320
Ile Trp Ala Pro Leu Ala Gly Thr Cys Gly Val Leu Leu Leu Ser Leu
                325                 330                 335
Val Ile Thr Leu Tyr Cys Lys Arg Gly Arg Lys Lys Leu Leu Tyr Ile
            340                 345                 350
Phe Lys Gln Pro Phe Met Arg Pro Val Gln Thr Thr Gln Glu Glu Asp
        355                 360                 365
Gly Cys Ser Cys Arg Phe Pro Glu Glu Glu Glu Gly Gly Cys Glu Leu
    370                 375                 380
Thr Arg Arg Val Lys Phe Ser Arg Ser Ala Asp Ala Pro Ala Tyr Gln
385                 390                 395                 400
Gln Gly Gln Asn Gln Leu Tyr Asn Glu Leu Asn Leu Gly Arg Arg Glu
                405                 410                 415
Glu Tyr Asp Val Leu Asp Lys Arg Arg Gly Arg Asp Pro Glu Met Gly
            420                 425                 430
Gly Lys Pro Arg Arg Lys Asn Pro Gln Glu Gly Leu Tyr Asn Glu Leu
        435                 440                 445
Gln Lys Asp Lys Met Ala Glu Ala Tyr Ser Glu Ile Gly Met Lys Gly
    450                 455                 460
Glu Arg Arg Arg Gly Lys Gly His Asp Gly Leu Tyr Gln Gly Leu Ser
465                 470                 475                 480
Thr Ala Thr Lys Asp Thr Tyr Asp Ala Leu His Met Gln Ala Leu Pro
                485                 490                 495
Pro Arg


<210> 46
<211> 271
<212> PRT
<213> Artificial Sequence


<220>
<223> CD20 scFv

<400> 46
Asp Ile Gln Leu Thr Gln Ser Pro Ala Ile Leu Ser Ala Ser Pro Gly
1               5                   10                  15
Glu Lys Val Thr Met Thr Cys Arg Ala Ser Ser Ser Leu Ser Phe Met
            20                  25                  30
His Trp Tyr Gln Gln Lys Pro Gly Ser Ser Pro Lys Pro Trp Ile Tyr
        35                  40                  45
Ala Thr Ser Asn Leu Ala Ser Gly Val Pro Ala Arg Phe Ser Gly Ser
    50                  55                  60
Gly Ser Gly Thr Ser Tyr Ser Leu Thr Ile Ser Thr Val Glu Ala Glu
65                  70                  75                  80
Asp Ala Ala Ser Tyr Phe Cys His Gln Trp Ser Ser Asn Pro Leu Thr
                85                  90                  95
Phe Gly Ala Gly Thr Lys Leu Glu Ile Ser Ser Gly Gly Gly Gly Ser
            100                 105                 110
Gly Gly Gly Gly Ser Gly Asp Val Met Gly Val Asp Ser Gly Gly Gly
        115                 120                 125
Leu Val Gln Pro Gly Gly Ser Arg Lys Leu Ser Cys Ala Ala Pro Gly

130                 135                 140
Phe Thr Phe Ser Ser Phe Gly Met His Trp Val Arg Gln Ala Pro Glu
145                 150                 155                 160
Lys Gly Leu Glu Trp Val Ala Tyr Ile Ser Ser Pro Ser Ser Thr Leu
                165                 170                 175
His Tyr Ala Asp Arg Val Lys Gly Arg Phe Thr Ile Ser Arg Asp Asn
            180                 185                 190
Pro Lys Asn Thr Leu Phe Leu Gln Met Lys Leu Pro Ser Leu Cys Tyr
        195                 200                 205
Gly Leu Leu Gly Pro Arg Asp His Val His Arg Leu Leu Lys His His
    210                 215                 220
His His His His Leu Glu Tyr Pro Tyr Asp Val Pro Asp Tyr Ala Thr
225                 230                 235                 240
Thr Asp Ala Ala His Pro Gly Arg Ser Val Val Pro Ala Leu Leu Pro
                245                 250                 255
Leu Leu Ala Gly Thr Leu Leu Leu Leu Glu Thr Ala Thr Ala Pro
            260                 265                 270

<210> 47
<211> 492
<212> PRT
<213> Artificial Sequence

<220>
<223> Chimeric receptor

<400> 47
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Ser Thr Ser Gly Ser Gly Lys Pro Gly Ser Gly Glu Gly Ser Thr
    130                 135                 140
Lys Gly Glu Val Lys Leu Gln Glu Ser Gly Pro Gly Leu Val Ala Pro
145                 150                 155                 160
Ser Gln Ser Leu Ser Val Thr Cys Thr Val Ser Gly Val Ser Leu Pro
                165                 170                 175
Asp Tyr Gly Val Ser Trp Ile Arg Gln Pro Pro Arg Lys Gly Leu Glu
            180                 185                 190
Trp Leu Gly Val Ile Trp Gly Ser Glu Thr Thr Tyr Tyr Asn Ser Ala
        195                 200                 205
Leu Lys Ser Arg Leu Thr Ile Ile Lys Asp Asn Ser Lys Ser Gln Val
    210                 215                 220
Phe Leu Lys Met Asn Ser Leu Gln Thr Asp Asp Thr Ala Ile Tyr Tyr
225                 230                 235                 240
Cys Ala Lys His Tyr Tyr Tyr Gly Gly Ser Tyr Ala Met Asp Tyr Trp
                245                 250                 255
Gly Gln Gly Thr Ser Val Thr Val Ser Ser Met His Thr Thr Thr Pro
            260                 265                 270
Ala Pro Arg Pro Pro Thr Pro Ala Pro Thr Ile Ala Ser Gln Pro Leu

275                 280                 285
Ser Leu Arg Pro Glu Ala Cys Arg Pro Ala Ala Gly Gly Ala Val His
    290                 295                 300
Thr Arg Gly Leu Asp Phe Ala Cys Asp Ile Tyr Ile Trp Ala Pro Leu
305                 310                 315                 320
Ala Gly Thr Cys Gly Val Leu Leu Leu Ser Leu Val Ile Thr Leu Tyr
                325                 330                 335
Cys Arg Ser Lys Arg Ser Arg Leu Leu His Ser Asp Tyr Met Asn Met
            340                 345                 350
Thr Pro Arg Arg Pro Gly Pro Thr Arg Lys His Tyr Gln Pro Tyr Ala
        355                 360                 365
Pro Pro Arg Asp Phe Ala Ala Tyr Arg Ser Thr Arg Arg Val Lys Phe
    370                 375                 380
Ser Arg Ser Ala Asp Ala Pro Ala Tyr Gln Gln Gly Gln Asn Gln Leu
385                 390                 395                 400
Tyr Asn Glu Leu Asn Leu Gly Arg Arg Glu Glu Tyr Asp Val Leu Asp
                405                 410                 415
Lys Arg Arg Gly Arg Asp Pro Glu Met Gly Gly Lys Pro Arg Arg Lys
            420                 425                 430
Asn Pro Gln Glu Gly Leu Tyr Asn Glu Leu Gln Lys Asp Lys Met Ala
        435                 440                 445
Glu Ala Tyr Ser Glu Ile Gly Met Lys Gly Glu Arg Arg Arg Gly Lys
    450                 455                 460
Gly His Asp Gly Leu Tyr Gln Gly Leu Ser Thr Ala Thr Lys Asp Thr
465                 470                 475                 480
Tyr Asp Ala Leu His Met Gln Ala Leu Pro Pro Arg
                485                 490

<210> 48
<211> 471
<212> PRT
<213> Artificial Sequence

<220>
<223> Chimeric receptor

<400> 48
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys
        115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Ile
                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn

195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser Met His Thr Thr Thr Pro Ala Pro Arg Pro Pro
                245                 250                 255
Thr Pro Ala Pro Thr Ile Ala Ser Gln Pro Leu Ser Leu Arg Pro Glu
            260                 265                 270
Ala Cys Arg Pro Ala Ala Gly Gly Ala Val His Thr Arg Gly Leu Asp
        275                 280                 285
Phe Ala Cys Asp Ile Tyr Ile Trp Ala Pro Leu Ala Gly Thr Cys Gly
    290                 295                 300
Val Leu Leu Leu Ser Leu Val Ile Thr Leu Tyr Cys Arg Ser Lys Arg
305                 310                 315                 320
Ser Arg Leu Leu His Ser Asp Tyr Met Asn Met Thr Pro Arg Arg Pro
                325                 330                 335
Gly Pro Thr Arg Lys His Tyr Gln Pro Tyr Ala Pro Pro Arg Asp Phe
            340                 345                 350
Ala Ala Tyr Arg Ser Thr Arg Arg Val Lys Phe Ser Arg Ser Ala Asp
        355                 360                 365
Ala Pro Ala Tyr Gln Gln Gly Gln Asn Gln Leu Tyr Asn Glu Leu Asn
    370                 375                 380
Leu Gly Arg Arg Glu Glu Tyr Asp Val Leu Asp Lys Arg Arg Gly Arg
385                 390                 395                 400
Asp Pro Glu Met Gly Gly Lys Pro Arg Arg Lys Asn Pro Gln Glu Gly
                405                 410                 415
Leu Tyr Asn Glu Leu Gln Lys Asp Lys Met Ala Glu Ala Tyr Ser Glu
            420                 425                 430
Ile Gly Met Lys Gly Glu Arg Arg Arg Gly Lys Gly His Asp Gly Leu
        435                 440                 445
Tyr Gln Gly Leu Ser Thr Ala Thr Lys Asp Thr Tyr Asp Ala Leu His
    450                 455                 460
Met Gln Ala Leu Pro Pro Arg
465                 470

<210> 49
<211> 223
<212> PRT
<213> Artificial Sequence

<220>
<223> Chimeric receptor

<400> 49
Thr Thr Thr Pro Ala Pro Arg Pro Pro Thr Pro Ala Pro Thr Ile Ala
1               5                   10                  15
Ser Gln Pro Leu Ser Leu Arg Pro Glu Ala Cys Arg Pro Ala Ala Gly
            20                  25                  30
Gly Ala Val His Thr Arg Gly Leu Asp Phe Ala Cys Asp Ile Tyr Ile
        35                  40                  45
Trp Ala Pro Leu Ala Gly Thr Cys Gly Val Leu Leu Leu Ser Leu Val
    50                  55                  60
Ile Thr Leu Tyr Cys Lys Arg Gly Arg Lys Lys Leu Leu Tyr Ile Phe
65                  70                  75                  80
Lys Gln Pro Phe Met Arg Pro Val Gln Thr Thr Gln Glu Glu Asp Gly
                85                  90                  95
Cys Ser Cys Arg Phe Pro Glu Glu Glu Glu Gly Gly Cys Glu Leu Arg
            100                 105                 110
Val Lys Phe Ser Arg Ser Ala Asp Ala Pro Ala Tyr Gln Gln Gly Gln
        115                 120                 125
Asn Gln Leu Tyr Asn Glu Leu Asn Leu Gly Arg Arg Glu Glu Tyr Asp

130                 135                 140
Val Leu Asp Lys Arg Arg Gly Arg Asp Pro Glu Met Gly Gly Lys Pro
145                 150                 155                 160
Arg Arg Lys Asn Pro Gln Glu Gly Leu Tyr Asn Glu Leu Gln Lys Asp
                165                 170                 175
Lys Met Ala Glu Ala Tyr Ser Glu Ile Gly Met Lys Gly Glu Arg Arg
            180                 185                 190
Arg Gly Lys Gly His Asp Gly Leu Tyr Gln Gly Leu Ser Thr Ala Thr
        195                 200                 205
Lys Asp Thr Tyr Asp Ala Leu His Met Gln Ala Leu Pro Pro Arg
    210                 215                 220

<210> 50
<211> 236
<212> PRT
<213> Artificial Sequence

<220>
<223> Chimeric receptor

<400> 50
Asn Trp Ser His Pro Gln Phe Glu Lys Met His Thr Thr Thr Pro Ala
1               5                   10                  15
Pro Arg Pro Pro Thr Pro Ala Pro Thr Ile Ala Ser Gln Pro Leu Ser
            20                  25                  30
Leu Arg Pro Glu Ala Cys Arg Pro Ala Ala Gly Gly Ala Val His Thr
        35                  40                  45
Arg Gly Leu Asp Phe Ala Cys Asp Ile Tyr Ile Trp Ala Pro Leu Ala
    50                  55                  60
Gly Thr Cys Gly Val Leu Leu Leu Ser Leu Val Ile Thr Leu Tyr Cys
65                  70                  75                  80
Lys Arg Gly Arg Lys Lys Leu Leu Tyr Ile Phe Lys Gln Pro Phe Met
                85                  90                  95
Arg Pro Val Gln Thr Thr Gln Glu Glu Asp Gly Cys Ser Cys Arg Phe
            100                 105                 110
Pro Glu Glu Glu Glu Gly Gly Cys Glu Leu Thr Arg Arg Val Lys Phe
        115                 120                 125
Ser Arg Ser Ala Asp Ala Pro Ala Tyr Gln Gln Gly Gln Asn Gln Leu
    130                 135                 140
Tyr Asn Glu Leu Asn Leu Gly Arg Arg Glu Glu Tyr Asp Val Leu Asp
145                 150                 155                 160
Lys Arg Arg Gly Arg Asp Pro Glu Met Gly Gly Lys Pro Arg Arg Lys
                165                 170                 175
Asn Pro Gln Glu Gly Leu Tyr Asn Glu Leu Gln Lys Asp Lys Met Ala
            180                 185                 190
Glu Ala Tyr Ser Glu Ile Gly Met Lys Gly Glu Arg Arg Arg Gly Lys
        195                 200                 205
Gly His Asp Gly Leu Tyr Gln Gly Leu Ser Thr Ala Thr Lys Asp Thr
    210                 215                 220
Tyr Asp Ala Leu His Met Gln Ala Leu Pro Pro Arg
225                 230                 235

<210> 51
<211> 226
<212> PRT
<213> Artificial Sequence

<220>
<223> Chimeric receptor

<400> 51

Met His Thr Thr Thr Pro Ala Pro Arg Pro Pro Thr Pro Ala Pro Thr
1               5                   10                  15
Ile Ala Ser Gln Pro Leu Ser Leu Arg Pro Glu Ala Cys Arg Pro Ala
            20                  25                  30
Ala Gly Gly Ala Val His Thr Arg Gly Leu Asp Phe Ala Cys Asp Ile
        35                  40                  45
Tyr Ile Trp Ala Pro Leu Ala Gly Thr Cys Gly Val Leu Leu Leu Ser
    50                  55                  60
Leu Val Ile Thr Leu Tyr Cys Arg Ser Lys Arg Ser Arg Leu Leu His
65                  70                  75                  80
Ser Asp Tyr Met Asn Met Thr Pro Arg Arg Pro Gly Pro Thr Arg Lys
                85                  90                  95
His Tyr Gln Pro Tyr Ala Pro Pro Arg Asp Phe Ala Ala Tyr Arg Ser
            100                 105                 110
Thr Arg Arg Val Lys Phe Ser Arg Ser Ala Asp Ala Pro Ala Tyr Gln
        115                 120                 125
Gln Gly Gln Asn Gln Leu Tyr Asn Glu Leu Asn Leu Gly Arg Arg Glu
    130                 135                 140
Glu Tyr Asp Val Leu Asp Lys Arg Arg Gly Arg Asp Pro Glu Met Gly
145                 150                 155                 160
Gly Lys Pro Arg Arg Lys Asn Pro Gln Glu Gly Leu Tyr Asn Glu Leu
                165                 170                 175
Gln Lys Asp Lys Met Ala Glu Ala Tyr Ser Glu Ile Gly Met Lys Gly
            180                 185                 190
Glu Arg Arg Arg Gly Lys Gly His Asp Gly Leu Tyr Gln Gly Leu Ser
        195                 200                 205
Thr Ala Thr Lys Asp Thr Tyr Asp Ala Leu His Met Gln Ala Leu Pro
    210                 215                 220
Pro Arg
225

<210> 52
<211> 501
<212> PRT
<213> Artificial Sequence

<220>
<223> Chimeric receptor

<400> 52
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Ser Thr Ser Gly Ser Gly Lys Pro Gly Ser Gly Glu Gly Ser Thr
    130                 135                 140
Lys Gly Glu Val Lys Leu Gln Glu Ser Gly Pro Gly Leu Val Ala Pro
145                 150                 155                 160
Ser Gln Ser Leu Ser Val Thr Cys Thr Val Ser Gly Val Ser Leu Pro
                165                 170                 175

Asp Tyr Gly Val Ser Trp Ile Arg Gln Pro Pro Arg Lys Gly Leu Glu
            180                 185                 190
Trp Leu Gly Val Ile Trp Gly Ser Glu Thr Thr Tyr Tyr Asn Ser Ala
        195                 200                 205
Leu Lys Ser Arg Leu Thr Ile Ile Lys Asp Asn Ser Lys Ser Gln Val
    210                 215                 220
Phe Leu Lys Met Asn Ser Leu Gln Thr Asp Asp Thr Ala Ile Tyr Tyr
225                 230                 235                 240
Cys Ala Lys His Tyr Tyr Tyr Gly Gly Ser Tyr Ala Met Asp Tyr Trp
                245                 250                 255
Gly Gln Gly Thr Ser Val Thr Val Ser Ser Asn Trp Ser His Pro Gln
            260                 265                 270
Phe Glu Lys Met His Thr Thr Thr Pro Ala Pro Arg Pro Pro Thr Pro
        275                 280                 285
Ala Pro Thr Ile Ala Ser Gln Pro Leu Ser Leu Arg Pro Glu Ala Cys
    290                 295                 300
Arg Pro Ala Ala Gly Gly Ala Val His Thr Arg Gly Leu Asp Phe Ala
305                 310                 315                 320
Cys Asp Ile Tyr Ile Trp Ala Pro Leu Ala Gly Thr Cys Gly Val Leu
                325                 330                 335
Leu Leu Ser Leu Val Ile Thr Leu Tyr Cys Arg Ser Lys Arg Ser Arg
            340                 345                 350
Leu Leu His Ser Asp Tyr Met Asn Met Thr Pro Arg Arg Pro Gly Pro
        355                 360                 365
Thr Arg Lys His Tyr Gln Pro Tyr Ala Pro Pro Arg Asp Phe Ala Ala
    370                 375                 380
Tyr Arg Ser Thr Arg Arg Val Lys Phe Ser Arg Ser Ala Asp Ala Pro
385                 390                 395                 400
Ala Tyr Gln Gln Gly Gln Asn Gln Leu Tyr Asn Glu Leu Asn Leu Gly
                405                 410                 415
Arg Arg Glu Glu Tyr Asp Val Leu Asp Lys Arg Arg Gly Arg Asp Pro
            420                 425                 430
Glu Met Gly Gly Lys Pro Arg Arg Lys Asn Pro Gln Glu Gly Leu Tyr
        435                 440                 445
Asn Glu Leu Gln Lys Asp Lys Met Ala Glu Ala Tyr Ser Glu Ile Gly
    450                 455                 460
Met Lys Gly Glu Arg Arg Arg Gly Lys Gly His Asp Gly Leu Tyr Gln
465                 470                 475                 480
Gly Leu Ser Thr Ala Thr Lys Asp Thr Tyr Asp Ala Leu His Met Gln
                485                 490                 495
Ala Leu Pro Pro Arg
            500

<210> 53
<211> 480
<212> PRT
<213> Artificial Sequence

<220>
<223> Chimeric receptor

<400> 53
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys
        115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Ile
                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn
        195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser Asn Trp Ser His Pro Gln Phe Glu Lys Met His
                245                 250                 255
Thr Thr Thr Pro Ala Pro Arg Pro Pro Thr Pro Ala Pro Thr Ile Ala
            260                 265                 270
Ser Gln Pro Leu Ser Leu Arg Pro Glu Ala Cys Arg Pro Ala Ala Gly
        275                 280                 285
Gly Ala Val His Thr Arg Gly Leu Asp Phe Ala Cys Asp Ile Tyr Ile
    290                 295                 300
Trp Ala Pro Leu Ala Gly Thr Cys Gly Val Leu Leu Leu Ser Leu Val
305                 310                 315                 320
Ile Thr Leu Tyr Cys Arg Ser Lys Arg Ser Arg Leu Leu His Ser Asp
                325                 330                 335
Tyr Met Asn Met Thr Pro Arg Arg Pro Gly Pro Thr Arg Lys His Tyr
            340                 345                 350
Gln Pro Tyr Ala Pro Pro Arg Asp Phe Ala Ala Tyr Arg Ser Thr Arg
        355                 360                 365
Arg Val Lys Phe Ser Arg Ser Ala Asp Ala Pro Ala Tyr Gln Gln Gly
    370                 375                 380
Gln Asn Gln Leu Tyr Asn Glu Leu Asn Leu Gly Arg Arg Glu Glu Tyr
385                 390                 395                 400
Asp Val Leu Asp Lys Arg Arg Gly Arg Asp Pro Glu Met Gly Gly Lys
                405                 410                 415
Pro Arg Arg Lys Asn Pro Gln Glu Gly Leu Tyr Asn Glu Leu Gln Lys
            420                 425                 430
Asp Lys Met Ala Glu Ala Tyr Ser Glu Ile Gly Met Lys Gly Glu Arg
        435                 440                 445
Arg Arg Gly Lys Gly His Asp Gly Leu Tyr Gln Gly Leu Ser Thr Ala
    450                 455                 460
Thr Lys Asp Thr Tyr Asp Ala Leu His Met Gln Ala Leu Pro Pro Arg
465                 470                 475                 480

<210> 54
<211> 504
<212> PRT
<213> Artificial Sequence

<220>
<223> Chimeric receptor

<400> 54

Met Ala Leu Pro Val Thr Ala Leu Leu Leu Pro Leu Ala Leu Leu Leu
1               5                   10                  15
His Ala Ala Arg Pro Ser Gln Val Gln Leu Gln Gln Pro Gly Ala Glu
            20                  25                  30
Leu Val Lys Pro Gly Ala Ser Val Lys Met Ser Cys Lys Ala Ser Gly
        35                  40                  45
Tyr Thr Phe Thr Ser Tyr Asn Met His Trp Val Lys Gln Thr Pro Gly
    50                  55                  60
Arg Gly Leu Glu Trp Ile Gly Ala Ile Tyr Pro Gly Asn Gly Asp Thr
65                  70                  75                  80
Ser Tyr Asn Gln Lys Phe Lys Gly Lys Ala Thr Leu Thr Ala Asp Lys
                85                  90                  95
Ser Ser Ser Thr Ala Tyr Met Gln Leu Ser Ser Leu Thr Ser Glu Asp
            100                 105                 110
Ser Ala Val Tyr Tyr Cys Ala Arg Ser Thr Tyr Tyr Gly Gly Asp Trp
        115                 120                 125
Tyr Phe Asn Val Trp Gly Ala Gly Thr Thr Val Thr Val Ser Ser Gly
    130                 135                 140
Gly Gly Gly Ser Gly Gly Gly Gly Ser Gly Gly Gly Gly Ser Gln Ile
145                 150                 155                 160
Val Leu Ser Gln Ser Pro Ala Ile Leu Ser Ala Ser Pro Gly Glu Lys
                165                 170                 175
Val Thr Met Thr Cys Arg Ala Ser Ser Ser Val Ser Tyr Ile His Trp
            180                 185                 190
Phe Gln Gln Lys Pro Gly Ser Ser Pro Lys Pro Trp Ile Tyr Ala Thr
        195                 200                 205
Ser Asn Leu Ala Ser Gly Val Pro Val Arg Phe Ser Gly Ser Gly Ser
    210                 215                 220
Gly Thr Ser Tyr Ser Leu Thr Ile Ser Arg Val Glu Ala Glu Asp Ala
225                 230                 235                 240
Ala Thr Tyr Tyr Cys Gln Gln Trp Thr Ser Asn Pro Pro Thr Phe Gly
                245                 250                 255
Gly Gly Thr Lys Leu Glu Ile Lys Arg Ser Ala Trp Ser His Pro Gln
            260                 265                 270
Phe Glu Lys Ser Gly Met His Thr Thr Thr Pro Ala Pro Arg Pro Pro
        275                 280                 285
Thr Pro Ala Pro Thr Ile Ala Ser Gln Pro Leu Ser Leu Arg Pro Glu
    290                 295                 300
Ala Cys Arg Pro Ala Ala Gly Gly Ala Val His Thr Arg Gly Leu Asp
305                 310                 315                 320
Phe Ala Cys Asp Ile Tyr Ile Trp Ala Pro Leu Ala Gly Thr Cys Gly
                325                 330                 335
Val Leu Leu Leu Ser Leu Val Ile Thr Leu Tyr Cys Lys Arg Gly Arg
            340                 345                 350
Lys Lys Leu Leu Tyr Ile Phe Lys Gln Pro Phe Met Arg Pro Val Gln
        355                 360                 365
Thr Thr Gln Glu Glu Asp Gly Cys Ser Cys Arg Phe Pro Glu Glu Glu
    370                 375                 380
Glu Gly Gly Cys Glu Leu Thr Arg Arg Val Lys Phe Ser Arg Ser Ala
385                 390                 395                 400
Asp Ala Pro Ala Tyr Gln Gln Gly Gln Asn Gln Leu Tyr Asn Glu Leu
                405                 410                 415
Asn Leu Gly Arg Arg Glu Glu Tyr Asp Val Leu Asp Lys Arg Arg Gly
            420                 425                 430
Arg Asp Pro Glu Met Gly Gly Lys Pro Arg Arg Lys Asn Pro Gln Glu
        435                 440                 445
Gly Leu Tyr Asn Glu Leu Gln Lys Asp Lys Met Ala Glu Ala Tyr Ser
    450                 455                 460
Glu Ile Gly Met Lys Gly Glu Arg Arg Arg Gly Lys Gly His Asp Gly
465                 470                 475                 480
Leu Tyr Gln Gly Leu Ser Thr Ala Thr Lys Asp Thr Tyr Asp Ala Leu
                485                 490                 495
His Met Gln Ala Leu Pro Pro Arg

            500

<210> 55
<211> 483
<212> PRT
<213> Artificial Sequence

<220>
<223> Chimeric receptor

<400> 55
Ser Gln Val Gln Leu Gln Gln Pro Gly Ala Glu Leu Val Lys Pro Gly
1               5                   10                  15
Ala Ser Val Lys Met Ser Cys Lys Ala Ser Gly Tyr Thr Phe Thr Ser
            20                  25                  30
Tyr Asn Met His Trp Val Lys Gln Thr Pro Gly Arg Gly Leu Glu Trp
        35                  40                  45
Ile Gly Ala Ile Tyr Pro Gly Asn Gly Asp Thr Ser Tyr Asn Gln Lys
    50                  55                  60
Phe Lys Gly Lys Ala Thr Leu Thr Ala Asp Lys Ser Ser Ser Thr Ala
65                  70                  75                  80
Tyr Met Gln Leu Ser Ser Leu Thr Ser Glu Asp Ser Ala Val Tyr Tyr
                85                  90                  95
Cys Ala Arg Ser Thr Tyr Tyr Gly Gly Asp Trp Tyr Phe Asn Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Gln Ile Val Leu Ser Gln Ser
    130                 135                 140
Pro Ala Ile Leu Ser Ala Ser Pro Gly Glu Lys Val Thr Met Thr Cys
145                 150                 155                 160
Arg Ala Ser Ser Ser Val Ser Tyr Ile His Trp Phe Gln Gln Lys Pro
                165                 170                 175
Gly Ser Ser Pro Lys Pro Trp Ile Tyr Ala Thr Ser Asn Leu Ala Ser
            180                 185                 190
Gly Val Pro Val Arg Phe Ser Gly Ser Gly Ser Gly Thr Ser Tyr Ser
        195                 200                 205
Leu Thr Ile Ser Arg Val Glu Ala Glu Asp Ala Ala Thr Tyr Tyr Cys
    210                 215                 220
Gln Gln Trp Thr Ser Asn Pro Pro Thr Phe Gly Gly Gly Thr Lys Leu
225                 230                 235                 240
Glu Ile Lys Arg Ser Ala Trp Ser His Pro Gln Phe Glu Lys Ser Gly
                245                 250                 255
Met His Thr Thr Thr Pro Ala Pro Arg Pro Pro Thr Pro Ala Pro Thr
            260                 265                 270
Ile Ala Ser Gln Pro Leu Ser Leu Arg Pro Glu Ala Cys Arg Pro Ala
        275                 280                 285
Ala Gly Gly Ala Val His Thr Arg Gly Leu Asp Phe Ala Cys Asp Ile
    290                 295                 300
Tyr Ile Trp Ala Pro Leu Ala Gly Thr Cys Gly Val Leu Leu Leu Ser
305                 310                 315                 320
Leu Val Ile Thr Leu Tyr Cys Lys Arg Gly Arg Lys Lys Leu Leu Tyr
                325                 330                 335
Ile Phe Lys Gln Pro Phe Met Arg Pro Val Gln Thr Thr Gln Glu Glu
            340                 345                 350
Asp Gly Cys Ser Cys Arg Phe Pro Glu Glu Glu Glu Gly Gly Cys Glu
        355                 360                 365
Leu Thr Arg Arg Val Lys Phe Ser Arg Ser Ala Asp Ala Pro Ala Tyr
    370                 375                 380
Gln Gln Gly Gln Asn Gln Leu Tyr Asn Glu Leu Asn Leu Gly Arg Arg
385                 390                 395                 400
Glu Glu Tyr Asp Val Leu Asp Lys Arg Arg Gly Arg Asp Pro Glu Met

405                 410                 415
Gly Gly Lys Pro Arg Arg Lys Asn Pro Gln Glu Gly Leu Tyr Asn Glu
            420                 425                 430
Leu Gln Lys Asp Lys Met Ala Glu Ala Tyr Ser Glu Ile Gly Met Lys
        435                 440                 445
Gly Glu Arg Arg Arg Gly Lys Gly His Asp Gly Leu Tyr Gln Gly Leu
    450                 455                 460
Ser Thr Ala Thr Lys Asp Thr Tyr Asp Ala Leu His Met Gln Ala Leu
465                 470                 475                 480
Pro Pro Arg

<210> 56
<211> 244
<212> PRT
<213> Artificial Sequence

<220>
<223> CD20 scFv

<400> 56
Ser Gln Val Gln Leu Gln Gln Pro Gly Ala Glu Leu Val Lys Pro Gly
1               5                   10                  15
Ala Ser Val Lys Met Ser Cys Lys Ala Ser Gly Tyr Thr Phe Thr Ser
            20                  25                  30
Tyr Asn Met His Trp Val Lys Gln Thr Pro Gly Arg Gly Leu Glu Trp
        35                  40                  45
Ile Gly Ala Ile Tyr Pro Gly Asn Gly Asp Thr Ser Tyr Asn Gln Lys
    50                  55                  60
Phe Lys Gly Lys Ala Thr Leu Thr Ala Asp Lys Ser Ser Ser Thr Ala
65                  70                  75                  80
Tyr Met Gln Leu Ser Ser Leu Thr Ser Glu Asp Ser Ala Val Tyr Tyr
                85                  90                  95
Cys Ala Arg Ser Thr Tyr Tyr Gly Gly Asp Trp Tyr Phe Asn Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Gln Ile Val Leu Ser Gln Ser
    130                 135                 140
Pro Ala Ile Leu Ser Ala Ser Pro Gly Glu Lys Val Thr Met Thr Cys
145                 150                 155                 160
Arg Ala Ser Ser Ser Val Ser Tyr Ile His Trp Phe Gln Gln Lys Pro
                165                 170                 175
Gly Ser Ser Pro Lys Pro Trp Ile Tyr Ala Thr Ser Asn Leu Ala Ser
            180                 185                 190
Gly Val Pro Val Arg Phe Ser Gly Ser Gly Ser Gly Thr Ser Tyr Ser
        195                 200                 205
Leu Thr Ile Ser Arg Val Glu Ala Glu Asp Ala Ala Thr Tyr Tyr Cys
    210                 215                 220
Gln Gln Trp Thr Ser Asn Pro Pro Thr Phe Gly Gly Gly Thr Lys Leu
225                 230                 235                 240
Glu Ile Lys Arg

<210> 57
<211> 509
<212> PRT
<213> Artificial Sequence

<220>
<223> Chimeric receptor

<400> 57
Met Ala Leu Pro Val Thr Ala Leu Leu Leu Pro Leu Ala Leu Leu Leu
1               5                   10                  15
His Ala Ala Arg Pro Ser Gln Val Gln Leu Val Gln Ser Gly Ala Glu
            20                  25                  30
Leu Val Lys Pro Gly Ala Ser Val Lys Met Ser Cys Lys Ala Ser Gly
        35                  40                  45
Tyr Thr Phe Thr Ser Tyr Asn Met His Trp Val Lys Gln Thr Pro Gly
    50                  55                  60
Gln Gly Leu Glu Trp Ile Gly Ala Ile Tyr Pro Gly Asn Gly Asp Thr
65                  70                  75                  80
Ser Tyr Asn Gln Lys Phe Lys Gly Lys Ala Thr Leu Thr Ala Asp Lys
                85                  90                  95
Ser Ser Ser Thr Ala Tyr Met Gln Leu Ser Ser Leu Thr Ser Glu Asp
            100                 105                 110
Ser Ala Val Tyr Tyr Cys Ala Arg Ala Gln Leu Arg Pro Asn Tyr Trp
        115                 120                 125
Tyr Phe Asp Val Trp Gly Ala Gly Thr Thr Val Thr Val Ser Lys Ile
    130                 135                 140
Ser Gly Gly Gly Gly Ser Gly Gly Gly Gly Ser Gly Gly Gly Gly Ser
145                 150                 155                 160
Gly Gly Ser Ser Asp Ile Val Leu Ser Gln Ser Pro Ala Ile Leu Ser
                165                 170                 175
Ala Ser Pro Gly Glu Lys Val Thr Met Thr Cys Arg Ala Ser Ser Ser
            180                 185                 190
Val Ser Tyr Met His Trp Tyr Gln Gln Lys Pro Gly Ser Ser Pro Lys
        195                 200                 205
Pro Trp Ile Tyr Ala Thr Ser Asn Leu Ala Ser Gly Val Pro Ala Arg
    210                 215                 220
Phe Ser Gly Ser Gly Ser Gly Thr Ser Tyr Ser Leu Thr Ile Ser Arg
225                 230                 235                 240
Val Glu Ala Glu Asp Ala Ala Thr Tyr Tyr Cys Gln Gln Trp Ile Ser
                245                 250                 255
Asn Pro Pro Thr Phe Gly Ala Gly Thr Lys Leu Glu Leu Lys Ser Ala
            260                 265                 270
Trp Ser His Pro Gln Phe Glu Lys Ser Gly Met His Thr Thr Thr Pro
        275                 280                 285
Ala Pro Arg Pro Pro Thr Pro Ala Pro Thr Ile Ala Ser Gln Pro Leu
    290                 295                 300
Ser Leu Arg Pro Glu Ala Cys Arg Pro Ala Ala Gly Gly Ala Val His
305                 310                 315                 320
Thr Arg Gly Leu Asp Phe Ala Cys Asp Ile Tyr Ile Trp Ala Pro Leu
                325                 330                 335
Ala Gly Thr Cys Gly Val Leu Leu Leu Ser Leu Val Ile Thr Leu Tyr
            340                 345                 350
Cys Lys Arg Gly Arg Lys Lys Leu Leu Tyr Ile Phe Lys Gln Pro Phe
        355                 360                 365
Met Arg Pro Val Gln Thr Thr Gln Glu Glu Asp Gly Cys Ser Cys Arg
    370                 375                 380
Phe Pro Glu Glu Glu Glu Gly Gly Cys Glu Leu Thr Arg Arg Val Lys
385                 390                 395                 400
Phe Ser Arg Ser Ala Asp Ala Pro Ala Tyr Gln Gln Gly Gln Asn Gln
                405                 410                 415
Leu Tyr Asn Glu Leu Asn Leu Gly Arg Arg Glu Glu Tyr Asp Val Leu
            420                 425                 430
Asp Lys Arg Arg Gly Arg Asp Pro Glu Met Gly Gly Lys Pro Arg Arg
        435                 440                 445
Lys Asn Pro Gln Glu Gly Leu Tyr Asn Glu Leu Gln Lys Asp Lys Met
    450                 455                 460
Ala Glu Ala Tyr Ser Glu Ile Gly Met Lys Gly Glu Arg Arg Arg Gly
465                 470                 475                 480
Lys Gly His Asp Gly Leu Tyr Gln Gly Leu Ser Thr Ala Thr Lys Asp

485                     490                     495
Thr Tyr Asp Ala Leu His Met Gln Ala Leu Pro Pro Arg
                500                 505

<210> 58
<211> 488
<212> PRT
<213> Artificial Sequence

<220>
<223> Chimeric receptor

<400> 58
Ser Gln Val Gln Leu Val Gln Ser Gly Ala Glu Leu Val Lys Pro Gly
1               5                   10                  15
Ala Ser Val Lys Met Ser Cys Lys Ala Ser Gly Tyr Thr Phe Thr Ser
            20                  25                  30
Tyr Asn Met His Trp Val Lys Gln Thr Pro Gly Gln Gly Leu Glu Trp
        35                  40                  45
Ile Gly Ala Ile Tyr Pro Gly Asn Gly Asp Thr Ser Tyr Asn Gln Lys
    50                  55                  60
Phe Lys Gly Lys Ala Thr Leu Thr Ala Asp Lys Ser Ser Ser Thr Ala
65                  70                  75                  80
Tyr Met Gln Leu Ser Ser Leu Thr Ser Glu Asp Ser Ala Val Tyr Tyr
                85                  90                  95
Cys Ala Arg Ala Gln Leu Arg Pro Asn Tyr Trp Tyr Phe Asp Val Trp
            100                 105                 110
Gly Ala Gly Thr Thr Val Thr Val Ser Lys Ile Ser Gly Gly Gly Gly
        115                 120                 125
Ser Gly Gly Gly Gly Ser Gly Gly Gly Gly Ser Gly Gly Ser Ser Asp
    130                 135                 140
Ile Val Leu Ser Gln Ser Pro Ala Ile Leu Ser Ala Ser Pro Gly Glu
145                 150                 155                 160
Lys Val Thr Met Thr Cys Arg Ala Ser Ser Ser Val Ser Tyr Met His
                165                 170                 175
Trp Tyr Gln Gln Lys Pro Gly Ser Ser Pro Lys Pro Trp Ile Tyr Ala
            180                 185                 190
Thr Ser Asn Leu Ala Ser Gly Val Pro Ala Arg Phe Ser Gly Ser Gly
        195                 200                 205
Ser Gly Thr Ser Tyr Ser Leu Thr Ile Ser Arg Val Glu Ala Glu Asp
    210                 215                 220
Ala Ala Thr Tyr Tyr Cys Gln Gln Trp Ile Ser Asn Pro Pro Thr Phe
225                 230                 235                 240
Gly Ala Gly Thr Lys Leu Glu Leu Lys Ser Ala Trp Ser His Pro Gln
                245                 250                 255
Phe Glu Lys Ser Gly Met His Thr Thr Thr Pro Ala Pro Arg Pro Pro
            260                 265                 270
Thr Pro Ala Pro Thr Ile Ala Ser Gln Pro Leu Ser Leu Arg Pro Glu
        275                 280                 285
Ala Cys Arg Pro Ala Ala Gly Gly Ala Val His Thr Arg Gly Leu Asp
    290                 295                 300
Phe Ala Cys Asp Ile Tyr Ile Trp Ala Pro Leu Ala Gly Thr Cys Gly
305                 310                 315                 320
Val Leu Leu Leu Ser Leu Val Ile Thr Leu Tyr Cys Lys Arg Gly Arg
                325                 330                 335
Lys Lys Leu Leu Tyr Ile Phe Lys Gln Pro Phe Met Arg Pro Val Gln
            340                 345                 350
Thr Thr Gln Glu Glu Asp Gly Cys Ser Cys Arg Phe Pro Glu Glu Glu
        355                 360                 365
Glu Gly Gly Cys Glu Leu Thr Arg Arg Val Lys Phe Ser Arg Ser Ala
    370                 375                 380
Asp Ala Pro Ala Tyr Gln Gln Gly Gln Asn Gln Leu Tyr Asn Glu Leu

385                     390                     395                     400
Asn Leu Gly Arg Arg Glu Glu Tyr Asp Val Leu Asp Lys Arg Arg Gly
                405                     410                     415
Arg Asp Pro Glu Met Gly Gly Lys Pro Arg Arg Lys Asn Pro Gln Glu
            420                     425                     430
Gly Leu Tyr Asn Glu Leu Gln Lys Asp Lys Met Ala Glu Ala Tyr Ser
        435                     440                     445
Glu Ile Gly Met Lys Gly Glu Arg Arg Arg Gly Lys Gly His Asp Gly
    450                     455                     460
Leu Tyr Gln Gly Leu Ser Thr Ala Thr Lys Asp Thr Tyr Asp Ala Leu
465                     470                     475                     480
His Met Gln Ala Leu Pro Pro Arg
                485

<210> 59
<211> 249
<212> PRT
<213> Artificial Sequence

<220>
<223> CD20 scFv

<400> 59
Ser Gln Val Gln Leu Val Gln Ser Gly Ala Glu Leu Val Lys Pro Gly
1               5                   10                  15
Ala Ser Val Lys Met Ser Cys Lys Ala Ser Gly Tyr Thr Phe Thr Ser
            20                  25                  30
Tyr Asn Met His Trp Val Lys Gln Thr Pro Gly Gln Gly Leu Glu Trp
        35                  40                  45
Ile Gly Ala Ile Tyr Pro Gly Asn Gly Asp Thr Ser Tyr Asn Gln Lys
    50                  55                  60
Phe Lys Gly Lys Ala Thr Leu Thr Ala Asp Lys Ser Ser Ser Thr Ala
65                  70                  75                  80
Tyr Met Gln Leu Ser Ser Leu Thr Ser Glu Asp Ser Ala Val Tyr Tyr
                85                  90                  95
Cys Ala Arg Ala Gln Leu Arg Pro Asn Tyr Trp Tyr Phe Asp Val Trp
            100                 105                 110
Gly Ala Gly Thr Thr Val Thr Val Ser Lys Ile Ser Gly Gly Gly Gly
        115                 120                 125
Ser Gly Gly Gly Gly Ser Gly Gly Gly Gly Ser Gly Gly Ser Ser Asp
    130                 135                 140
Ile Val Leu Ser Gln Ser Pro Ala Ile Leu Ser Ala Ser Pro Gly Glu
145                 150                 155                 160
Lys Val Thr Met Thr Cys Arg Ala Ser Ser Ser Val Ser Tyr Met His
                165                 170                 175
Trp Tyr Gln Gln Lys Pro Gly Ser Ser Pro Lys Pro Trp Ile Tyr Ala
            180                 185                 190
Thr Ser Asn Leu Ala Ser Gly Val Pro Ala Arg Phe Ser Gly Ser Gly
        195                 200                 205
Ser Gly Thr Ser Tyr Ser Leu Thr Ile Ser Arg Val Glu Ala Glu Asp
    210                 215                 220
Ala Ala Thr Tyr Tyr Cys Gln Gln Trp Ile Ser Asn Pro Pro Thr Phe
225                 230                 235                 240
Gly Ala Gly Thr Lys Leu Glu Leu Lys
                245

<210> 60
<211> 504
<212> PRT
<213> Artificial Sequence

<220>
<223> Chimeric receptor

<400> 60
Met Ala Leu Pro Val Thr Ala Leu Leu Leu Pro Leu Ala Leu Leu Leu
1               5                   10                  15
His Ala Ala Arg Pro Ser Gln Ile Val Leu Ser Gln Ser Pro Ala Ile
            20                  25                  30
Leu Ser Ala Ser Pro Gly Glu Lys Val Thr Met Thr Cys Arg Ala Ser
        35                  40                  45
Ser Ser Leu Ser Phe Met His Trp Tyr Gln Gln Lys Pro Gly Ser Ser
    50                  55                  60
Pro Lys Pro Trp Ile Tyr Ala Thr Ser Asn Leu Ala Ser Gly Val Pro
65                  70                  75                  80
Ala Arg Phe Ser Gly Ser Gly Ser Gly Thr Ser Tyr Ser Leu Thr Ile
                85                  90                  95
Ser Arg Val Glu Ala Glu Asp Ala Ala Thr Tyr Phe Cys His Gln Trp
            100                 105                 110
Ser Ser Asn Pro Leu Thr Phe Gly Ala Gly Thr Lys Leu Glu Leu Lys
        115                 120                 125
Arg Ser Gly Gly Gly Gly Ser Gly Gly Gly Gly Ser Gly Gly Gly Gly
    130                 135                 140
Ser Gly Ser Gln Val Gln Leu Arg Gln Pro Gly Ala Glu Leu Val Lys
145                 150                 155                 160
Pro Gly Ala Ser Val Lys Met Ser Cys Lys Ala Ser Gly Tyr Thr Phe
                165                 170                 175
Thr Ser Tyr Asn Met His Trp Val Lys Gln Thr Pro Gly Gln Gly Leu
            180                 185                 190
Glu Trp Ile Gly Ala Ile Tyr Pro Gly Asn Gly Asp Thr Ser Tyr Asn
        195                 200                 205
Gln Lys Phe Lys Gly Lys Ala Thr Leu Thr Ala Asp Lys Ser Ser Ser
    210                 215                 220
Thr Ala Tyr Met Gln Leu Ser Ser Leu Thr Ser Glu Asp Ser Ala Val
225                 230                 235                 240
Tyr Tyr Cys Ala Arg Ser His Tyr Gly Ser Asn Tyr Val Asp Tyr Trp
                245                 250                 255
Gly Gln Gly Thr Thr Leu Thr Val Ser Ser Ala Trp Ser His Pro Gln
            260                 265                 270
Phe Glu Lys Ser Gly Met His Thr Thr Thr Pro Ala Pro Arg Pro Pro
        275                 280                 285
Thr Pro Ala Pro Thr Ile Ala Ser Gln Pro Leu Ser Leu Arg Pro Glu
    290                 295                 300
Ala Cys Arg Pro Ala Ala Gly Gly Ala Val His Thr Arg Gly Leu Asp
305                 310                 315                 320
Phe Ala Cys Asp Ile Tyr Ile Trp Ala Pro Leu Ala Gly Thr Cys Gly
                325                 330                 335
Val Leu Leu Leu Ser Leu Val Ile Thr Leu Tyr Cys Lys Arg Gly Arg
            340                 345                 350
Lys Lys Leu Leu Tyr Ile Phe Lys Gln Pro Phe Met Arg Pro Val Gln
        355                 360                 365
Thr Thr Gln Glu Glu Asp Gly Cys Ser Cys Arg Phe Pro Glu Glu Glu
    370                 375                 380
Glu Gly Gly Cys Glu Leu Thr Arg Arg Val Lys Phe Ser Arg Ser Ala
385                 390                 395                 400
Asp Ala Pro Ala Tyr Gln Gln Gly Gln Asn Gln Leu Tyr Asn Glu Leu
                405                 410                 415
Asn Leu Gly Arg Arg Glu Glu Tyr Asp Val Leu Asp Lys Arg Arg Gly
            420                 425                 430
Arg Asp Pro Glu Met Gly Gly Lys Pro Arg Arg Lys Asn Pro Gln Glu
        435                 440                 445
Gly Leu Tyr Asn Glu Leu Gln Lys Asp Lys Met Ala Glu Ala Tyr Ser
    450                 455                 460
Glu Ile Gly Met Lys Gly Glu Arg Arg Arg Gly Lys Gly His Asp Gly

465                 470                 475                 480
Leu Tyr Gln Gly Leu Ser Thr Ala Thr Lys Asp Thr Tyr Asp Ala Leu
                485                 490                 495
His Met Gln Ala Leu Pro Pro Arg
            500

<210> 61
<211> 483
<212> PRT
<213> Artificial Sequence

<220>
<223> Chimeric receptor

<400> 61
Ser Gln Ile Val Leu Ser Gln Ser Pro Ala Ile Leu Ser Ala Ser Pro
1               5                   10                  15
Gly Glu Lys Val Thr Met Thr Cys Arg Ala Ser Ser Ser Leu Ser Phe
            20                  25                  30
Met His Trp Tyr Gln Gln Lys Pro Gly Ser Ser Pro Lys Pro Trp Ile
        35                  40                  45
Tyr Ala Thr Ser Asn Leu Ala Ser Gly Val Pro Ala Arg Phe Ser Gly
    50                  55                  60
Ser Gly Ser Gly Thr Ser Tyr Ser Leu Thr Ile Ser Arg Val Glu Ala
65                  70                  75                  80
Glu Asp Ala Ala Thr Tyr Phe Cys His Gln Trp Ser Ser Asn Pro Leu
                85                  90                  95
Thr Phe Gly Ala Gly Thr Lys Leu Glu Leu Lys Arg Ser Gly Gly Gly
            100                 105                 110
Gly Ser Gly Gly Gly Gly Ser Gly Gly Gly Gly Ser Gly Ser Gln Val
        115                 120                 125
Gln Leu Arg Gln Pro Gly Ala Glu Leu Val Lys Pro Gly Ala Ser Val
    130                 135                 140
Lys Met Ser Cys Lys Ala Ser Gly Tyr Thr Phe Thr Ser Tyr Asn Met
145                 150                 155                 160
His Trp Val Lys Gln Thr Pro Gly Gln Gly Leu Glu Trp Ile Gly Ala
                165                 170                 175
Ile Tyr Pro Gly Asn Gly Asp Thr Ser Tyr Asn Gln Lys Phe Lys Gly
            180                 185                 190
Lys Ala Thr Leu Thr Ala Asp Lys Ser Ser Ser Thr Ala Tyr Met Gln
        195                 200                 205
Leu Ser Ser Leu Thr Ser Glu Asp Ser Ala Val Tyr Tyr Cys Ala Arg
    210                 215                 220
Ser His Tyr Gly Ser Asn Tyr Val Asp Tyr Trp Gly Gln Gly Thr Thr
225                 230                 235                 240
Leu Thr Val Ser Ser Ala Trp Ser His Pro Gln Phe Glu Lys Ser Gly
                245                 250                 255
Met His Thr Thr Thr Pro Ala Pro Arg Pro Pro Thr Pro Ala Pro Thr
            260                 265                 270
Ile Ala Ser Gln Pro Leu Ser Leu Arg Pro Glu Ala Cys Arg Pro Ala
        275                 280                 285
Ala Gly Gly Ala Val His Thr Arg Gly Leu Asp Phe Ala Cys Asp Ile
    290                 295                 300
Tyr Ile Trp Ala Pro Leu Ala Gly Thr Cys Gly Val Leu Leu Leu Ser
305                 310                 315                 320
Leu Val Ile Thr Leu Tyr Cys Lys Arg Gly Arg Lys Lys Leu Leu Tyr
                325                 330                 335
Ile Phe Lys Gln Pro Phe Met Arg Pro Val Gln Thr Thr Gln Glu Glu
            340                 345                 350
Asp Gly Cys Ser Cys Arg Phe Pro Glu Glu Glu Glu Gly Gly Cys Glu
        355                 360                 365
Leu Thr Arg Arg Val Lys Phe Ser Arg Ser Ala Asp Ala Pro Ala Tyr

370                 375                 380
Gln Gln Gly Gln Asn Gln Leu Tyr Asn Glu Leu Asn Leu Gly Arg Arg
385                 390                 395                 400
Glu Glu Tyr Asp Val Leu Asp Lys Arg Arg Gly Arg Asp Pro Glu Met
                405                 410                 415
Gly Gly Lys Pro Arg Arg Lys Asn Pro Gln Glu Gly Leu Tyr Asn Glu
            420                 425                 430
Leu Gln Lys Asp Lys Met Ala Glu Ala Tyr Ser Glu Ile Gly Met Lys
        435                 440                 445
Gly Glu Arg Arg Arg Gly Lys Gly His Asp Gly Leu Tyr Gln Gly Leu
    450                 455                 460
Ser Thr Ala Thr Lys Asp Thr Tyr Asp Ala Leu His Met Gln Ala Leu
465                 470                 475                 480
Pro Pro Arg


<210> 62
<211> 245
<212> PRT
<213> Artificial Sequence


<220>
<223> CD20 scFv

<400> 62
Ser Gln Ile Val Leu Ser Gln Ser Pro Ala Ile Leu Ser Ala Ser Pro
1               5                   10                  15
Gly Glu Lys Val Thr Met Thr Cys Arg Ala Ser Ser Ser Leu Ser Phe
            20                  25                  30
Met His Trp Tyr Gln Gln Lys Pro Gly Ser Ser Pro Lys Pro Trp Ile
        35                  40                  45
Tyr Ala Thr Ser Asn Leu Ala Ser Gly Val Pro Ala Arg Phe Ser Gly
    50                  55                  60
Ser Gly Ser Gly Thr Ser Tyr Ser Leu Thr Ile Ser Arg Val Glu Ala
65                  70                  75                  80
Glu Asp Ala Ala Thr Tyr Phe Cys His Gln Trp Ser Ser Asn Pro Leu
                85                  90                  95
Thr Phe Gly Ala Gly Thr Lys Leu Glu Leu Lys Arg Ser Gly Gly Gly
            100                 105                 110
Gly Ser Gly Gly Gly Gly Ser Gly Gly Gly Gly Ser Gly Ser Gln Val
        115                 120                 125
Gln Leu Arg Gln Pro Gly Ala Glu Leu Val Lys Pro Gly Ala Ser Val
    130                 135                 140
Lys Met Ser Cys Lys Ala Ser Gly Tyr Thr Phe Thr Ser Tyr Asn Met
145                 150                 155                 160
His Trp Val Lys Gln Thr Pro Gly Gln Gly Leu Glu Trp Ile Gly Ala
                165                 170                 175
Ile Tyr Pro Gly Asn Gly Asp Thr Ser Tyr Asn Gln Lys Phe Lys Gly
            180                 185                 190
Lys Ala Thr Leu Thr Ala Asp Lys Ser Ser Ser Thr Ala Tyr Met Gln
        195                 200                 205
Leu Ser Ser Leu Thr Ser Glu Asp Ser Ala Val Tyr Tyr Cys Ala Arg
    210                 215                 220
Ser His Tyr Gly Ser Asn Tyr Val Asp Tyr Trp Gly Gln Gly Thr Thr
225                 230                 235                 240
Leu Thr Val Ser Ser
                245

<210> 63
<211> 9
<212> PRT
<213> Artificial Sequence

<220>
<223> Motif

<400> 63
Leu Ala Gly Leu Ile Asp Ala Asp Gly
1               5

<210> 64
<211> 9
<212> PRT
<213> Artificial Sequence


<220>
<223> Tag

<400> 64
Asn Trp Ser His Pro Gln Phe Glu Lys
1               5

<210> 65
<211> 10
<212> PRT
<213> Artificial Sequence


<220>
<223> Tag

<400> 65
Ser Ala Trp Ser His Pro Gln Phe Glu Lys
1               5                   10

<210> 66
<211> 113
<212> PRT
<213> Artificial Sequence


<220>
<223> CD3 zeta

<400> 66
Arg Val Lys Phe Ser Arg Ser Ala Asp Ala Pro Ala Tyr Lys Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Gln Arg Arg Lys Asn Pro Gln Glu Gly Leu Tyr Asn Glu Leu Gln
    50                  55                  60
Lys Asp Lys Met Ala Glu Ala Tyr Ser Glu Ile Gly Met Lys Gly Glu
65                  70                  75                  80
Arg Arg Arg Gly Lys Gly His Asp Gly Leu Tyr Gln Gly Leu Ser Thr
                85                  90                  95
Ala Thr Lys Asp Thr Tyr Asp Ala Leu His Met Gln Ala Leu Pro Pro
            100                 105                 110
Arg


<210> 67

<211> 113
<212> PRT
<213> Artificial Sequence

<220>
<223> CD3 zeta

<400> 67
Arg Val Lys Phe Ser Arg Ser Ala Asp Ala Pro Ala Tyr Gln Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Gln Arg Arg Lys Asn Pro Gln Glu Gly Leu Tyr Asn Glu Leu Gln
    50                  55                  60
Lys Asp Lys Met Ala Glu Ala Tyr Ser Glu Ile Gly Met Lys Gly Glu
65                  70                  75                  80
Arg Arg Arg Gly Lys Gly His Asp Gly Leu Tyr Gln Gly Leu Ser Thr
                85                  90                  95
Ala Thr Lys Asp Thr Tyr Asp Ala Leu His Met Gln Ala Leu Pro Pro
            100                 105                 110
Arg

<210> 68
<211> 223
<212> PRT
<213> Artificial Sequence

<220>
<223> Chimeric receptor

<400> 68
Ala Ala Ala Ile Glu Val Met Tyr Pro Pro Pro Tyr Leu Asp Asn Glu
1               5                   10                  15
Lys Ser Asn Gly Thr Ile Ile His Val Lys Gly Lys His Leu Cys Pro
            20                  25                  30
Ser Pro Leu Phe Pro Gly Pro Ser Lys Pro Phe Trp Val Leu Val Val
        35                  40                  45
Val Gly Gly Val Leu Ala Cys Tyr Ser Leu Leu Val Thr Val Ala Phe
    50                  55                  60
Ile Ile Phe Trp Val Arg Ser Lys Arg Ser Arg Leu Leu His Ser Asp
65                  70                  75                  80
Tyr Met Asn Met Thr Pro Arg Arg Pro Gly Pro Thr Arg Lys His Tyr
                85                  90                  95
Gln Pro Tyr Ala Pro Pro Arg Asp Phe Ala Ala Tyr Arg Ser Arg Val
            100                 105                 110
Lys Phe Ser Arg Ser Ala Asp Ala Pro Ala Tyr Gln Gln Gly Gln Asn
        115                 120                 125
Gln Leu Tyr Asn Glu Leu Asn Leu Gly Arg Arg Glu Glu Tyr Asp Val
    130                 135                 140
Leu Asp Lys Arg Arg Gly Arg Asp Pro Glu Met Gly Gly Lys Pro Gln
145                 150                 155                 160
Arg Arg Lys Asn Pro Gln Glu Gly Leu Tyr Asn Glu Leu Gln Lys Asp
                165                 170                 175
Lys Met Ala Glu Ala Tyr Ser Glu Ile Gly Met Lys Gly Glu Arg Arg
            180                 185                 190
Arg Gly Lys Gly His Asp Gly Leu Tyr Gln Gly Leu Ser Thr Ala Thr
        195                 200                 205
Lys Asp Thr Tyr Asp Ala Leu His Met Gln Ala Leu Pro Pro Arg

210                 215                 220

<210> 69
<211> 490
<212> PRT
<213> Artificial Sequence

<220>
<223> Chimeric receptor

<400> 69
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Ser Thr Ser Gly Ser Gly Lys Pro Gly Ser Gly Glu Gly Ser Thr
    130                 135                 140
Lys Gly Glu Val Lys Leu Gln Glu Ser Gly Pro Gly Leu Val Ala Pro
145                 150                 155                 160
Ser Gln Ser Leu Ser Val Thr Cys Thr Val Ser Gly Val Ser Leu Pro
                165                 170                 175
Asp Tyr Gly Val Ser Trp Ile Arg Gln Pro Pro Arg Lys Gly Leu Glu
            180                 185                 190
Trp Leu Gly Val Ile Trp Gly Ser Glu Thr Thr Tyr Tyr Asn Ser Ala
        195                 200                 205
Leu Lys Ser Arg Leu Thr Ile Ile Lys Asp Asn Ser Lys Ser Gln Val
    210                 215                 220
Phe Leu Lys Met Asn Ser Leu Gln Thr Asp Asp Thr Ala Ile Tyr Tyr
225                 230                 235                 240
Cys Ala Lys His Tyr Tyr Tyr Gly Gly Ser Tyr Ala Met Asp Tyr Trp
                245                 250                 255
Gly Gln Gly Thr Ser Val Thr Val Ser Ser Thr Thr Thr Pro Ala Pro
            260                 265                 270
Arg Pro Pro Thr Pro Ala Pro Thr Ile Ala Ser Gln Pro Leu Ser Leu
        275                 280                 285
Arg Pro Glu Ala Cys Arg Pro Ala Ala Gly Gly Ala Val His Thr Arg
    290                 295                 300
Gly Leu Asp Phe Ala Cys Asp Ile Tyr Ile Trp Ala Pro Leu Ala Gly
305                 310                 315                 320
Thr Cys Gly Val Leu Leu Leu Ser Leu Val Ile Thr Leu Tyr Cys Lys
                325                 330                 335
Arg Gly Arg Lys Lys Leu Leu Tyr Ile Phe Lys Gln Pro Phe Met Arg
            340                 345                 350
Pro Val Gln Thr Thr Gln Glu Glu Asp Gly Cys Ser Cys Arg Phe Pro
        355                 360                 365
Glu Glu Glu Glu Gly Gly Cys Glu Leu Arg Val Lys Phe Ser Arg Ser
    370                 375                 380
Ala Asp Ala Pro Ala Tyr Gln Gln Gly Gln Asn Gln Leu Tyr Asn Glu
385                 390                 395                 400
Leu Asn Leu Gly Arg Arg Glu Glu Tyr Asp Val Leu Asp Lys Arg Arg

                    405                     410                     415
Gly Arg Asp Pro Glu Met Gly Gly Lys Pro Gln Arg Arg Lys Asn Pro
                420                     425                     430
Gln Glu Gly Leu Tyr Asn Glu Leu Gln Lys Asp Lys Met Ala Glu Ala
            435                     440                     445
Tyr Ser Glu Ile Gly Met Lys Gly Glu Arg Arg Arg Gly Lys Gly His
    450                     455                     460
Asp Gly Leu Tyr Gln Gly Leu Ser Thr Ala Thr Lys Asp Thr Tyr Asp
465                     470                     475                     480
Ala Leu His Met Gln Ala Leu Pro Pro Arg
                485                     490

<210> 70
<211> 469
<212> PRT
<213> Artificial Sequence

<220>
<223> Chimeric receptor

<400> 70
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys
        115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Ile
                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn
        195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser Thr Thr Thr Pro Ala Pro Arg Pro Pro Thr Pro
                245                 250                 255
Ala Pro Thr Ile Ala Ser Gln Pro Leu Ser Leu Arg Pro Glu Ala Cys
            260                 265                 270
Arg Pro Ala Ala Gly Gly Ala Val His Thr Arg Gly Leu Asp Phe Ala
        275                 280                 285
Cys Asp Ile Tyr Ile Trp Ala Pro Leu Ala Gly Thr Cys Gly Val Leu
    290                 295                 300
Leu Leu Ser Leu Val Ile Thr Leu Tyr Cys Lys Arg Gly Arg Lys Lys
305                 310                 315                 320
Leu Leu Tyr Ile Phe Lys Gln Pro Phe Met Arg Pro Val Gln Thr Thr

325                 330                 335
Gln Glu Glu Asp Gly Cys Ser Cys Arg Phe Pro Glu Glu Glu Glu Gly
            340                 345                 350
Gly Cys Glu Leu Arg Val Lys Phe Ser Arg Ser Ala Asp Ala Pro Ala
        355                 360                 365
Tyr Gln Gln Gly Gln Asn Gln Leu Tyr Asn Glu Leu Asn Leu Gly Arg
    370                 375                 380
Arg Glu Glu Tyr Asp Val Leu Asp Lys Arg Arg Gly Arg Asp Pro Glu
385                 390                 395                 400
Met Gly Gly Lys Pro Gln Arg Arg Lys Asn Pro Gln Glu Gly Leu Tyr
                405                 410                 415
Asn Glu Leu Gln Lys Asp Lys Met Ala Glu Ala Tyr Ser Glu Ile Gly
            420                 425                 430
Met Lys Gly Glu Arg Arg Arg Gly Lys Gly His Asp Gly Leu Tyr Gln
        435                 440                 445
Gly Leu Ser Thr Ala Thr Lys Asp Thr Tyr Asp Ala Leu His Met Gln
    450                 455                 460
Ala Leu Pro Pro Arg
465

<210> 71
<211> 503
<212> PRT
<213> Artificial Sequence

<220>
<223> Chimeric receptor

<400> 71
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Ser Thr Ser Gly Ser Gly Lys Pro Gly Ser Gly Glu Gly Ser Thr
    130                 135                 140
Lys Gly Glu Val Lys Leu Gln Glu Ser Gly Pro Gly Leu Val Ala Pro
145                 150                 155                 160
Ser Gln Ser Leu Ser Val Thr Cys Thr Val Ser Gly Val Ser Leu Pro
                165                 170                 175
Asp Tyr Gly Val Ser Trp Ile Arg Gln Pro Pro Arg Lys Gly Leu Glu
            180                 185                 190
Trp Leu Gly Val Ile Trp Gly Ser Glu Thr Thr Tyr Tyr Asn Ser Ala
        195                 200                 205
Leu Lys Ser Arg Leu Thr Ile Ile Lys Asp Asn Ser Lys Ser Gln Val
    210                 215                 220
Phe Leu Lys Met Asn Ser Leu Gln Thr Asp Asp Thr Ala Ile Tyr Tyr
225                 230                 235                 240
Cys Ala Lys His Tyr Tyr Tyr Gly Gly Ser Tyr Ala Met Asp Tyr Trp
                245                 250                 255
Gly Gln Gly Thr Ser Val Thr Val Ser Ser Asn Trp Ser His Pro Gln

260                 265                 270
Phe Glu Lys Met His Thr Thr Thr Pro Ala Pro Arg Pro Pro Thr Pro
        275                 280                 285
Ala Pro Thr Ile Ala Ser Gln Pro Leu Ser Leu Arg Pro Glu Ala Cys
    290                 295                 300
Arg Pro Ala Ala Gly Gly Ala Val His Thr Arg Gly Leu Asp Phe Ala
305                 310                 315                 320
Cys Asp Ile Tyr Ile Trp Ala Pro Leu Ala Gly Thr Cys Gly Val Leu
                325                 330                 335
Leu Leu Ser Leu Val Ile Thr Leu Tyr Cys Lys Arg Gly Arg Lys Lys
            340                 345                 350
Leu Leu Tyr Ile Phe Lys Gln Pro Phe Met Arg Pro Val Gln Thr Thr
        355                 360                 365
Gln Glu Glu Asp Gly Cys Ser Cys Arg Phe Pro Glu Glu Glu Glu Gly
    370                 375                 380
Gly Cys Glu Leu Thr Arg Arg Val Lys Phe Ser Arg Ser Ala Asp Ala
385                 390                 395                 400
Pro Ala Tyr Gln Gln Gly Gln Asn Gln Leu Tyr Asn Glu Leu Asn Leu
                405                 410                 415
Gly Arg Arg Glu Glu Tyr Asp Val Leu Asp Lys Arg Arg Gly Arg Asp
            420                 425                 430
Pro Glu Met Gly Gly Lys Pro Gln Arg Arg Lys Asn Pro Gln Glu Gly
        435                 440                 445
Leu Tyr Asn Glu Leu Gln Lys Asp Lys Met Ala Glu Ala Tyr Ser Glu
    450                 455                 460
Ile Gly Met Lys Gly Glu Arg Arg Arg Gly Lys Gly His Asp Gly Leu
465                 470                 475                 480
Tyr Gln Gly Leu Ser Thr Ala Thr Lys Asp Thr Tyr Asp Ala Leu His
                485                 490                 495
Met Gln Ala Leu Pro Pro Arg
            500

<210> 72
<211> 482
<212> PRT
<213> Artificial Sequence

<220>
<223> Chimeric receptor

<400> 72
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys
        115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Ile

                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn
        195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser Asn Trp Ser His Pro Gln Phe Glu Lys Met His
                245                 250                 255
Thr Thr Thr Pro Ala Pro Arg Pro Pro Thr Pro Ala Pro Thr Ile Ala
            260                 265                 270
Ser Gln Pro Leu Ser Leu Arg Pro Glu Ala Cys Arg Pro Ala Ala Gly
        275                 280                 285
Gly Ala Val His Thr Arg Gly Leu Asp Phe Ala Cys Asp Ile Tyr Ile
    290                 295                 300
Trp Ala Pro Leu Ala Gly Thr Cys Gly Val Leu Leu Leu Ser Leu Val
305                 310                 315                 320
Ile Thr Leu Tyr Cys Lys Arg Gly Arg Lys Lys Leu Leu Tyr Ile Phe
                325                 330                 335
Lys Gln Pro Phe Met Arg Pro Val Gln Thr Thr Gln Glu Glu Asp Gly
            340                 345                 350
Cys Ser Cys Arg Phe Pro Glu Glu Glu Glu Gly Gly Cys Glu Leu Thr
        355                 360                 365
Arg Arg Val Lys Phe Ser Arg Ser Ala Asp Ala Pro Ala Tyr Gln Gln
    370                 375                 380
Gly Gln Asn Gln Leu Tyr Asn Glu Leu Asn Leu Gly Arg Arg Glu Glu
385                 390                 395                 400
Tyr Asp Val Leu Asp Lys Arg Arg Gly Arg Asp Pro Glu Met Gly Gly
                405                 410                 415
Lys Pro Gln Arg Arg Lys Asn Pro Gln Glu Gly Leu Tyr Asn Glu Leu
            420                 425                 430
Gln Lys Asp Lys Met Ala Glu Ala Tyr Ser Glu Ile Gly Met Lys Gly
        435                 440                 445
Glu Arg Arg Arg Gly Lys Gly His Asp Gly Leu Tyr Gln Gly Leu Ser
    450                 455                 460
Thr Ala Thr Lys Asp Thr Tyr Asp Ala Leu His Met Gln Ala Leu Pro
465                 470                 475                 480
Pro Arg

<210> 73
<211> 520
<212> PRT
<213> Artificial Sequence

<220>
<223> Chimeric receptor

<400> 73
Met Ala Leu Pro Val Thr Ala Leu Leu Leu Pro Leu Ala Leu Leu Leu
1               5                   10                  15
His Ala Ala Arg Pro Asp Ile Gln Leu Thr Gln Ser Pro Ala Ile Leu
            20                  25                  30
Ser Ala Ser Pro Gly Glu Lys Val Thr Met Thr Cys Arg Ala Ser Ser
        35                  40                  45
Ser Leu Ser Phe Met His Trp Tyr Gln Gln Lys Pro Gly Ser Ser Pro
    50                  55                  60
Lys Pro Trp Ile Tyr Ala Thr Ser Asn Leu Ala Ser Gly Val Pro Ala
65                  70                  75                  80
Arg Phe Ser Gly Ser Gly Ser Gly Thr Ser Tyr Ser Leu Thr Ile Ser

85                  90                  95
Thr Val Glu Ala Glu Asp Ala Ala Ser Tyr Phe Cys His Gln Trp Ser
            100                 105                 110
Ser Asn Pro Leu Thr Phe Gly Ala Gly Thr Lys Leu Glu Ile Ser Ser
        115                 120                 125
Gly Gly Gly Gly Ser Gly Gly Gly Gly Ser Gly Asp Val Met Gly Val
    130                 135                 140
Asp Ser Gly Gly Gly Leu Val Gln Pro Gly Gly Ser Arg Lys Leu Ser
145                 150                 155                 160
Cys Ala Ala Pro Gly Phe Thr Phe Ser Ser Phe Gly Met His Trp Val
                165                 170                 175
Arg Gln Ala Pro Glu Lys Gly Leu Glu Trp Val Ala Tyr Ile Ser Ser
            180                 185                 190
Pro Ser Ser Thr Leu His Tyr Ala Asp Arg Val Lys Gly Arg Phe Thr
        195                 200                 205
Ile Ser Arg Asp Asn Pro Lys Asn Thr Leu Phe Leu Gln Met Lys Leu
    210                 215                 220
Pro Ser Leu Cys Tyr Gly Leu Leu Gly Pro Arg Asp His Val His Arg
225                 230                 235                 240
Leu Leu Lys His His His His His His Leu Glu Tyr Pro Tyr Asp Val
                245                 250                 255
Pro Asp Tyr Ala Thr Thr Asp Ala Ala His Pro Gly Arg Ser Val Val
            260                 265                 270
Pro Ala Leu Leu Pro Leu Leu Ala Gly Thr Leu Leu Leu Leu Glu Thr
        275                 280                 285
Ala Thr Ala Pro Met His Thr Thr Thr Pro Ala Pro Arg Pro Pro Thr
    290                 295                 300
Pro Ala Pro Thr Ile Ala Ser Gln Pro Leu Ser Leu Arg Pro Glu Ala
305                 310                 315                 320
Cys Arg Pro Ala Ala Gly Gly Ala Val His Thr Arg Gly Leu Asp Phe
                325                 330                 335
Ala Cys Asp Ile Tyr Ile Trp Ala Pro Leu Ala Gly Thr Cys Gly Val
            340                 345                 350
Leu Leu Leu Ser Leu Val Ile Thr Leu Tyr Cys Lys Arg Gly Arg Lys
        355                 360                 365
Lys Leu Leu Tyr Ile Phe Lys Gln Pro Phe Met Arg Pro Val Gln Thr
    370                 375                 380
Thr Gln Glu Glu Asp Gly Cys Ser Cys Arg Phe Pro Glu Glu Glu Glu
385                 390                 395                 400
Gly Gly Cys Glu Leu Thr Arg Arg Val Lys Phe Ser Arg Ser Ala Asp
                405                 410                 415
Ala Pro Ala Tyr Gln Gln Gly Gln Asn Gln Leu Tyr Asn Glu Leu Asn
            420                 425                 430
Leu Gly Arg Arg Glu Glu Tyr Asp Val Leu Asp Lys Arg Arg Gly Arg
        435                 440                 445
Asp Pro Glu Met Gly Gly Lys Pro Gln Arg Arg Lys Asn Pro Gln Glu
    450                 455                 460
Gly Leu Tyr Asn Glu Leu Gln Lys Asp Lys Met Ala Glu Ala Tyr Ser
465                 470                 475                 480
Glu Ile Gly Met Lys Gly Glu Arg Arg Arg Gly Lys Gly His Asp Gly
                485                 490                 495
Leu Tyr Gln Gly Leu Ser Thr Ala Thr Lys Asp Thr Tyr Asp Ala Leu
            500                 505                 510
His Met Gln Ala Leu Pro Pro Arg
        515                 520

<210> 74
<211> 499
<212> PRT
<213> Artificial Sequence

<220>

<223> Chimeric receptor

<400> 74
Asp Ile Gln Leu Thr Gln Ser Pro Ala Ile Leu Ser Ala Ser Pro Gly
1               5                   10                  15
Glu Lys Val Thr Met Thr Cys Arg Ala Ser Ser Ser Leu Ser Phe Met
            20                  25                  30
His Trp Tyr Gln Gln Lys Pro Gly Ser Ser Pro Lys Pro Trp Ile Tyr
        35                  40                  45
Ala Thr Ser Asn Leu Ala Ser Gly Val Pro Ala Arg Phe Ser Gly Ser
    50                  55                  60
Gly Ser Gly Thr Ser Tyr Ser Leu Thr Ile Ser Thr Val Glu Ala Glu
65                  70                  75                  80
Asp Ala Ala Ser Tyr Phe Cys His Gln Trp Ser Ser Asn Pro Leu Thr
                85                  90                  95
Phe Gly Ala Gly Thr Lys Leu Glu Ile Ser Ser Gly Gly Gly Gly Ser
            100                 105                 110
Gly Gly Gly Gly Ser Gly Asp Val Met Gly Val Asp Ser Gly Gly Gly
        115                 120                 125
Leu Val Gln Pro Gly Gly Ser Arg Lys Leu Ser Cys Ala Ala Pro Gly
    130                 135                 140
Phe Thr Phe Ser Ser Phe Gly Met His Trp Val Arg Gln Ala Pro Glu
145                 150                 155                 160
Lys Gly Leu Glu Trp Val Ala Tyr Ile Ser Ser Pro Ser Ser Thr Leu
                165                 170                 175
His Tyr Ala Asp Arg Val Lys Gly Arg Phe Thr Ile Ser Arg Asp Asn
            180                 185                 190
Pro Lys Asn Thr Leu Phe Leu Gln Met Lys Leu Pro Ser Leu Cys Tyr
        195                 200                 205
Gly Leu Leu Gly Pro Arg Asp His Val His Arg Leu Leu Lys His His
    210                 215                 220
His His His His Leu Glu Tyr Pro Tyr Asp Val Pro Asp Tyr Ala Thr
225                 230                 235                 240
Thr Asp Ala Ala His Pro Gly Arg Ser Val Val Pro Ala Leu Leu Pro
                245                 250                 255
Leu Leu Ala Gly Thr Leu Leu Leu Leu Glu Thr Ala Thr Ala Pro Met
            260                 265                 270
His Thr Thr Thr Pro Ala Pro Arg Pro Pro Thr Pro Ala Pro Thr Ile
        275                 280                 285
Ala Ser Gln Pro Leu Ser Leu Arg Pro Glu Ala Cys Arg Pro Ala Ala
    290                 295                 300
Gly Gly Ala Val His Thr Arg Gly Leu Asp Phe Ala Cys Asp Ile Tyr
305                 310                 315                 320
Ile Trp Ala Pro Leu Ala Gly Thr Cys Gly Val Leu Leu Leu Ser Leu
                325                 330                 335
Val Ile Thr Leu Tyr Cys Lys Arg Gly Arg Lys Lys Leu Leu Tyr Ile
            340                 345                 350
Phe Lys Gln Pro Phe Met Arg Pro Val Gln Thr Thr Gln Glu Glu Asp
        355                 360                 365
Gly Cys Ser Cys Arg Phe Pro Glu Glu Glu Glu Gly Gly Cys Glu Leu
    370                 375                 380
Thr Arg Arg Val Lys Phe Ser Arg Ser Ala Asp Ala Pro Ala Tyr Gln
385                 390                 395                 400
Gln Gly Gln Asn Gln Leu Tyr Asn Glu Leu Asn Leu Gly Arg Arg Glu
                405                 410                 415
Glu Tyr Asp Val Leu Asp Lys Arg Arg Gly Arg Asp Pro Glu Met Gly
            420                 425                 430
Gly Lys Pro Gln Arg Arg Lys Asn Pro Gln Glu Gly Leu Tyr Asn Glu
        435                 440                 445
Leu Gln Lys Asp Lys Met Ala Glu Ala Tyr Ser Glu Ile Gly Met Lys
    450                 455                 460
Gly Glu Arg Arg Arg Gly Lys Gly His Asp Gly Leu Tyr Gln Gly Leu
465                 470                 475                 480

Ser Thr Ala Thr Lys Asp Thr Tyr Asp Ala Leu His Met Gln Ala Leu
                485                 490                 495
Pro Pro Arg



<210> 75
<211> 493
<212> PRT
<213> Artificial Sequence



<220>
<223> Chimeric receptor

<400> 75
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Ser Thr Ser Gly Ser Gly Lys Pro Gly Ser Gly Glu Gly Ser Thr
    130                 135                 140
Lys Gly Glu Val Lys Leu Gln Glu Ser Gly Pro Gly Leu Val Ala Pro
145                 150                 155                 160
Ser Gln Ser Leu Ser Val Thr Cys Thr Val Ser Gly Val Ser Leu Pro
                165                 170                 175
Asp Tyr Gly Val Ser Trp Ile Arg Gln Pro Pro Arg Lys Gly Leu Glu
            180                 185                 190
Trp Leu Gly Val Ile Trp Gly Ser Glu Thr Thr Tyr Tyr Asn Ser Ala
        195                 200                 205
Leu Lys Ser Arg Leu Thr Ile Ile Lys Asp Asn Ser Lys Ser Gln Val
    210                 215                 220
Phe Leu Lys Met Asn Ser Leu Gln Thr Asp Asp Thr Ala Ile Tyr Tyr
225                 230                 235                 240
Cys Ala Lys His Tyr Tyr Tyr Gly Gly Ser Tyr Ala Met Asp Tyr Trp
                245                 250                 255
Gly Gln Gly Thr Ser Val Thr Val Ser Ser Met His Thr Thr Thr Pro
            260                 265                 270
Ala Pro Arg Pro Pro Thr Pro Ala Pro Thr Ile Ala Ser Gln Pro Leu
        275                 280                 285
Ser Leu Arg Pro Glu Ala Cys Arg Pro Ala Ala Gly Gly Ala Val His
    290                 295                 300
Thr Arg Gly Leu Asp Phe Ala Cys Asp Ile Tyr Ile Trp Ala Pro Leu
305                 310                 315                 320
Ala Gly Thr Cys Gly Val Leu Leu Leu Ser Leu Val Ile Thr Leu Tyr
                325                 330                 335
Cys Arg Ser Lys Arg Ser Arg Leu Leu His Ser Asp Tyr Met Asn Met
            340                 345                 350
Thr Pro Arg Arg Pro Gly Pro Thr Arg Lys His Tyr Gln Pro Tyr Ala
        355                 360                 365
Pro Pro Arg Asp Phe Ala Ala Tyr Arg Ser Thr Arg Arg Val Lys Phe
    370                 375                 380

Ser Arg Ser Ala Asp Ala Pro Ala Tyr Gln Gln Gly Gln Asn Gln Leu
385                 390                 395                 400
Tyr Asn Glu Leu Asn Leu Gly Arg Arg Glu Glu Tyr Asp Val Leu Asp
                405                 410                 415
Lys Arg Arg Gly Arg Asp Pro Glu Met Gly Gly Lys Pro Gln Arg Arg
            420                 425                 430
Lys Asn Pro Gln Glu Gly Leu Tyr Asn Glu Leu Gln Lys Asp Lys Met
        435                 440                 445
Ala Glu Ala Tyr Ser Glu Ile Gly Met Lys Gly Glu Arg Arg Arg Gly
    450                 455                 460
Lys Gly His Asp Gly Leu Tyr Gln Gly Leu Ser Thr Ala Thr Lys Asp
465                 470                 475                 480
Thr Tyr Asp Ala Leu His Met Gln Ala Leu Pro Pro Arg
                485                 490

<210> 76
<211> 472
<212> PRT
<213> Artificial Sequence

<220>
<223> Chimeric receptor

<400> 76
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys
        115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Ile
                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn
        195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser Met His Thr Thr Thr Pro Ala Pro Arg Pro Pro
                245                 250                 255
Thr Pro Ala Pro Thr Ile Ala Ser Gln Pro Leu Ser Leu Arg Pro Glu
            260                 265                 270
Ala Cys Arg Pro Ala Ala Gly Gly Ala Val His Thr Arg Gly Leu Asp
        275                 280                 285
Phe Ala Cys Asp Ile Tyr Ile Trp Ala Pro Leu Ala Gly Thr Cys Gly
    290                 295                 300

Val Leu Leu Leu Ser Leu Val Ile Thr Leu Tyr Cys Arg Ser Lys Arg
305                 310                 315                 320
Ser Arg Leu Leu His Ser Asp Tyr Met Asn Met Thr Pro Arg Arg Pro
                325                 330                 335
Gly Pro Thr Arg Lys His Tyr Gln Pro Tyr Ala Pro Pro Arg Asp Phe
            340                 345                 350
Ala Ala Tyr Arg Ser Thr Arg Arg Val Lys Phe Ser Arg Ser Ala Asp
        355                 360                 365
Ala Pro Ala Tyr Gln Gln Gly Gln Asn Gln Leu Tyr Asn Glu Leu Asn
    370                 375                 380
Leu Gly Arg Arg Glu Glu Tyr Asp Val Leu Asp Lys Arg Arg Gly Arg
385                 390                 395                 400
Asp Pro Glu Met Gly Gly Lys Pro Gln Arg Arg Lys Asn Pro Gln Glu
                405                 410                 415
Gly Leu Tyr Asn Glu Leu Gln Lys Asp Lys Met Ala Glu Ala Tyr Ser
            420                 425                 430
Glu Ile Gly Met Lys Gly Glu Arg Arg Arg Gly Lys Gly His Asp Gly
        435                 440                 445
Leu Tyr Gln Gly Leu Ser Thr Ala Thr Lys Asp Thr Tyr Asp Ala Leu
    450                 455                 460
His Met Gln Ala Leu Pro Pro Arg
465                 470

<210> 77
<211> 224
<212> PRT
<213> Artificial Sequence

<220>
<223> Chimeric receptor

<400> 77
Thr Thr Thr Pro Ala Pro Arg Pro Pro Thr Pro Ala Pro Thr Ile Ala
1               5                   10                  15
Ser Gln Pro Leu Ser Leu Arg Pro Glu Ala Cys Arg Pro Ala Ala Gly
            20                  25                  30
Gly Ala Val His Thr Arg Gly Leu Asp Phe Ala Cys Asp Ile Tyr Ile
        35                  40                  45
Trp Ala Pro Leu Ala Gly Thr Cys Gly Val Leu Leu Leu Ser Leu Val
    50                  55                  60
Ile Thr Leu Tyr Cys Lys Arg Gly Arg Lys Lys Leu Leu Tyr Ile Phe
65                  70                  75                  80
Lys Gln Pro Phe Met Arg Pro Val Gln Thr Thr Gln Glu Glu Asp Gly
                85                  90                  95
Cys Ser Cys Arg Phe Pro Glu Glu Glu Glu Gly Gly Cys Glu Leu Arg
            100                 105                 110
Val Lys Phe Ser Arg Ser Ala Asp Ala Pro Ala Tyr Gln Gln Gly Gln
        115                 120                 125
Asn Gln Leu Tyr Asn Glu Leu Asn Leu Gly Arg Arg Glu Glu Tyr Asp
    130                 135                 140
Val Leu Asp Lys Arg Arg Gly Arg Asp Pro Glu Met Gly Gly Lys Pro
145                 150                 155                 160
Gln Arg Arg Lys Asn Pro Gln Glu Gly Leu Tyr Asn Glu Leu Gln Lys
                165                 170                 175
Asp Lys Met Ala Glu Ala Tyr Ser Glu Ile Gly Met Lys Gly Glu Arg
            180                 185                 190
Arg Arg Gly Lys Gly His Asp Gly Leu Tyr Gln Gly Leu Ser Thr Ala
        195                 200                 205
Thr Lys Asp Thr Tyr Asp Ala Leu His Met Gln Ala Leu Pro Pro Arg
    210                 215                 220

<210> 78
<211> 237
<212> PRT
<213> Artificial Sequence

<220>
<223> Chimeric receptor

<400> 78
Asn Trp Ser His Pro Gln Phe Glu Lys Met His Thr Thr Thr Pro Ala
1               5                   10                  15
Pro Arg Pro Pro Thr Pro Ala Pro Thr Ile Ala Ser Gln Pro Leu Ser
            20                  25                  30
Leu Arg Pro Glu Ala Cys Arg Pro Ala Ala Gly Gly Ala Val His Thr
        35                  40                  45
Arg Gly Leu Asp Phe Ala Cys Asp Ile Tyr Ile Trp Ala Pro Leu Ala
    50                  55                  60
Gly Thr Cys Gly Val Leu Leu Leu Ser Leu Val Ile Thr Leu Tyr Cys
65                  70                  75                  80
Lys Arg Gly Arg Lys Lys Leu Leu Tyr Ile Phe Lys Gln Pro Phe Met
                85                  90                  95
Arg Pro Val Gln Thr Thr Gln Glu Glu Asp Gly Cys Ser Cys Arg Phe
            100                 105                 110
Pro Glu Glu Glu Glu Gly Gly Cys Glu Leu Thr Arg Arg Val Lys Phe
        115                 120                 125
Ser Arg Ser Ala Asp Ala Pro Ala Tyr Gln Gln Gly Gln Asn Gln Leu
    130                 135                 140
Tyr Asn Glu Leu Asn Leu Gly Arg Arg Glu Glu Tyr Asp Val Leu Asp
145                 150                 155                 160
Lys Arg Arg Gly Arg Asp Pro Glu Met Gly Gly Lys Pro Gln Arg Arg
                165                 170                 175
Lys Asn Pro Gln Glu Gly Leu Tyr Asn Glu Leu Gln Lys Asp Lys Met
            180                 185                 190
Ala Glu Ala Tyr Ser Glu Ile Gly Met Lys Gly Glu Arg Arg Arg Gly
        195                 200                 205
Lys Gly His Asp Gly Leu Tyr Gln Gly Leu Ser Thr Ala Thr Lys Asp
    210                 215                 220
Thr Tyr Asp Ala Leu His Met Gln Ala Leu Pro Pro Arg
225                 230                 235

<210> 79
<211> 227
<212> PRT
<213> Artificial Sequence

<220>
<223> Chimeric receptor

<400> 79
Met His Thr Thr Thr Pro Ala Pro Arg Pro Pro Thr Pro Ala Pro Thr
1               5                   10                  15
Ile Ala Ser Gln Pro Leu Ser Leu Arg Pro Glu Ala Cys Arg Pro Ala
            20                  25                  30
Ala Gly Gly Ala Val His Thr Arg Gly Leu Asp Phe Ala Cys Asp Ile
        35                  40                  45
Tyr Ile Trp Ala Pro Leu Ala Gly Thr Cys Gly Val Leu Leu Leu Ser
    50                  55                  60
Leu Val Ile Thr Leu Tyr Cys Arg Ser Lys Arg Ser Arg Leu Leu His
65                  70                  75                  80
Ser Asp Tyr Met Asn Met Thr Pro Arg Arg Pro Gly Pro Thr Arg Lys

                85                  90                  95
His Tyr Gln Pro Tyr Ala Pro Pro Arg Asp Phe Ala Ala Tyr Arg Ser
            100                 105                 110
Thr Arg Arg Val Lys Phe Ser Arg Ser Ala Asp Ala Pro Ala Tyr Gln
        115                 120                 125
Gln Gly Gln Asn Gln Leu Tyr Asn Glu Leu Asn Leu Gly Arg Arg Glu
    130                 135                 140
Glu Tyr Asp Val Leu Asp Lys Arg Arg Gly Arg Asp Pro Glu Met Gly
145                 150                 155                 160
Gly Lys Pro Gln Arg Arg Lys Asn Pro Gln Glu Gly Leu Tyr Asn Glu
                165                 170                 175
Leu Gln Lys Asp Lys Met Ala Glu Ala Tyr Ser Glu Ile Gly Met Lys
            180                 185                 190
Gly Glu Arg Arg Arg Gly Lys Gly His Asp Gly Leu Tyr Gln Gly Leu
        195                 200                 205
Ser Thr Ala Thr Lys Asp Thr Tyr Asp Ala Leu His Met Gln Ala Leu
    210                 215                 220
Pro Pro Arg
225

<210> 80
<211> 502
<212> PRT
<213> Artificial Sequence

<220>
<223> Chimeric receptor

<400> 80
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Ser Thr Ser Gly Ser Gly Lys Pro Gly Ser Gly Glu Gly Ser Thr
    130                 135                 140
Lys Gly Glu Val Lys Leu Gln Glu Ser Gly Pro Gly Leu Val Ala Pro
145                 150                 155                 160
Ser Gln Ser Leu Ser Val Thr Cys Thr Val Ser Gly Val Ser Leu Pro
                165                 170                 175
Asp Tyr Gly Val Ser Trp Ile Arg Gln Pro Pro Arg Lys Gly Leu Glu
            180                 185                 190
Trp Leu Gly Val Ile Trp Gly Ser Glu Thr Thr Tyr Tyr Asn Ser Ala
        195                 200                 205
Leu Lys Ser Arg Leu Thr Ile Ile Lys Asp Asn Ser Lys Ser Gln Val
    210                 215                 220
Phe Leu Lys Met Asn Ser Leu Gln Thr Asp Asp Thr Ala Ile Tyr Tyr
225                 230                 235                 240
Cys Ala Lys His Tyr Tyr Tyr Gly Gly Ser Tyr Ala Met Asp Tyr Trp
                245                 250                 255
Gly Gln Gly Thr Ser Val Thr Val Ser Ser Asn Trp Ser His Pro Gln

260                 265                 270
Phe Glu Lys Met His Thr Thr Thr Pro Ala Pro Arg Pro Pro Thr Pro
        275                 280                 285
Ala Pro Thr Ile Ala Ser Gln Pro Leu Ser Leu Arg Pro Glu Ala Cys
    290                 295                 300
Arg Pro Ala Ala Gly Gly Ala Val His Thr Arg Gly Leu Asp Phe Ala
305                 310                 315                 320
Cys Asp Ile Tyr Ile Trp Ala Pro Leu Ala Gly Thr Cys Gly Val Leu
                325                 330                 335
Leu Leu Ser Leu Val Ile Thr Leu Tyr Cys Arg Ser Lys Arg Ser Arg
            340                 345                 350
Leu Leu His Ser Asp Tyr Met Asn Met Thr Pro Arg Arg Pro Gly Pro
        355                 360                 365
Thr Arg Lys His Tyr Gln Pro Tyr Ala Pro Pro Arg Asp Phe Ala Ala
    370                 375                 380
Tyr Arg Ser Thr Arg Arg Val Lys Phe Ser Arg Ser Ala Asp Ala Pro
385                 390                 395                 400
Ala Tyr Gln Gln Gly Gln Asn Gln Leu Tyr Asn Glu Leu Asn Leu Gly
                405                 410                 415
Arg Arg Glu Glu Tyr Asp Val Leu Asp Lys Arg Arg Gly Arg Asp Pro
            420                 425                 430
Glu Met Gly Gly Lys Pro Gln Arg Arg Lys Asn Pro Gln Glu Gly Leu
        435                 440                 445
Tyr Asn Glu Leu Gln Lys Asp Lys Met Ala Glu Ala Tyr Ser Glu Ile
    450                 455                 460
Gly Met Lys Gly Glu Arg Arg Arg Gly Lys Gly His Asp Gly Leu Tyr
465                 470                 475                 480
Gln Gly Leu Ser Thr Ala Thr Lys Asp Thr Tyr Asp Ala Leu His Met
                485                 490                 495
Gln Ala Leu Pro Pro Arg
            500

<210> 81
<211> 481
<212> PRT
<213> Artificial Sequence

<220>
<223> Chimeric receptor

<400> 81
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Ser Thr Ser Gly
            100                 105                 110
Ser Gly Lys Pro Gly Ser Gly Glu Gly Ser Thr Lys Gly Glu Val Lys
        115                 120                 125
Leu Gln Glu Ser Gly Pro Gly Leu Val Ala Pro Ser Gln Ser Leu Ser
    130                 135                 140
Val Thr Cys Thr Val Ser Gly Val Ser Leu Pro Asp Tyr Gly Val Ser
145                 150                 155                 160
Trp Ile Arg Gln Pro Pro Arg Lys Gly Leu Glu Trp Leu Gly Val Ile

                165                 170                 175
Trp Gly Ser Glu Thr Thr Tyr Tyr Asn Ser Ala Leu Lys Ser Arg Leu
            180                 185                 190
Thr Ile Ile Lys Asp Asn Ser Lys Ser Gln Val Phe Leu Lys Met Asn
        195                 200                 205
Ser Leu Gln Thr Asp Asp Thr Ala Ile Tyr Tyr Cys Ala Lys His Tyr
    210                 215                 220
Tyr Tyr Gly Gly Ser Tyr Ala Met Asp Tyr Trp Gly Gln Gly Thr Ser
225                 230                 235                 240
Val Thr Val Ser Ser Asn Trp Ser His Pro Gln Phe Glu Lys Met His
                245                 250                 255
Thr Thr Thr Pro Ala Pro Arg Pro Pro Thr Pro Ala Pro Thr Ile Ala
            260                 265                 270
Ser Gln Pro Leu Ser Leu Arg Pro Glu Ala Cys Arg Pro Ala Ala Gly
        275                 280                 285
Gly Ala Val His Thr Arg Gly Leu Asp Phe Ala Cys Asp Ile Tyr Ile
    290                 295                 300
Trp Ala Pro Leu Ala Gly Thr Cys Gly Val Leu Leu Leu Ser Leu Val
305                 310                 315                 320
Ile Thr Leu Tyr Cys Arg Ser Lys Arg Ser Arg Leu Leu His Ser Asp
                325                 330                 335
Tyr Met Asn Met Thr Pro Arg Arg Pro Gly Pro Thr Arg Lys His Tyr
            340                 345                 350
Gln Pro Tyr Ala Pro Pro Arg Asp Phe Ala Ala Tyr Arg Ser Thr Arg
        355                 360                 365
Arg Val Lys Phe Ser Arg Ser Ala Asp Ala Pro Ala Tyr Gln Gln Gly
    370                 375                 380
Gln Asn Gln Leu Tyr Asn Glu Leu Asn Leu Gly Arg Arg Glu Glu Tyr
385                 390                 395                 400
Asp Val Leu Asp Lys Arg Arg Gly Arg Asp Pro Glu Met Gly Gly Lys
                405                 410                 415
Pro Gln Arg Arg Lys Asn Pro Gln Glu Gly Leu Tyr Asn Glu Leu Gln
            420                 425                 430
Lys Asp Lys Met Ala Glu Ala Tyr Ser Glu Ile Gly Met Lys Gly Glu
        435                 440                 445
Arg Arg Arg Gly Lys Gly His Asp Gly Leu Tyr Gln Gly Leu Ser Thr
    450                 455                 460
Ala Thr Lys Asp Thr Tyr Asp Ala Leu His Met Gln Ala Leu Pro Pro
465                 470                 475                 480
Arg

<210> 82
<211> 505
<212> PRT
<213> Artificial Sequence

<220>
<223> Chimeric receptor

<400> 82
Met Ala Leu Pro Val Thr Ala Leu Leu Leu Pro Leu Ala Leu Leu Leu
1               5                   10                  15
His Ala Ala Arg Pro Ser Gln Val Gln Leu Gln Gln Pro Gly Ala Glu
            20                  25                  30
Leu Val Lys Pro Gly Ala Ser Val Lys Met Ser Cys Lys Ala Ser Gly
        35                  40                  45
Tyr Thr Phe Thr Ser Tyr Asn Met His Trp Val Lys Gln Thr Pro Gly
    50                  55                  60
Arg Gly Leu Glu Trp Ile Gly Ala Ile Tyr Pro Gly Asn Gly Asp Thr
65                  70                  75                  80
Ser Tyr Asn Gln Lys Phe Lys Gly Lys Ala Thr Leu Thr Ala Asp Lys

                    85                          90                          95
Ser Ser Ser Thr Ala Tyr Met Gln Leu Ser Ser Leu Thr Ser Glu Asp
                100                         105                         110
Ser Ala Val Tyr Tyr Cys Ala Arg Ser Thr Tyr Tyr Gly Gly Asp Trp
            115                         120                         125
Tyr Phe Asn Val Trp Gly Ala Gly Thr Thr Val Thr Val Ser Ser Gly
    130                         135                         140
Gly Gly Gly Ser Gly Gly Gly Gly Ser Gly Gly Gly Gly Ser Gln Ile
145                         150                         155                         160
Val Leu Ser Gln Ser Pro Ala Ile Leu Ser Ala Ser Pro Gly Glu Lys
                165                         170                         175
Val Thr Met Thr Cys Arg Ala Ser Ser Ser Val Ser Tyr Ile His Trp
                180                         185                         190
Phe Gln Gln Lys Pro Gly Ser Ser Pro Lys Pro Trp Ile Tyr Ala Thr
            195                         200                         205
Ser Asn Leu Ala Ser Gly Val Pro Val Arg Phe Ser Gly Ser Gly Ser
    210                         215                         220
Gly Thr Ser Tyr Ser Leu Thr Ile Ser Arg Val Glu Ala Glu Asp Ala
225                         230                         235                         240
Ala Thr Tyr Tyr Cys Gln Gln Trp Thr Ser Asn Pro Pro Thr Phe Gly
                245                         250                         255
Gly Gly Thr Lys Leu Glu Ile Lys Arg Ser Ala Trp Ser His Pro Gln
            260                         265                         270
Phe Glu Lys Ser Gly Met His Thr Thr Thr Pro Ala Pro Arg Pro Pro
        275                         280                         285
Thr Pro Ala Pro Thr Ile Ala Ser Gln Pro Leu Ser Leu Arg Pro Glu
    290                         295                         300
Ala Cys Arg Pro Ala Ala Gly Gly Ala Val His Thr Arg Gly Leu Asp
305                         310                         315                         320
Phe Ala Cys Asp Ile Tyr Ile Trp Ala Pro Leu Ala Gly Thr Cys Gly
                325                         330                         335
Val Leu Leu Leu Ser Leu Val Ile Thr Leu Tyr Cys Lys Arg Gly Arg
            340                         345                         350
Lys Lys Leu Leu Tyr Ile Phe Lys Gln Pro Phe Met Arg Pro Val Gln
        355                         360                         365
Thr Thr Gln Glu Glu Asp Gly Cys Ser Cys Arg Phe Pro Glu Glu Glu
    370                         375                         380
Glu Gly Gly Cys Glu Leu Thr Arg Arg Val Lys Phe Ser Arg Ser Ala
385                         390                         395                         400
Asp Ala Pro Ala Tyr Gln Gln Gly Gln Asn Gln Leu Tyr Asn Glu Leu
                405                         410                         415
Asn Leu Gly Arg Arg Glu Glu Tyr Asp Val Leu Asp Lys Arg Arg Gly
            420                         425                         430
Arg Asp Pro Glu Met Gly Gly Lys Pro Gln Arg Arg Lys Asn Pro Gln
        435                         440                         445
Glu Gly Leu Tyr Asn Glu Leu Gln Lys Asp Lys Met Ala Glu Ala Tyr
    450                         455                         460
Ser Glu Ile Gly Met Lys Gly Glu Arg Arg Arg Gly Lys Gly His Asp
465                         470                         475                         480
Gly Leu Tyr Gln Gly Leu Ser Thr Ala Thr Lys Asp Thr Tyr Asp Ala
                485                         490                         495
Leu His Met Gln Ala Leu Pro Pro Arg
            500                         505

<210> 83
<211> 484
<212> PRT
<213> Artificial Sequence

<220>
<223> Chimeric receptor

<400> 83
Ser Gln Val Gln Leu Gln Gln Pro Gly Ala Glu Leu Val Lys Pro Gly
1               5                   10                  15
Ala Ser Val Lys Met Ser Cys Lys Ala Ser Gly Tyr Thr Phe Thr Ser
            20                  25                  30
Tyr Asn Met His Trp Val Lys Gln Thr Pro Gly Arg Gly Leu Glu Trp
        35                  40                  45
Ile Gly Ala Ile Tyr Pro Gly Asn Gly Asp Thr Ser Tyr Asn Gln Lys
    50                  55                  60
Phe Lys Gly Lys Ala Thr Leu Thr Ala Asp Lys Ser Ser Ser Thr Ala
65                  70                  75                  80
Tyr Met Gln Leu Ser Ser Leu Thr Ser Glu Asp Ser Ala Val Tyr Tyr
                85                  90                  95
Cys Ala Arg Ser Thr Tyr Tyr Gly Gly Asp Trp Tyr Phe Asn Val Trp
            100                 105                 110
Gly Ala Gly Thr Thr Val Thr Val Ser Ser Gly Gly Gly Gly Ser Gly
        115                 120                 125
Gly Gly Gly Ser Gly Gly Gly Gly Ser Gln Ile Val Leu Ser Gln Ser
    130                 135                 140
Pro Ala Ile Leu Ser Ala Ser Pro Gly Glu Lys Val Thr Met Thr Cys
145                 150                 155                 160
Arg Ala Ser Ser Ser Val Ser Tyr Ile His Trp Phe Gln Gln Lys Pro
                165                 170                 175
Gly Ser Ser Pro Lys Pro Trp Ile Tyr Ala Thr Ser Asn Leu Ala Ser
            180                 185                 190
Gly Val Pro Val Arg Phe Ser Gly Ser Gly Ser Gly Thr Ser Tyr Ser
        195                 200                 205
Leu Thr Ile Ser Arg Val Glu Ala Glu Asp Ala Ala Thr Tyr Tyr Cys
    210                 215                 220
Gln Gln Trp Thr Ser Asn Pro Pro Thr Phe Gly Gly Gly Thr Lys Leu
225                 230                 235                 240
Glu Ile Lys Arg Ser Ala Trp Ser His Pro Gln Phe Glu Lys Ser Gly
                245                 250                 255
Met His Thr Thr Thr Pro Ala Pro Arg Pro Pro Thr Pro Ala Pro Thr
            260                 265                 270
Ile Ala Ser Gln Pro Leu Ser Leu Arg Pro Glu Ala Cys Arg Pro Ala
        275                 280                 285
Ala Gly Gly Ala Val His Thr Arg Gly Leu Asp Phe Ala Cys Asp Ile
    290                 295                 300
Tyr Ile Trp Ala Pro Leu Ala Gly Thr Cys Gly Val Leu Leu Leu Ser
305                 310                 315                 320
Leu Val Ile Thr Leu Tyr Cys Lys Arg Gly Arg Lys Lys Leu Leu Tyr
                325                 330                 335
Ile Phe Lys Gln Pro Phe Met Arg Pro Val Gln Thr Thr Gln Glu Glu
            340                 345                 350
Asp Gly Cys Ser Cys Arg Phe Pro Glu Glu Glu Glu Gly Gly Cys Glu
        355                 360                 365
Leu Thr Arg Arg Val Lys Phe Ser Arg Ser Ala Asp Ala Pro Ala Tyr
    370                 375                 380
Gln Gln Gly Gln Asn Gln Leu Tyr Asn Glu Leu Asn Leu Gly Arg Arg
385                 390                 395                 400
Glu Glu Tyr Asp Val Leu Asp Lys Arg Arg Gly Arg Asp Pro Glu Met
                405                 410                 415
Gly Gly Lys Pro Gln Arg Arg Lys Asn Pro Gln Glu Gly Leu Tyr Asn
            420                 425                 430
Glu Leu Gln Lys Asp Lys Met Ala Glu Ala Tyr Ser Glu Ile Gly Met
        435                 440                 445
Lys Gly Glu Arg Arg Arg Gly Lys Gly His Asp Gly Leu Tyr Gln Gly
    450                 455                 460
Leu Ser Thr Ala Thr Lys Asp Thr Tyr Asp Ala Leu His Met Gln Ala
465                 470                 475                 480
Leu Pro Pro Arg

<210> 84
<211> 510
<212> PRT
<213> Artificial Sequence

<220>
<223> Chimeric receptor

<400> 84
Met Ala Leu Pro Val Thr Ala Leu Leu Leu Pro Leu Ala Leu Leu Leu
1               5                   10                  15
His Ala Ala Arg Pro Ser Gln Val Gln Leu Val Gln Ser Gly Ala Glu
            20                  25                  30
Leu Val Lys Pro Gly Ala Ser Val Lys Met Ser Cys Lys Ala Ser Gly
        35                  40                  45
Tyr Thr Phe Thr Ser Tyr Asn Met His Trp Val Lys Gln Thr Pro Gly
    50                  55                  60
Gln Gly Leu Glu Trp Ile Gly Ala Ile Tyr Pro Gly Asn Gly Asp Thr
65                  70                  75                  80
Ser Tyr Asn Gln Lys Phe Lys Gly Lys Ala Thr Leu Thr Ala Asp Lys
                85                  90                  95
Ser Ser Ser Thr Ala Tyr Met Gln Leu Ser Ser Leu Thr Ser Glu Asp
            100                 105                 110
Ser Ala Val Tyr Tyr Cys Ala Arg Ala Gln Leu Arg Pro Asn Tyr Trp
        115                 120                 125
Tyr Phe Asp Val Trp Gly Ala Gly Thr Thr Val Thr Val Ser Lys Ile
    130                 135                 140
Ser Gly Gly Gly Gly Ser Gly Gly Gly Gly Ser Gly Gly Gly Gly Ser
145                 150                 155                 160
Gly Gly Ser Ser Asp Ile Val Leu Ser Gln Ser Pro Ala Ile Leu Ser
                165                 170                 175
Ala Ser Pro Gly Glu Lys Val Thr Met Thr Cys Arg Ala Ser Ser Ser
            180                 185                 190
Val Ser Tyr Met His Trp Tyr Gln Gln Lys Pro Gly Ser Ser Pro Lys
        195                 200                 205
Pro Trp Ile Tyr Ala Thr Ser Asn Leu Ala Ser Gly Val Pro Ala Arg
    210                 215                 220
Phe Ser Gly Ser Gly Ser Gly Thr Ser Tyr Ser Leu Thr Ile Ser Arg
225                 230                 235                 240
Val Glu Ala Glu Asp Ala Ala Thr Tyr Tyr Cys Gln Gln Trp Ile Ser
                245                 250                 255
Asn Pro Pro Thr Phe Gly Ala Gly Thr Lys Leu Glu Leu Lys Ser Ala
            260                 265                 270
Trp Ser His Pro Gln Phe Glu Lys Ser Gly Met His Thr Thr Thr Pro
        275                 280                 285
Ala Pro Arg Pro Pro Thr Pro Ala Pro Thr Ile Ala Ser Gln Pro Leu
    290                 295                 300
Ser Leu Arg Pro Glu Ala Cys Arg Pro Ala Ala Gly Gly Ala Val His
305                 310                 315                 320
Thr Arg Gly Leu Asp Phe Ala Cys Asp Ile Tyr Ile Trp Ala Pro Leu
                325                 330                 335
Ala Gly Thr Cys Gly Val Leu Leu Leu Ser Leu Val Ile Thr Leu Tyr
            340                 345                 350
Cys Lys Arg Gly Arg Lys Lys Leu Leu Tyr Ile Phe Lys Gln Pro Phe
        355                 360                 365
Met Arg Pro Val Gln Thr Thr Gln Glu Glu Asp Gly Cys Ser Cys Arg
    370                 375                 380
Phe Pro Glu Glu Glu Glu Gly Gly Cys Glu Leu Thr Arg Arg Val Lys
385                 390                 395                 400
Phe Ser Arg Ser Ala Asp Ala Pro Ala Tyr Gln Gln Gly Gln Asn Gln
                405                 410                 415

Leu Tyr Asn Glu Leu Asn Leu Gly Arg Arg Glu Glu Tyr Asp Val Leu
                420                 425                 430
Asp Lys Arg Arg Gly Arg Asp Pro Glu Met Gly Gly Lys Pro Gln Arg
            435                 440                 445
Arg Lys Asn Pro Gln Glu Gly Leu Tyr Asn Glu Leu Gln Lys Asp Lys
        450                 455                 460
Met Ala Glu Ala Tyr Ser Glu Ile Gly Met Lys Gly Glu Arg Arg Arg
465                 470                 475                 480
Gly Lys Gly His Asp Gly Leu Tyr Gln Gly Leu Ser Thr Ala Thr Lys
                485                 490                 495
Asp Thr Tyr Asp Ala Leu His Met Gln Ala Leu Pro Pro Arg
                500                 505                 510

<210> 85
<211> 489
<212> PRT
<213> Artificial Sequence

<220>
<223> Chimeric receptor

<400> 85
Ser Gln Val Gln Leu Val Gln Ser Gly Ala Glu Leu Val Lys Pro Gly
1               5                   10                  15
Ala Ser Val Lys Met Ser Cys Lys Ala Ser Gly Tyr Thr Phe Thr Ser
            20                  25                  30
Tyr Asn Met His Trp Val Lys Gln Thr Pro Gly Gln Gly Leu Glu Trp
        35                  40                  45
Ile Gly Ala Ile Tyr Pro Gly Asn Gly Asp Thr Ser Tyr Asn Gln Lys
    50                  55                  60
Phe Lys Gly Lys Ala Thr Leu Thr Ala Asp Lys Ser Ser Ser Thr Ala
65                  70                  75                  80
Tyr Met Gln Leu Ser Ser Leu Thr Ser Glu Asp Ser Ala Val Tyr Tyr
                85                  90                  95
Cys Ala Arg Ala Gln Leu Arg Pro Asn Tyr Trp Tyr Phe Asp Val Trp
            100                 105                 110
Gly Ala Gly Thr Thr Val Thr Val Ser Lys Ile Ser Gly Gly Gly Gly
        115                 120                 125
Ser Gly Gly Gly Gly Ser Gly Gly Gly Gly Ser Gly Gly Ser Ser Asp
    130                 135                 140
Ile Val Leu Ser Gln Ser Pro Ala Ile Leu Ser Ala Ser Pro Gly Glu
145                 150                 155                 160
Lys Val Thr Met Thr Cys Arg Ala Ser Ser Ser Val Ser Tyr Met His
                165                 170                 175
Trp Tyr Gln Gln Lys Pro Gly Ser Ser Pro Lys Pro Trp Ile Tyr Ala
            180                 185                 190
Thr Ser Asn Leu Ala Ser Gly Val Pro Ala Arg Phe Ser Gly Ser Gly
        195                 200                 205
Ser Gly Thr Ser Tyr Ser Leu Thr Ile Ser Arg Val Glu Ala Glu Asp
    210                 215                 220
Ala Ala Thr Tyr Tyr Cys Gln Gln Trp Ile Ser Asn Pro Pro Thr Phe
225                 230                 235                 240
Gly Ala Gly Thr Lys Leu Glu Leu Lys Ser Ala Trp Ser His Pro Gln
                245                 250                 255
Phe Glu Lys Ser Gly Met His Thr Thr Thr Pro Ala Pro Arg Pro Pro
            260                 265                 270
Thr Pro Ala Pro Thr Ile Ala Ser Gln Pro Leu Ser Leu Arg Pro Glu
        275                 280                 285
Ala Cys Arg Pro Ala Ala Gly Gly Ala Val His Thr Arg Gly Leu Asp
    290                 295                 300
Phe Ala Cys Asp Ile Tyr Ile Trp Ala Pro Leu Ala Gly Thr Cys Gly
305                 310                 315                 320

Val Leu Leu Leu Ser Leu Val Ile Thr Leu Tyr Cys Lys Arg Gly Arg
                325                 330                 335
Lys Lys Leu Leu Tyr Ile Phe Lys Gln Pro Phe Met Arg Pro Val Gln
            340                 345                 350
Thr Thr Gln Glu Glu Asp Gly Cys Ser Cys Arg Phe Pro Glu Glu Glu
        355                 360                 365
Glu Gly Gly Cys Glu Leu Thr Arg Arg Val Lys Phe Ser Arg Ser Ala
    370                 375                 380
Asp Ala Pro Ala Tyr Gln Gln Gly Gln Asn Gln Leu Tyr Asn Glu Leu
385                 390                 395                 400
Asn Leu Gly Arg Arg Glu Glu Tyr Asp Val Leu Asp Lys Arg Arg Gly
                405                 410                 415
Arg Asp Pro Glu Met Gly Gly Lys Pro Gln Arg Arg Lys Asn Pro Gln
            420                 425                 430
Glu Gly Leu Tyr Asn Glu Leu Gln Lys Asp Lys Met Ala Glu Ala Tyr
        435                 440                 445
Ser Glu Ile Gly Met Lys Gly Glu Arg Arg Arg Gly Lys Gly His Asp
    450                 455                 460
Gly Leu Tyr Gln Gly Leu Ser Thr Ala Thr Lys Asp Thr Tyr Asp Ala
465                 470                 475                 480
Leu His Met Gln Ala Leu Pro Pro Arg
                485

<210> 86
<211> 505
<212> PRT
<213> Artificial Sequence

<220>
<223> Chimeric receptor

<400> 86
Met Ala Leu Pro Val Thr Ala Leu Leu Leu Pro Leu Ala Leu Leu Leu
1               5                   10                  15
His Ala Ala Arg Pro Ser Gln Ile Val Leu Ser Gln Ser Pro Ala Ile
            20                  25                  30
Leu Ser Ala Ser Pro Gly Glu Lys Val Thr Met Thr Cys Arg Ala Ser
        35                  40                  45
Ser Ser Leu Ser Phe Met His Trp Tyr Gln Gln Lys Pro Gly Ser Ser
    50                  55                  60
Pro Lys Pro Trp Ile Tyr Ala Thr Ser Asn Leu Ala Ser Gly Val Pro
65                  70                  75                  80
Ala Arg Phe Ser Gly Ser Gly Ser Gly Thr Ser Tyr Ser Leu Thr Ile
                85                  90                  95
Ser Arg Val Glu Ala Glu Asp Ala Ala Thr Tyr Phe Cys His Gln Trp
            100                 105                 110
Ser Ser Asn Pro Leu Thr Phe Gly Ala Gly Thr Lys Leu Glu Leu Lys
        115                 120                 125
Arg Ser Gly Gly Gly Gly Ser Gly Gly Gly Gly Ser Gly Gly Gly Gly
    130                 135                 140
Ser Gly Ser Gln Val Gln Leu Arg Gln Pro Gly Ala Glu Leu Val Lys
145                 150                 155                 160
Pro Gly Ala Ser Val Lys Met Ser Cys Lys Ala Ser Gly Tyr Thr Phe
                165                 170                 175
Thr Ser Tyr Asn Met His Trp Val Lys Gln Thr Pro Gly Gln Gly Leu
            180                 185                 190
Glu Trp Ile Gly Ala Ile Tyr Pro Gly Asn Gly Asp Thr Ser Tyr Asn
        195                 200                 205
Gln Lys Phe Lys Gly Lys Ala Thr Leu Thr Ala Asp Lys Ser Ser Ser
    210                 215                 220
Thr Ala Tyr Met Gln Leu Ser Ser Leu Thr Ser Glu Asp Ser Ala Val
225                 230                 235                 240

Tyr Tyr Cys Ala Arg Ser His Tyr Gly Ser Asn Tyr Val Asp Tyr Trp
                245                 250                 255
Gly Gln Gly Thr Thr Leu Thr Val Ser Ser Ala Trp Ser His Pro Gln
            260                 265                 270
Phe Glu Lys Ser Gly Met His Thr Thr Thr Pro Ala Pro Arg Pro Pro
        275                 280                 285
Thr Pro Ala Pro Thr Ile Ala Ser Gln Pro Leu Ser Leu Arg Pro Glu
    290                 295                 300
Ala Cys Arg Pro Ala Ala Gly Gly Ala Val His Thr Arg Gly Leu Asp
305                 310                 315                 320
Phe Ala Cys Asp Ile Tyr Ile Trp Ala Pro Leu Ala Gly Thr Cys Gly
                325                 330                 335
Val Leu Leu Leu Ser Leu Val Ile Thr Leu Tyr Cys Lys Arg Gly Arg
            340                 345                 350
Lys Lys Leu Leu Tyr Ile Phe Lys Gln Pro Phe Met Arg Pro Val Gln
        355                 360                 365
Thr Thr Gln Glu Glu Asp Gly Cys Ser Cys Arg Phe Pro Glu Glu Glu
    370                 375                 380
Glu Gly Gly Cys Glu Leu Thr Arg Arg Val Lys Phe Ser Arg Ser Ala
385                 390                 395                 400
Asp Ala Pro Ala Tyr Gln Gln Gly Gln Asn Gln Leu Tyr Asn Glu Leu
                405                 410                 415
Asn Leu Gly Arg Arg Glu Glu Tyr Asp Val Leu Asp Lys Arg Arg Gly
            420                 425                 430
Arg Asp Pro Glu Met Gly Gly Lys Pro Gln Arg Arg Lys Asn Pro Gln
        435                 440                 445
Glu Gly Leu Tyr Asn Glu Leu Gln Lys Asp Lys Met Ala Glu Ala Tyr
    450                 455                 460
Ser Glu Ile Gly Met Lys Gly Glu Arg Arg Arg Gly Lys Gly His Asp
465                 470                 475                 480
Gly Leu Tyr Gln Gly Leu Ser Thr Ala Thr Lys Asp Thr Tyr Asp Ala
                485                 490                 495
Leu His Met Gln Ala Leu Pro Pro Arg
            500                 505

<210> 87
<211> 484
<212> PRT
<213> Artificial Sequence

<220>
<223> Chimeric receptor

<400> 87
Ser Gln Ile Val Leu Ser Gln Ser Pro Ala Ile Leu Ser Ala Ser Pro
1               5                   10                  15
Gly Glu Lys Val Thr Met Thr Cys Arg Ala Ser Ser Ser Leu Ser Phe
            20                  25                  30
Met His Trp Tyr Gln Gln Lys Pro Gly Ser Ser Pro Lys Pro Trp Ile
        35                  40                  45
Tyr Ala Thr Ser Asn Leu Ala Ser Gly Val Pro Ala Arg Phe Ser Gly
    50                  55                  60
Ser Gly Ser Gly Thr Ser Tyr Ser Leu Thr Ile Ser Arg Val Glu Ala
65                  70                  75                  80
Glu Asp Ala Ala Thr Tyr Phe Cys His Gln Trp Ser Ser Asn Pro Leu
                85                  90                  95
Thr Phe Gly Ala Gly Thr Lys Leu Glu Leu Lys Arg Ser Gly Gly Gly
            100                 105                 110
Gly Ser Gly Gly Gly Gly Ser Gly Gly Gly Gly Ser Gly Ser Gln Val
        115                 120                 125
Gln Leu Arg Gln Pro Gly Ala Glu Leu Val Lys Pro Gly Ala Ser Val
    130                 135                 140

Lys Met Ser Cys Lys Ala Ser Gly Tyr Thr Phe Thr Ser Tyr Asn Met
145                 150                 155                 160
His Trp Val Lys Gln Thr Pro Gly Gln Gly Leu Glu Trp Ile Gly Ala
                165                 170                 175
Ile Tyr Pro Gly Asn Gly Asp Thr Ser Tyr Asn Gln Lys Phe Lys Gly
            180                 185                 190
Lys Ala Thr Leu Thr Ala Asp Lys Ser Ser Ser Thr Ala Tyr Met Gln
        195                 200                 205
Leu Ser Ser Leu Thr Ser Glu Asp Ser Ala Val Tyr Tyr Cys Ala Arg
    210                 215                 220
Ser His Tyr Gly Ser Asn Tyr Val Asp Tyr Trp Gly Gln Gly Thr Thr
225                 230                 235                 240
Leu Thr Val Ser Ser Ala Trp Ser His Pro Gln Phe Glu Lys Ser Gly
                245                 250                 255
Met His Thr Thr Thr Pro Ala Pro Arg Pro Pro Thr Pro Ala Pro Thr
            260                 265                 270
Ile Ala Ser Gln Pro Leu Ser Leu Arg Pro Glu Ala Cys Arg Pro Ala
        275                 280                 285
Ala Gly Gly Ala Val His Thr Arg Gly Leu Asp Phe Ala Cys Asp Ile
    290                 295                 300
Tyr Ile Trp Ala Pro Leu Ala Gly Thr Cys Gly Val Leu Leu Leu Ser
305                 310                 315                 320
Leu Val Ile Thr Leu Tyr Cys Lys Arg Gly Arg Lys Lys Leu Leu Tyr
                325                 330                 335
Ile Phe Lys Gln Pro Phe Met Arg Pro Val Gln Thr Thr Gln Glu Glu
            340                 345                 350
Asp Gly Cys Ser Cys Arg Phe Pro Glu Glu Glu Glu Gly Gly Cys Glu
        355                 360                 365
Leu Thr Arg Arg Val Lys Phe Ser Arg Ser Ala Asp Ala Pro Ala Tyr
    370                 375                 380
Gln Gln Gly Gln Asn Gln Leu Tyr Asn Glu Leu Asn Leu Gly Arg Arg
385                 390                 395                 400
Glu Glu Tyr Asp Val Leu Asp Lys Arg Arg Gly Arg Asp Pro Glu Met
                405                 410                 415
Gly Gly Lys Pro Gln Arg Arg Lys Asn Pro Gln Glu Gly Leu Tyr Asn
            420                 425                 430
Glu Leu Gln Lys Asp Lys Met Ala Glu Ala Tyr Ser Glu Ile Gly Met
        435                 440                 445
Lys Gly Glu Arg Arg Arg Gly Lys Gly His Asp Gly Leu Tyr Gln Gly
    450                 455                 460
Leu Ser Thr Ala Thr Lys Asp Thr Tyr Asp Ala Leu His Met Gln Ala
465                 470                 475                 480
Leu Pro Pro Arg

## Description

### FIELD OF INVENTION

The present invention relates to the field of immunotherapy. In particular, the present invention relates to the use of a monospecific regulatory T cell population (monospecific Treg cell population) for treating autoantibody mediated autoimmune diseases, wherein said monospecific Treg cell population is cytotoxic for B cells. According to the invention, the cells of the monospecific Treg cell population of the invention express a chimeric receptor at its surface, wherein said chimeric receptor recognizes a B cell marker.

### BACKGROUND OF INVENTION

Autoimmune diseases are caused by an abnormal immune response to autoantigens resulting in damages or dysfunctions in tissues or organs. Two different categories of autoimmune diseases may be distinguished (although not exclusive): those caused by T cells, and those caused by B cells producing antibodies directed to autoantigens (autoantibodies).

Autoantibody mediated autoimmune diseases are currently treated by general immune suppression methods. A first example is apheresis, wherein total antibodies are removed from blood of a patient before re-administration of the remaining blood components. A second example is treatment with rituximab, an anti-CD20 monoclonal antibody. CD20 is primarily found on the surface of B cells. Administration of rituximab to a patient thus results in the elimination of B cells.

However, these general immune suppression methods present drawbacks associated with the complete elimination of antibodies or B cells, thus exposing for example the treated patient to increased risks of infection.

WO2015/168613 disclosed effector T cells expressing a chimeric autoantibody receptor (CAAR), wherein the expression of said CAAR redirects the cytotoxicity of effector T cells to B cells producing specific autoantibodies. The cells of WO2015/168613 thus allow specifically targeting autoreactive B cells, thereby avoiding the drawbacks of methods of the prior art.

However, using effector T cells in autoimmune disease patients, i.e. in patients with an over-reactive immune system, may present severe drawbacks, in particular due to the secretion by said cells of specific cytokines, including IFNγ or TNFα.

The Applicant herein provides evidences supporting the therapeutic use of at least one monospecific regulatory T cell population for treating autoantibody mediated autoimmune diseases. Indeed, the Applicant demonstrated that a monospecific regulatory T cell population, wherein the cells are genetically modified for expressing a chimeric receptor directed to B cell surface marker does present cytotoxicity for B cells, whereas they do not before genetic modification.

The present invention thus relates to the use of at least one monospecific regulatory T cell population expressing a chimeric receptor directed to a B cell surface marker and presenting cytotoxicity for B cells. The cells of the present invention thus present the advantage of both killing autoreactive B cells and providing a regulatory effect on T cells, in particular in autoimmune diseases with both B and T cells counterpart.

### SUMMARY

The present invention relates to a monospecific Treg cell population, wherein the Treg cells of the population comprise a chimeric receptor, wherein said chimeric receptor recognizes at least one B cell surface marker on B cells and wherein said Treg cells are cytotoxic for B cells.

In one embodiment, the chimeric receptor comprises (i) an extracellular B cell surface marker binding domain, (ii) optionally an extracellular hinge domain, (iii) optionally a transmembrane domain, and (iv) an intracellular signaling domain, and optionally the chimeric receptor further comprises a tag and/or a leader sequence.

In one embodiment, the cells of the monospecific Treg cell population are cytotoxic for B cells in the conditions of Test A.

In one embodiment, the cytotoxicity for B cells is chimeric receptor dependent and TCR independent.

In one embodiment, the B cell is a mature activated B cell.

In one embodiment, the at least one B cell surface marker is selected from CD 19, CD20, BCMA, IgM, IgA, IgG, IgE, IgD, CD1, CD5, CD21, CD22, CD23, CD24, CD25, CD27, CD30, CD38, CD40, CD78, CD80, CD138, CD319, PDL-2, CXCR3, CXCR4, CXCR5, CXCR6, Notch2, TLR4, IL-6, IL-10 and TGFβ, preferably CD19 or CD20.

In one embodiment, the at least one B cell surface marker is CD 19 and the chimeric receptor comprises an antibody directed to CD 19, or an antigen binding fragment thereof.

In one embodiment, the at least one B cell surface marker is CD20 and the chimeric receptor comprises an antibody directed to CD20, or an antigen binding fragment thereof.

The present invention further relates to a pharmaceutical composition comprising at least one monospecific Treg cell population as defined hereinabove and at least one pharmaceutically acceptable excipient or carrier.

The present invention further relates to a medicament comprising at least one monospecific Treg cell population as defined hereinabove.

The present invention further relates to a monospecific Treg cell population, a pharmaceutical composition or a medicament as defined hereinabove for treating an autoimmune disease in a subject in need thereof.

In one embodiment, said autoimmune disease is an autoantibody-mediated autoimmune disease.

In one embodiment, said autoimmune disease is selected from the group comprising bullous pemphigoid, lupus (including systemic lupus erythematosus (SLE), lupus nephritis (LN), discoid lupus, lupus erythematosus profundus, Chilbrain lupus erythematosus, tumidus lupus erythematosus, severe systemic lupus erythematosus, acute cutaneous lupus, chronic cutaneous lupus), multiple sclerosis, neuromyelitis optica (NMO), autoimmune limbic encephalitis (LE), Hashimoto's disease, N-methyl-D-aspartate receptor (NMDAR) encephalitis, autoimmune hemolytic anemia, pemphigus vulgaris, myasthenia gravis, Graves' disease, idiopathic thrombocytopenic purpura, Goodpasture's syndrome, rheumatoid arthritis, celiac disease, pernicious anemia, vitiligo, scleroderma, psoriasis, ulcerative colitis (UC), Crohn's disease, Sjogren's syndrome, Wegener granulomatosis, polymyositis, dermatomyositis, primary biliary cirrhosis, antiphospholipid syndrome, mixed connective tissue disease, Miller Fisher syndrome, Guillain- Barré syndrome, acute motor axonal neuropathy, autoimmune hepatitis, dermatitis herpetiformis, Churg-Strauss disease, microscopic polyangiitis, IgA nephropathy, vasculitis caused by ANCA and other ANCA associated diseases, acute rheumatic fever, pernicious anemia, type 1 diabetes (T1D), reactive arthritis (Reiter syndrome), membranous nephropathy, chronic inflammatory demyelinating polyneuropathy, thrombotic thrombocytopenic purpura, hyperviscosity in monoclonal gammopathies, hemolytic uremic syndrome (atypical, due to antibody to factor H), Wilson disease, fulminant, Lambert-Eaton myasthenic syndrome, RBC alloimmunization in pregnancy, mushroom poisoning, acute disseminated encephalomyelitis, hemolytic uremic syndrome (atypical, due to complement factor mutations), autoimmune hemolytic anemia (life-threatening cold agglutinin disease), myeloma cast nephropathy, post-transfusion purpura, autoimmune hemolytic anemia (warm autoimmune hemolytic anemia), hypertriglyceridemic pancreatitis, thyroid storm, stiff person syndrome, Hemolytic uremic syndrome (typical diarrhea-associated), immune thrombocytopenia, ABO-incompatible solid organ transplantation (SOT), cryoglobulinemia, heparin-induced thrombocytopenia, thyroid storm, chronic inflammatory demyelinating polyradiculoneuropathy, focal segmental glomerulosclerosis and fulminant hepatic failure.

In one embodiment, said autoimmune disease is selected from the group comprising bullous pemphigoid, lupus (including systemic lupus erythematosus (SLE), lupus nephritis (LN), discoid lupus, lupus erythematosus profundus, Chilbrain lupus erythematosus, tumidus lupus erythematosus, severe systemic lupus erythematosus, acute cutaneous lupus, chronic cutaneous lupus) and multiple sclerosis.

### DEFINITIONS

In the present invention, the following terms have the following meanings:
- The terms "a" and "an" refer to one or to more than one (i.e., to at least one) of the grammatical object of the article. By way of example, "an element" means one element or more than one element.
- The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.
- The term "monospecific", when applied to a regulatory T cell (Treg cell) population, refers to a population of Treg cells wherein each Treg cell of the population recognizes the same antigen, via the TCR molecule (this specificity may be referred to as TCR-monospecificity). In one embodiment, the monospecific population is monoclonal, and consequently all the cells of the population recognize the same epitope on the antigen. In another embodiment, the monospecific population is polyclonal, and consequently each cell of the population may recognize a different epitope on the antigen.

In one embodiment, the monospecific Treg cell population of the invention suppresses, inhibits or prevents excessive or unwanted inflammatory responses, such as, for example, autoimmunity or allergic reactions.

In one embodiment, the monospecific Treg cell population of the invention are capable of suppressive activity. In one embodiment, said suppressive activity is contact independent.

In one embodiment, the monospecific Treg cell population of the invention secretes IL-10 after activation. In one embodiment, the monospecific Treg cell population of the invention secretes an amount of IL-10 of at least about 50 pg/ml, 100 pg/ml, 200 pg/ml, 300 pg/ml, 400 pg/ml, 500 pg/mL or more, or at least about 1, 2, 4, 6, 8, 10, 12, 14, 16, 18, or 20 thousand pg IL-10/ml or more. The cytokine production of the monospecific Treg cell population is typically evaluated in cultures of cells after activation with polyclonal activators of T lymphocytes such as anti-CD3 + anti-CD28 antibodies or Interleukin-2, PMA + ionomycin. Alternatively, the cytokine production is evaluated in cultures of cells after activation with the antigen to which the monospecific Treg cell population is directed, wherein said antigen is presented by antigen presenting cells Usually, the monospecific Treg cell population is activated for about 48 hours and the cytokine production is evaluated 24 hours after activation.

In one embodiment, the suppressive activity of the monospecific Treg cell population is IL-10 dependent.

In one embodiment, the cells of the monospecific Treg cell population are CD4⁺CD25⁺.

In one embodiment, the cells of the monospecific Treg cell population are FoxP3⁻.

In one embodiment, the cells of the monospecific Treg cell population are CD127^{lo/-}.

In one embodiment, the cells of the monospecific Treg cell population are CTLA-4^{int}.

In one embodiment, the cells of the monospecific Treg cell population are CD39⁺.

In one embodiment, the cells of the monospecific Treg cell population are Helios^{int}.

In one embodiment, the cells of the monospecific Treg cell population are CD62L^{lo/-}.

In one embodiment, the cells of the monospecific Treg cell population are VLA4⁺.

In one embodiment, the cells of the monospecific Treg cell population are LFA1⁺.

In one embodiment, the cells of the monospecific Treg cell population are CD49b^{int}.

In one embodiment, the cells of the monospecific Treg cell population are TTGb7^{int}.

In one embodiment, the cells of the monospecific Treg cell population are PSGL-1⁺.

In one embodiment, the cells of the monospecific Treg cell population are ICOS⁺.

In one embodiment, the cells of the monospecific Treg cell population are GITR⁺.

In one embodiment, the cells of the monospecific Treg cell population are PD-1^{lo/-}.

In one embodiment, the cells of the monospecific Treg cell population are Perf^{lo/-}.

In one embodiment, the cells of the monospecific Treg cell population are CCR7^{lo/int}.

In one embodiment, the cells of the monospecific Treg cell population express Granzyme A and/or Granzyme B.

In one embodiment, the determination of the expression level of molecules is conducted by flow cytometry, immunofluorescence or image analysis, for example high content analysis. Preferably, the determination of the expression level of molecules is conducted by flow cytometry. In one embodiment, before conducting flow cytometry analysis, cells are fixed and permeabilized, thereby allowing detecting intracellular proteins.

In one embodiment, the determination of the expression level of a molecule in a cell population comprises determining the percentage of cells of the cell population expressing the molecule (i.e. cells "+" for the molecule). Preferably, said percentage of cells expressing the molecule is measured by FACS.

The terms "expressing (or +)" and "not expressing (or -)" are well known in the art and refer to the expression level of the cell marker of interest, in that the expression level of the cell marker corresponding to "+" is high or intermediate, also referred as "+/-", and the expression level of the cell marker corresponding to "-" is null.

The term "low" or "lo" or "lo/-" is well known in the art and refers to the expression level of the cell marker of interest, in that the expression level of the cell marker is low by comparison with the expression level of that cell marker in the population of cells being analyzed as a whole. More particularly, the term "lo" refers to a distinct population of cells that express the cell marker at a lower level than one or more other distinct population of cells.

The term "high" or "hi" or "bright" is well known in the art and refers to the expression level of the cell marker of interest, in that the expression level of the cell marker is high by comparison with the expression level of that cell marker in the population of cells being analyzed as a whole.

Generally, cells in the top 2, 3, 4, or 5% of staining intensity are designated "hi", with those falling in the top half of the population categorized as being "+". Those cells falling below 50%, of fluorescence intensity are designated as "lo" cells and below 5% as "-" cells.

The expression level of the cell marker of interest is determined by comparing the Median Fluorescence Intensity (MFI) of the cells from the cell population stained with fluorescently labeled antibody specific for this marker to the fluorescence intensity (FI) of the cells from the same cell population stained with fluorescently labeled antibody with an irrelevant specificity but with the same isotype, the same fluorescent probe and originated from the same specie (referred as Isotype control). The cells from the population stained with fluorescently labeled antibody specific for this marker and that show equivalent MFI or a lower MFI than the cells stained with the isotype controls are not expressing this marker and then are designated (-) or negative. The cells from the population stained with fluorescently labeled antibody specific for this marker and that show a MFI value superior to the cells stained with the isotype controls are expressing this marker and then are designated (+) or positive.

In one embodiment, the monospecific Treg cell population is obtained by *in vitro* differentiation of naive T cells.

In one embodiment, the monospecific Treg cell population comprises or consists of engineered Treg cells. In one embodiment, said engineered monospecific Treg cells population expresses a chimeric receptor (CR) as defined herein, wherein said chimeric receptor recognizes a ligand. In this embodiment, the monospecific Treg cell population is TCR-monospecific (i.e. all the Treg cells recognize the same antigen with their TCR) and CR-monospecific (i.e. all the Treg cells recognize the same antigen with the CR they express).
- The term "cytotoxic molecule" refers to a protein involved in the cytotoxic pathway, preferably to a protein of the serine protease family or to the granzyme family or to granulysin or to perforin. The granzyme family is a family of serine proteases that are released by cytoplasmic vesicles, which can be found for example within cytotoxic T cells and natural killer cells. In one embodiment, the protease from the granzyme family is selected from the group comprising granzyme A, granzyme B, granzyme M, granzyme K and granzyme H. Granulysin is a substance first described as released by cytotoxic T cells (CD8) when they are attached to infected body cells. Granulysin may create holes in a target cell membrane, but also induce apoptosis in target cells. Perforin is a cytolytic protein found in the secretory vesicles of Cytotoxic T lymphocytes (CTLs) and NK cells. Upon degranulation, perforin inserts itself into the target cell's plasma membrane, forming a pore.
- The term "chimeric receptor" refers to one polypeptide or to a set of polypeptides, typically two in the simplest embodiments, which when in an immune cell, provides the cell with specificity for a target ligand and with intracellular signal generation. In some embodiments, the set of polypeptides are contiguous with each other. In some embodiments, the chimeric receptor is a chimeric fusion protein comprising the set of polypeptides. In some embodiments, the set of polypeptides include a dimerization switch that, upon the presence of a dimerization molecule, can couple the polypeptides to one another, e.g., can couple a ligand binding domain to an intracellular signaling domain. In one embodiment, the chimeric receptor comprises an optional leader sequence at the amino-terminus (N-ter) of the chimeric receptor fusion protein. In one embodiment, the chimeric receptor further comprises a leader sequence at the N-terminus of the extracellular ligand binding domain, wherein the leader sequence is optionally cleaved from the ligand binding domain during cellular processing and localization of the chimeric receptor to the cellular membrane.
- The term "ligand" refers to a member of a pair ligand/receptor, and binds to the other member of the pair.
- The term "signaling domain" refers to the functional portion of a protein which acts by transmitting information within the cell to regulate cellular activity via defined signaling pathways by generating second messengers or functioning as effectors by responding to such messengers.
- The term "antibody", as used herein, refers to a protein, or polypeptide sequence derived from an immunoglobulin molecule which specifically binds with an antigen. Antibodies can be polyclonal or monoclonal, multiple or single chain, or intact immunoglobulins, and may be derived from natural sources or from recombinant sources. Antibodies can be multimers of immunoglobulin molecules, such as tetramers of immunoglobulin molecules.
- The term "antibody fragment" refers to at least one portion of an antibody, that retains the ability to specifically interact with (e.g., by binding, steric hindrance, stabilizing/destabilizing, spatial distribution) an epitope of an antigen. Examples of antibody fragments include, but are not limited to, Fab, Fab', F(ab')2, Fv fragments, scFv antibody fragments, disulfide-linked Fvs (sdFv), a Fd fragment consisting of the VH and CHI domains, linear antibodies, single domain antibodies such as sdAb (either VL or VH), camelid VHH domains, multi-specific antibodies formed from antibody fragments such as a bivalent fragment comprising two Fab fragments linked by a disulfide brudge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (see U.S. Patent No.: 6,703,199, which describes fibronectin polypeptide minibodies).
- The term "scFv" refers to a fusion protein comprising at least one antibody fragment comprising a variable region of a light chain and at least one antibody fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.
- The term "antibody heavy chain", refers to the larger of the two types of polypeptide chains present in antibody molecules in their naturally occurring conformations, and which normally determines the class to which the antibody belongs.
- The term "antibody light chain", refers to the smaller of the two types of polypeptide chains present in antibody molecules in their naturally occurring conformations. Kappa (K) and lambda (λ) light chains refer to the two major antibody light chain isotypes.
- The term "recombinant protein or peptide" refers to a protein or peptide (e.g. an antibody) which is generated using recombinant DNA technology, such as, for example, a protein or peptide (e.g. an antibody) expressed by a bacteriophage or yeast expression system. The term should also be construed to mean a protein or peptide (e.g. an antibody) which has been generated by the synthesis of a DNA molecule encoding the protein or peptide (e.g. the antibody) and which DNA molecule expresses a protein or peptide (e.g. an antibody), or an amino acid sequence specifying the protein or peptide (e.g. the antibody), wherein the DNA or amino acid sequence has been obtained using recombinant DNA or amino acid sequence technology which is available and well known in the art.
- The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequence or a partial nucleotide sequence encoding a protein that elicits an immune response therefore encodes an "antigen" as that term is used herein. Furthermore, one skilled in the art will understand that an antigen does not necessarily need to be encoded solely by a full-length nucleotide sequence of a gene. It is readily apparent that the present invention includes, but is not limited to, the use of partial nucleotide sequences of more than one gene and that these nucleotide sequences are arranged in various combinations to encode polypeptides that elicit the desired immune response. Moreover, a skilled artisan will understand that an antigen does not necessarily need to be encoded by a "gene" at all. It is readily apparent that an antigen can be synthesized or can be derived from a biological sample, or might be macromolecule besides a polypeptide. Such a biological sample can include, but is not limited to a tissue sample, a cell or a fluid with other biological components.
- The term "autologous" refers to any material derived from the same individual to whom it is later to be re-introduced.
- The term "allogeneic" refers to any material derived from a different individual of the same specie as the individual to whom the material is introduced. Two or more individuals are said to be allogeneic to one another when the genes at one or more loci are not identical. In some aspects, allogeneic material from individuals of the same species may be sufficiently unlike genetically to interact antigenically.
- "Derived from" as that term is used herein, indicates a relationship between a first and a second molecule. It generally refers to structural similarity between the first molecule and a second molecule and does not connote or include a process or source limitation on a first molecule that is derived from a second molecule. For example, in the case of an intracellular signaling domain that is derived from a CD3zeta molecule, the intracellular signaling domain retains sufficient CD3zeta structure such that is has the required function, namely, the ability to generate a signal under the appropriate conditions. It does not connote or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.
- The term "conservative sequence modifications" refers to amino acid modifications that do not significantly affect or alter the biologic function of the protein containing the amino acid sequence. Such conservative modifications include amino acid substitutions, additions and deletions. Modifications can be introduced into a protein by standard techniques known in the art, such as site-directed mutagenesis and PCR-mediated mutagenesis.
- Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a chimeric receptor of the invention can be replaced with other amino acid residues from the same side chain family and the altered chimeric receptor can be tested using the functional assays described herein.
- The term "stimulation", refers to a primary response induced by binding of a stimulatory molecule (e.g., a TCR/CD3 complex or chimeric receptor) with its cognate ligand thereby mediating a signal transduction event, such as, but not limited to, signal transduction via the TCR/CD3 complex or signal transduction via signaling domains of the chimeric receptor. Stimulation can mediate altered expression of certain molecules.
- The term "stimulatory molecule", refers to a molecule expressed by an immune cell (e.g., T cell, NK cell, B cell) that provides the cytoplasmic signaling sequence(s) that regulate activation of the immune cell in a stimulatory way for at least some aspect of the immune cell signaling pathway. In one aspect, the signal is a primary signal that is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM.
- The term "antigen presenting cell" or "APC" refers to an immune system cell such as an accessory cell (e.g., a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.
- An "intracellular signaling domain", as the term is used herein, refers to an intracellular portion of a molecule. The intracellular signaling domain generates a signal that promotes an immune effector function of the chimeric receptor containing cell. Examples of immune effector function in a chimeric receptor-T cell may include cytolytic activity and helper activity, including the secretion of cytokines.
- The term "zeta" or alternatively "zeta chain", "CD3-zeta" or "TCR-zeta" is defined as the protein provided as GenBank Acc. No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain, or functional derivatives thereof, that are sufficient to functionally transmit an initial signal necessary for T cell activation. In one embodiment, the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof.
- The term a "costimulatory molecule" refers to a cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that contribute to an efficient immune response. A costimulatory signaling domain can be the intracellular portion of a costimulatory molecule. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors.
- The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.
- Unless otherwise specified, a "nucleotide sequence encoding an amino acid sequence" includes all nucleotide sequences that are degenerate versions of each other and that encode the same amino acid sequence. The phrase "nucleotide sequence that encodes a protein or a RNA" may also include introns to the extent that the nucleotide sequence encoding the protein may in some version contain an intron(s).
- The terms "therapeutically effective amount" refer to an amount of cells of the monospecific Treg cell population or composition, as described herein effective to achieve a particular biological result. Thus, the terms "therapeutically effective amount" mean a level or amount of agent that is aimed at, without causing significant negative or adverse side effects to the target, (1) delaying or preventing the onset of the targeted disease or condition; (2) slowing down or stopping the progression, aggravation, or deterioration of one or more symptoms of the targeted disease or condition; (3) bringing about ameliorations of the symptoms of the targeted disease or condition; (4) reducing the severity or incidence of the targeted disease or condition; or (5) curing the targeted disease or condition. A therapeutically effective amount may be administered prior to the onset of the targeted disease or condition, for a prophylactic or preventive action. Alternatively, or additionally, the therapeutically effective amount may be administered after initiation of the targeted disease or condition, for a therapeutic action.

- The term "endogenous" refers to any material from or produced inside an organism, cell, tissue or system.
- The term "exogenous" refers to any material introduced from or produced outside an organism, cell, tissue or system.
- The term "expression" refers to the transcription and/or translation of a particular nucleotide sequence driven by a promoter.
- The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include non-plasmid and non-viral compounds which facilitate transfer of nucleic acid into cells, such as, for example, a poly lysine compound, liposome, and the like. Examples of viral transfer vectors include, but are not limited to, adenoviral vectors, adeno-associated virus vectors, retroviral vectors, lentiviral vectors, and the like.
- The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an *in vitro* expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.
- The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.
- The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic, include but are not limited to, the LENTIVECTOR® gene delivery technology from Oxford BioMedica, the LENTIMAX™ vector system from Lentigen and the like. Nonclinical types of lentiviral vectors are also available and would be known to one skilled in the art.
- The term "homology" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous. Thus, the term "homologous" or "identical", when used in a relationship between the sequences of two or more polypeptides or of two or more nucleic acid molecules, refers to the degree of sequence relatedness between polypeptides or nucleic acid molecules, as determined by the number of matches between strings of two or more amino acid or nucleotide residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.
- "Humanized" forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies and antibody fragments thereof are human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.
- "Fully human" refers to an immunoglobulin, such as an antibody or antibody fragment, where the whole molecule is of human origin or consists of an amino acid sequence identical to a human form of the antibody or immunoglobulin.
- The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or peptide can exist in substantially purified form, or can exist in a non-native environment such as, for example, a host cell. Typically, a preparation of isolated nucleic acid or peptide contains the nucleic acid or peptide at least about 80% pure, at least about 85% pure, at least about 90% pure, at least about 95% pure, greater than 95% pure, greater than 96% pure, greater than 97% pure, greater than 98% pure, or greater than 99% pure. An "isolated nucleic acid" is a nucleic acid that is substantially separated from other genome DNA sequences as well as proteins or complexes such as ribosomes and polymerases, which naturally accompany a native sequence. The term embraces a nucleic acid sequence that has been removed from its naturally occurring environment, and includes recombinant or cloned DNA isolates and chemically synthesized analogues or analogues biologically synthesized by heterologous systems. An "isolated polypeptide" is one that has been identified and separated and/or recovered from a component of its natural environment.
- In the context of the present invention, the following abbreviations for the commonly occurring nucleic acid bases are used. "A" refers to adenine, "C" refers to cytosine, "G" refers to guanine, "T" refers to thymine, and "U" refers to uracil.
- The term "operably linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.
- The term "nucleic acid" or "polynucleotide" refers to a polymer of nucleotides covalently linked by phosphodiester bonds, such as deoxyribonucleic acids (DNA) or ribonucleic acids (RNA), in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).
- The terms "peptide", "polypeptide", and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.
- The term "promoter" refers to a DNA sequence recognized by the synthetic machinery of the cell, or introduced synthetic machinery, required to initiate the specific transcription of a polynucleotide sequence.
- The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.
- The term "constitutive" promoter refers to a nucleotide sequence which, when operably linked with a polynucleotide which encodes or specifies a gene product, causes the gene product to be produced in a cell under most or all physiological conditions of the cell.
- The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.
- As used herein, a "5' cap" (also termed an RNA cap, an RNA 7-methylguanosine cap or an RNA m7G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.
- As used herein, *"in vitro* transcribed RNA" refers to RNA, preferably mRNA, that has been synthesized *in vitro.* Generally, the *in vitro* transcribed RNA is generated from an *in vitro* transcription vector. The *in vitro* transcription vector comprises a template that is used to generate the *in vitro* transcribed RNA.
- As used herein, a "poly(A)" is a series of adenosines monophosphate attached to the mRNA. In a preferred embodiment of a construct for transient expression, the polyA is between 50 and 5000 adenosines monophosphate, preferably greater than or equal to 64, more preferably greater than or equal to 100, most preferably greater than or equal to 300 or 400 adenosines monophosphate. Poly(A) sequences can be modified chemically or enzymatically to modulate mRNA functionality such as localization, stability or efficiency of translation.
- As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly (A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

- As used herein, "transient" refers to expression of a non-integrated transgene for a period of hours, days or weeks, wherein the period of time of expression is less than the period of time for expression of the gene if integrated into the genome or contained within a stable plasmid replicon in the host cell.
- As used herein, the terms "treat", "treatment" and "treating" refer to the reduction or amelioration of the progression, severity and/or duration of a targeted disease or condition, e.g. an autoimmune condition, or the amelioration of one or more symptoms (preferably, one or more discernible symptoms) of a targeted disease or condition, e.g. an autoimmune condition resulting from the administration of one or more therapies (e.g., one or more therapeutic agents such as a Treg cell of the invention). In specific embodiments, the terms "treat", "treatment" and "treating" refer to the amelioration of at least one measurable physical parameter of a targeted disease or condition, e.g. an autoimmune condition. In other embodiments, the terms "treat", "treatment" and "treating" refer to the inhibition of the progression of a targeted disease or condition, e.g. an autoimmune condition, either physically by, e.g., stabilization of a discernible symptom, physiologically by, e.g., stabilization of a physical parameter, or both. In other embodiments, the terms "treat", "treatment" and "treating" refer to the reduction or amelioration of the progression, severity and/or duration of a targeted disease or condition, e.g. an autoimmune disease, or the amelioration of one or more symptoms of a targeted disease or condition, e.g. an autoimmune disease. "Treating" or "treatment" refers to both therapeutic treatment and prophylactic or preventative measures; wherein the object is to prevent or slow down (lessen) the targeted disease or condition. Those in need of treatment include those already with the condition as well as those prone to have the condition or those in whom the condition is to be prevented. A subject is successfully "treated" for a disease or condition if, after receiving a therapeutic amount of a population of cells comprising a chimeric receptor according to the present invention, the subject shows observable and/or measurable reduction in or absence of one or more of the following: reduction in the number of pathogenic cells; reduction in the percent of total cells that are pathogenic; and/or relief to some extent, of one or more of the symptoms associated with the specific condition; reduced morbidity and mortality, and improvement in quality of life issues. The above parameters for assessing successful treatment and improvement in the condition are readily measurable by routine procedures familiar to a physician.
- The term "signal transduction pathway" refers to the biochemical relationship between a variety of signal transduction molecules that play a role in the transmission of a signal from one portion of a cell to another portion of a cell.
- The term "subject" is intended to include living organisms in which an immune response can be elicited (e.g., mammals, human). In one embodiment, a subject may be a "patient", i.e. a warm-blooded animal, more preferably a human, who/which is awaiting the receipt of, or is receiving medical care or was/is/will be the object of a medical procedure, or is monitored for the development of the targeted disease or condition, such as, for example, an inflammatory or autoimmune condition. In one embodiment, the subject is an adult (for example a subject above the age of 18). In another embodiment, the subject is a child (for example a subject below the age of 18). In one embodiment, the subject is a male. In another embodiment, the subject is a female. In one embodiment, the subject is affected, preferably is diagnosed, with an autoimmune disease, preferably an autoantibody-mediated autoimmune disease. In one embodiment, the subject is at risk of developing an autoimmune disease, preferably an autoantibody-mediated autoimmune disease. Examples of risks factor include, but are not limited to, genetic predisposition, or familial history of autoantibody-mediated autoimmune disease.
- The term "substantially purified cell" refers to a cell that is essentially free of other cell types. A substantially purified cell also refers to a cell which has been separated from other cell types with which it is normally associated in its naturally occurring state. In one embodiment, a substantially purified cell refers to a cell which is at least about 75% free, 80% free, or 85% free, and preferably about 90%, 95%, 96%, 97%, 98%, or 99% free, from other cell types with which it is normally associated in its naturally occurring state. In some instances, a population of substantially purified cells refers to a homogenous population of cells. In one embodiment, a population of substantially purified cells refers to a population of cells at least about 75% homogenous, 80% homogenous, or 85% homogenous, and preferably about 90%, 95%, 96%, 97%, 98%, or 99% homogenous. In other instances, this term refers simply to cell that have been separated from the cells with which they are naturally associated in their natural state. In some aspects, the cells are cultured *in vitro.* In other aspects, the cells are not cultured *in vitro.*

- The term "transfected" or "transformed" or "transduced" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.
- The term "engineered" or "modified" refers to a cell that has been transfected, transformed or transduced.
- The term "specifically binds," refers to an antibody, or a ligand, which recognizes and binds with a binding partner present in a sample, but which antibody or ligand does not substantially recognize or bind other molecules in the sample.

### DETAILED DESCRIPTION

The present invention relates to a monospecific Treg cell population, wherein the cells comprise a chimeric receptor, wherein said chimeric receptor recognizes a B cell surface marker, and wherein said Treg cells are cytotoxic for B cells, preferably are cytotoxic for B cells expressing at their surface the B cell surface marker recognized by the chimeric receptor.

In one embodiment, the cells of the monospecific Treg cell population of the invention express the chimeric receptor on the cell surface.

According to the invention, the cells of the monospecific Treg cell population of the invention are genetically modified Treg cells that have been genetically modified to express a chimeric receptor recognizing a B cell surface marker. The monospecific Treg cell population of the invention may thus be defined as a redirected monospecific Treg cell population.

As used herein, the term "redirected" refers to a Treg cell carrying a chimeric receptor as described herein, which confers to the Treg cell the ability to bind to and be activated by a ligand that is different from the one the Treg cell is or would have been specific or be activated by.

Preferably, cytotoxicity for B cells results from the expression of the chimeric receptor by the cells of the monospecific Treg cell population of the invention. In other words, the cells of the monospecific Treg cell population of the invention are preferably non cytotoxic for B cells when not expressing the chimeric receptor recognizing a B cell surface marker on a B cell.

Indeed, the Applicant demonstrated that the cells of the monospecific Treg cell population of the invention are not cytotoxic for B cell when not expressing a chimeric receptor as described herein, but become cytotoxic for B cells when genetically modified for expressing said chimeric receptor recognizing a B cell surface marker (see Examples). Without willing to be bound to a theory, the Applicant suggests that redirection of the cells of the monospecific Treg cell population of the invention in order to recognize and be activated by a B cell surface marker, results in the activation of a cytotoxicity pathway, thereby providing to the redirected Treg cell of the invention cytotoxic properties against B cells expressing the recognized marker.

According to one embodiment, the cytotoxicity for B cells of the Treg cells of the monospecific Treg cell population of the invention is dependent on the expression of the chimeric receptor by the Treg cell. Preferably, the cytotoxicity for B cells of the Treg cells of the monospecific Treg cell population of the invention is chimeric receptor dependent, i.e. results from the binding of the chimeric receptor to a marker presented at the surface of a B cell. Furthermore, in one embodiment, the cytotoxicity for B cells of the Treg cells of the monospecific Treg cell population of the invention is TCR independent, i.e. does not result from the binding of the TCR expressed by the Treg cells of the monospecific Treg cell population of the invention to a ligand presented at the surface of a B cell.

In one embodiment, cytotoxicity of the Treg cells of the monospecific Treg cell population of the invention for B cells may be determined by determining the survival of B cells in co-cultures of B cells with the monospecific Treg cell population of the invention. In one embodiment, the B cells and Treg cells of the invention are co-cultured in a ratio Treg cells:B cells of 1:1, 1:2, 1:3, 1:4, 1:5, 1:6, 1:7, 1:8, 1:9, or 1:10.

In one embodiment, the B cells express the B cell surface marker recognized by the B cell surface marker binding domain of the chimeric receptor expressed by the Treg cells of the monospecific Treg cell population of the invention.

Methods for assessing the survival of B cells may rely on determining the percentage of live cells (or alternatively dead cells) and include, for example, flow cytometry analysis to detect live/dead cells, or measure of a compound released in the co-culture medium upon lysis of the B cells by the Treg cells of the monospecific Treg cell population, said B cells having been previously labeled with the compound to be measured. Such "release" methods include, without being limited to, the detection of radioactive compounds, such as for example chromium (⁵¹Cr), or the detection of fluorescent dyes, such as for example, calcein-acetoxymethyl (calcein-AM). Other non-radioactive compounds that may be used in cytotoxic assays include, without being limited to, MTT (3-(4,5-Dimethyl-2-thiazolyl)-2,5-diphenyl-2H-tetrazolium bromide), methylumbelliferyl heptanoate, and Alamar blue (resazurin).

In one embodiment, the dying and dead B cells are specifically labeled with propidium iodide (PI) and detected by FACS analysis after co-culture with the Treg cells of the invention. Propidium iodide is a fluorescent intercalating agent that binds to double-stranded nucleic acids. Propidium iodide cannot pass through an intact cell membrane and thus is not imported into live cells.

In another embodiment, the B cells are labeled with calcein-AM and their survival is assessed through the detection by FACS analysis of calcein-AM released in the culture medium after co-culture with the monospecific Treg cell population of the invention.

Methods for co-culturing cells are well known to the person of ordinary skill in the art. For example, in one embodiment, the B cells and Treg cells are co-cultured at 37°C and with 5% CO₂ in RPMI-1640 medium supplemented with 10% fetal bovine serum and antibiotics.

In one embodiment, the Treg cells of the monospecific Treg cell population of the invention display a cytotoxic activity when the percentage of dead B cells after co-culture with the monospecific Treg cell population of the invention is at least 30%, at least 40%, or at least 50%, preferably at least 60%, at least 65%, or at least 70%, and more preferably at least 75%, at least 80%, at least 85%, at least 90%, or at least 95% of the total number of B cells in co-culture with the monospecific Treg cell population of the invention.

Alternatively, in another embodiment, the Treg cells of the monospecific Treg cell population of the invention display a cytotoxic activity when the rate of survival of B cells after co-culture with the monospecific Treg cell population of the invention is at most 70%, at most 60% or at most 50%, preferably at most 40%, at most 35%, or at most 30%, and more preferably at most 25%, at most 20%, at most 15%, at most 10%, or at most 5% as compared with the starting number of B cells in the co-culture with the monospecific Treg cell population of the invention.

In one embodiment, the cytotoxicity of the Treg cells of the monospecific Treg cell population of the invention is determined in the conditions of Test A.

Test A is a non-radioactive cell killing assay based on FACS analysis to detect B cells labeled with propidium iodide (PI). Briefly, B cells (5x10⁴ in 100 µl) are co-cultured with the monospecific Treg cell population of the invention at various Treg cells:B cells ratios in a 96-well U-bottom tissue culture plate (Costar, Cambridge, MA). The 96-well plate is centrifuged at 400g for 5 min prior to cell culture in a 37°C, 5% CO2 incubator. After 4 hours of co-culture, propidium iodide is added. The cells are resuspended and transferred to 4mL FACS tubes for FACS analysis.

In one embodiment, the Treg cells of the monospecific Treg cell population of the invention expresses at least one cytotoxic molecule, preferably expresses 2, 3, 4, 5, 6 or 7 cytotoxic molecules selected from the group comprising granzyme A, granzyme B, granzyme M, granzyme K, granzyme H, granulysin and perforin.

In one embodiment, the Treg cells of the monospecific Treg cell population of the invention expresses granzyme B and preferably further expresses at least one, preferably at least two or more cytotoxic molecules selected from granzyme A, granzyme M, granzyme K, granzyme H, granulysin and perforin.

In one embodiment, the Treg cells of the monospecific Treg cell population of the invention expresses granzyme B and granzyme H, and preferably further expresses at least one cytotoxic molecule selected from granzyme A, granzyme M, granzyme K, granulysin and perforin.

In one embodiment, the Treg cells of the monospecific Treg cell population of the invention expresses granzyme B and granzyme A.

In one embodiment, the monospecific Treg cell population of the invention is a mammal monospecific Treg cell population, preferably a human monospecific Treg cell population.

In one embodiment, the monospecific Treg cell population of the invention has been frozen and thawed.

In one embodiment, the monospecific Treg cell population of the invention is specific of an antigen, a fragment of an antigen, a variant of an antigen or a mixture thereof.

In one embodiment, the monospecific Treg cell population of the invention is specific of a food antigen from the common human diet.

The term "food antigen from common human diet" refers to an immunogenic peptide, which comes from foodstuffs common for humans, such as food antigens of the following non-limiting list: bovine antigens such as lipocalin, Ca-binding S100, alpha-lactalbumin, lactoglobulins such as beta-lactoglobulin, bovine serum albumin, caseins. Food-antigens may also be atlantic salmon antigens such as parvalbumin; chicken antigens such as ovomucoid, ovalbumin, Ag22, conalbumin, lysozyme or chicken serum albumin; peanut antigens; shrimp antigens such as tropomyosin; wheat antigens such as agglutinin or gliadin; celery antigens such as celery profilin; carrot antigens such as carrot profilin; apple antigens such as thaumatin, apple lipid transfer protein, or apple profilin; pear antigens such as pear profilin, or isoflavone reductase; avocado antigens such as endochitinase; apricot antigens such as apricot lipid transfer protein; peach antigens such as peach lipid transfer protein or peach profilin; soybean antigens such as HPS, soybean profilin or (SAM22) PR-I0 prot; fragments, variants and mixtures thereof.

In another embodiment, the monospecific Treg cell population of the invention is specific of an autoantigen, such as a multiple sclerosis-associated antigen, a joint-associated antigen, an eye-associated antigen, or a human HSP antigen.

The term "multiple sclerosis-associated antigen" refers to myelin basic protein (MBP). myelin associated glycoprotein (MAG), myelin oligodendrocyte glycoprotein (MOG), proteolipid protein (PLP), oligodendrocyte myelin oligoprotein (OMGP), myelin associated oligodendrocyte basic protein (MOBP), oligodendrocyte specific protein (OSP/Claudinl 1), heat shock proteins, oligodendrocyte specific proteins (OSP), NOGO A, glycoprotein Po, peripheral myelin protein 22 (PMP22), 2'3'-cyclic nucleotide 3'-phosphodiesterase (CNPase), fragments, variants and mixtures thereof.

The term "joint-associated antigen" refers to citrulline-substituted cyclic and linear filaggrin peptides, type II collagen peptides, human cartilage glycoprotein 39 (HCgp39) peptides, HSP, heterogeneous nuclear ribonucleoprotein (hnRNP) A2 peptides, hnRNP B1, hnRNP D, Ro60/52, HSP60, HSP65, HSP70 and HSP90, BiP, keratin, vimentin, fibrinogen, type I, III, IV and V collagen peptides, annexin V, Glucose 6 phosphate isomerase (GPI), acetyl-calpastatin, pyruvate dehydrogenase (PDH), aldolase, topoisomerase I, snRNP, PARP, Scl-70, Scl-100, phospholipid antigens including anionic cardiolipin and phosphatidylserine, neutrally charged phosphatidylethanolamine and phosphatidylcholine, matrix metalloproteinase, fibrillin, aggreccan, fragments, variants and mixtures thereof.

The term "eye-associated antigen" refers to type II collagen, retinal arrestin, S-arrestin, interphotoreceptor retinoid-binding proteins (IRBP1), beta-crystallin B 1, retinal proteins, choroid proteins and fragments, variants and mixtures thereof.

The term "human HSP antigen" refers to human HSP60, HSP70, HSP90, fragments, variants and mixtures thereof.

In another embodiment, the monospecific Treg cell population of the invention is specific of an inhaled allergen, an ingested allergen or a contact allergen.

In one embodiment, the monospecific Treg cell population of the invention is specific of an antigen selected from the group comprising ovalbumin, MOG, type II collagen fragments, variants and mixtures thereof.

In one embodiment, the monospecific Treg cell population of the invention is specific of ovalbumin, fragments, variants and mixtures thereof.

In another embodiment, the monospecific Treg cell population of the invention is specific of MOG, fragments, variants and mixtures thereof.

In another embodiment, the monospecific Treg cell population of the invention is specific of type II collagen, fragments, variants and mixtures thereof.

Examples of cells of the B cell lineage include, but are not limited to, proB cells, preB cells, immature (or transitional) B cells, mature naive B cells, activated B cells, memory B cells, plasma cells, lymphoblasts, marginal zone B cells, germinal center B cells, plasmablasts and regulatory B cells (Breg cells).

In one embodiment, the monospecific Treg cell population of the invention is cytotoxic for proB cells, preB cells, immature (or transitional) B cells, mature naive B cells, activated B cells, memory B cells, plasma cells, lymphoblasts, marginal zone B cells, germinal center B cells, plasmablasts and/or regulatory B cells (Breg cells).

In one embodiment, the monospecific Treg cell population of the invention is not cytotoxic for Breg cells.

In one embodiment, the monospecific Treg cell population of the invention is cytotoxic for B cells expressing and presenting an immunoglobulin at their surface. Examples of B cells expressing and presenting an immunoglobulin at their surface include, but are not limited to, preB cells, immature (or transitional) B cells, mature naive B cells, activated B cells, memory B cells, marginal zone B cells, germinal center B cells, and regulatory B cells (Breg cells).

In one embodiment, the monospecific Treg cell population of the invention is cytotoxic for mature B cells, preferably for mature activated B cells. Preferably, the monospecific Treg cell population of the invention is cytotoxic for mature B cells (preferably for mature activated B cells) expressing at their surface a B cell surface marker recognized by the chimeric receptor expressed by the cells of the monospecific Treg cell population.

In one embodiment, the monospecific Treg cell population of the invention is cytotoxic for at least one cell type selected from proB cells, preB cells, immature (or transitional) B cells, mature naive B cells, activated B cells, memory B cells, plasma cells, lymphoblasts, marginal zone B cells, germinal center B cells, plasmablasts and regulatory B cells (Breg cells); preferably is cytotoxic for at least one cell type selected from preB cells, immature (or transitional) B cells, mature naive B cells, activated B cells, memory B cells, marginal zone B cells, germinal center B cells, and regulatory B cells (Breg cells).

In one embodiment, the monospecific Treg cell population of the invention is cytotoxic for at least one cell type selected from proB cells, preB cells, immature (or transitional) B cells, mature naive B cells, activated B cells, memory B cells, plasma cells, lymphoblasts, marginal zone B cells, germinal center B cells, and plasmablasts; preferably is cytotoxic for at least one cell type selected from preB cells, immature (or transitional) B cells, mature naive B cells, activated B cells, memory B cells, marginal zone B cells, and germinal center B cells.

In one embodiment, the chimeric receptor comprises, in one or more polypeptides (i) an extracellular B cell surface marker binding domain, (ii) optionally an extracellular hinge domain, (iii) optionally a transmembrane domain, and (iv) an intracellular signaling domain. In one embodiment, the chimeric receptor further comprises a Tag and/or a leader sequence.

In one embodiment, the cells of the monospecific Treg cell population of the invention comprises at least one nucleic acid sequence encoding the chimeric receptor, wherein said at least one nucleic acid sequences comprises (i) a nucleic acid sequence of a B cell surface marker binding domain, (ii) optionally a nucleic acid sequence of an extracellular hinge domain, (iii) optionally a nucleic acid sequence of a transmembrane domain, (iv) one or more nucleic acid sequence(s) of an intracellular signaling domain. In one embodiment, the nucleic acid sequence further comprises a sequence encoding a Tag and/or a leader sequence.

In one embodiment, the B cell surface marker binding domain may be connected to a transmembrane domain by a hinge domain.

In one embodiment, a short oligo- or polypeptide linker, preferably having a length ranging from 2 and 10 amino acids may form the hinge domain.

For example, a glycine-serine doublet provides a particularly suitable hinge domain (GS linker). In one embodiment, the hinge domain is a Gly/Ser linker. Examples of Gly/Ser linkers include, but are not limited to, GS linkers, G₂S linkers, G₃S linkers, G₄S linkers.

Examples of G₂S linkers include, but are not limited to, GGS.

G₃S linkers comprise the amino acid sequence (Gly-Gly-Gly-Ser)ₙ also referred to as (GGGS)ₙ or (SEQ ID NO: 1)ₙ, where n is a positive integer equal to or greater than 1 (such as, example, n=1, n=2, n=3. n=4, n=5, n=6, n=7, n=8, n=9 or n=10). Examples of G₃S linkers include, but are not limited to, GGGSGGGSGGGSGGGS (SEQ ID NO: 6).

Examples of G₄S linkers include, but are not limited to, (Gly₄ Ser) corresponding to GGGGS (SEQ ID NO: 5); (Gly₄ Ser)₂ corresponding to GGGGSGGGGS (SEQ ID NO: 4); (Gly₄Ser)₃ corresponding to GGGGSGGGGSGGGGS (SEQ ID NO: 3); and (Gly₄ Ser)₄ corresponding to GGGGSGGGGSGGGGSGGGGS (SEQ ID NO: 2).

Also included within the scope of the invention are hinge domains described in WO2012/138475, incorporated herein by reference.

In one embodiment, the hinge domain comprises the amino acid sequence AGSSSSGGSTTGGSTT (SEQ ID NO: 7), the amino acid sequence GTTAASGSSGGSSSGA (SEQ ID NO: 8), the amino acid sequence SSATATAGTGSSTGST (SEQ ID NO: 9), and/or the amino acid sequence TSGSTGTAASSTSTST (SEQ ID NO: 10).

In one embodiment, the hinge domain is encoded by a nucleotide sequence of GGTGGCGGAGGTTCTGGAGGTGGAGGTTCC (SEQ ID NO: 11).

In another embodiment, the hinge domain is a KIR₂DS₂ hinge corresponding to KIRRDSS (SEQ ID NO: 12).

In one embodiment, the hinge domain comprises or consists in the amino acid sequence of a CD8 hinge (SEQ ID NO: 13) or an amino acid sequence with 95-99% identity to SEQ ID NO: 13. In one embodiment, the hinge domain is a CD8 hinge encoded by the nucleic acid sequence SEQ ID NO: 14 or a nucleic acid sequence with 95-99% identity to SEQ ID NO: 14.

In another embodiment, the hinge domain comprises or consists in the amino acid sequence of a IgG4 hinge (SEQ ID NO: 15), or an amino acid sequence with 95-99% identity to SEQ ID NO: 15. In one embodiment, the hinge domain is an IgG4 hinge encoded by the nucleic acid sequence SEQ ID NO: 16 or a nucleic acid sequence with 95-99% identity to SEQ ID NO: 16.

In another embodiment, the hinge domain comprises or consists in the amino acid sequence of a IgD hinge (SEQ ID NO: 17) or an amino acid sequence with 95-99% identity to SEQ ID NO: 17. In one embodiment, the hinge domain is an IgD hinge encoded by the nucleic acid sequence SEQ ID NO: 18 or a nucleic acid sequence with 95-99% identity to SEQ ID NO: 18.

In another embodiment, the hinge region comprises or consists in the amino acid sequence of a CD28 hinge (SEQ ID NO: 19) or an amino acid sequence with 95-99% identity to SEQ ID NO: 19. In one embodiment, the hinge domain is a CD28 hinge encoded by the nucleic acid SEQ ID NO: 20 or a nucleic acid sequence with 95-99% identity to SEQ ID NO: 20.

Examples of transmembrane domains that may be used in the chimeric receptor of the invention include, but are not limited to, transmembrane domains of an alpha, beta or zeta chain of a T-cell receptor, or of CD28, CD3 epsilon, CD45, CD4, CD5, CD8, CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7R a, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, PD1, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRT AM, Ly9 (CD229), CD160 (BY55), PSGL1, CDIOO (SEMA4D), SLAMF6 (NTB-A, Ly108), SLAM (SLAMF1, CD150, IPO-3), BLAME (SLAMF8), SELPLG (CD162), LTBR, PAG/Cbp, NKp44, NKp30, NKp46, NKG2D, and/or NKG2C.

In one embodiment, the transmembrane domain comprises or consists in the amino acid sequence of a CD8 transmembrane domain (SEQ ID NO: 21), or an amino acid sequence with 95-99% identity to SEQ ID NO: 21. In another embodiment, the transmembrane domain comprises or consists in an amino acid sequence having at least one, two or three modifications but not more than 20, 10 or 5 modifications of an amino acid sequence of SEQ ID NO: 21, or an amino acid sequence with 95-99% identity to SEQ ID NO: 21.

In another embodiment, the transmembrane domain is encoded by the nucleotide sequence of a CD8 transmembrane domain (SEQ ID NO: 22), or a nucleotide sequence with 95-99% identity to SEQ ID NO: 22.

In another embodiment, the transmembrane domain comprises or consists in the amino acid sequence of a CD28 transmembrane domain (SEQ ID NO: 23) or an amino acid sequence with 95-99% identity to SEQ ID NO: 23. In one embodiment, the transmembrane domain is a CD28 transmembrane domain encoded by the nucleic acid sequence SEQ ID NO: 24 or a nucleic acid sequence with 95-99% identity to SEQ ID NO: 24.

In one embodiment, the transmembrane domain may be recombinant, in which case it will comprise predominantly hydrophobic amino acids such as valine or leucine.

In one embodiment, the intracellular signaling domain can comprise the entire intracellular portion, or the entire native intracellular signaling domain, of the molecule from which it is derived, or a functional fragment or derivative thereof.

According to one embodiment, the intracellular signaling domain comprises a T cell primary signaling domain (or a sequence derived therefrom) and optionally one or more intracellular domain(s) of a T cell costimulatory molecule (or sequence(s) derived therefrom).

In one embodiment, the intracellular signaling domain of the chimeric receptor of the invention consists in a primary signaling domain.

In one embodiment, the intracellular signaling domain comprises one or more intracellular domain(s) of a T cell costimulatory molecule. In one embodiment, the intracellular signaling domain consists in one or more intracellular domain(s) of a T cell costimulatory molecule.

In another embodiment, the intracellular signaling domain of the chimeric receptor of the invention comprises at least one costimulatory domain and a primary signaling domain.

In another embodiment, the intracellular signaling domain of the chimeric receptor of the invention comprises at least two costimulatory domains and a primary signaling domain.

In one embodiment of the invention, the T cell primary signaling domain comprises a signaling domain of a protein selected in the group of CD3 zeta, CD3 gamma, CD3 delta, CD3 epsilon, common FcR gamma (FCER1G), FcR beta (Fc Epsilon Rib), CD79a, CD79b, Fcgamma RIIa, DAP10, and DAP 12 and sequences derived therefrom.

In one embodiment, the T cell primary signaling domain comprises or consists in a functional signaling domain of CD3 zeta.

In one embodiment, the T cell primary signaling domain comprises or consists in the amino acid sequence of the CD3-zeta domain of SEQ ID NO: 25, 26, 66 or 67, or an amino acid sequence with 95-99% identity to SEQ ID NO: 25, 26, 66 or 67.

In another embodiment, the CD3 zeta primary signaling domain comprises or consists in an amino acid sequence having at least one, two or three modifications but not more than 20, 10 or 5 modifications of an amino acid sequence of SEQ ID NO: 25, 26, 66 or 67, or an amino acid sequence with 95-99% identity to SEQ ID NO: 25, 26, 66 or 67.

Thus, in one embodiment, the nucleic acid sequence encoding the T cell primary signaling domain comprises or consists in the nucleic acid sequence of the CD3-zeta domain of SEQ ID NO: 27 or SEQ ID NO: 28, or a nucleotide sequence with 95-99% identity to SEQ ID NO: 27 or SEQ ID NO: 28.

T cell primary signaling domains that act in a stimulatory manner may comprise signaling motifs known as immunoreceptor tyrosine-based activation motifs (ITAMS).

Examples of ITAM containing T cell primary intracellular signaling domains that are of particular use in the invention include, but are not limited to, those of (or derived from) CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12.

In one embodiment, the T cell primary signaling domain comprises a modified ITAM domain, e.g., a mutated ITAM domain which has altered (e.g., increased or decreased) activity as compared to the native ITAM domain. In one embodiment, a primary signaling domain comprises a modified ITAM-containing primary intracellular signaling domain, e.g., an optimized and/or truncated ITAM-containing primary intracellular signaling domain. In an embodiment, a primary signaling domain comprises one, two, three, four or more ITAM motifs.

In one embodiment, the intracellular signaling domain of the chimeric receptor of the invention comprises a T cell primary signaling domain (such as, for example, a CD3-zeta signaling domain), combined with one or more costimulatory signaling domains. A costimulatory signaling domain(s) refers to a portion of the chimeric receptor comprising at least one intracellular domain of a costimulatory molecule. A costimulatory molecule is a cell surface molecule other than an antigen receptor or its ligands that is required for an efficient response of lymphocytes to an antigen.

Examples of intracellular domains of a T cell costimulatory molecule include, but are not limited to, the signaling domains of proteins selected in the group of CD27, CD28, 4-1BB (CD137), an MHC class I molecule, BTLA and a Toll ligand receptor, OX40, CD30, CD40, PD-1, ICOS, lymphocyte function-associated antigen-1 (LFA-1), CD2, CD7, LIGHT, NKG2C, B7-H3, a ligand that specifically binds with CD83, CDS, ICAM-1, GITR, BAFFR, HVEM (LIGHTR), SLAMF7, NKp80 (KLRF1), NKp44, NKp30, NKp46, CD160 (BY55), CD19, CD19a, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a,, ITGAM, CD11b, ITGAX, CD11c, ITGB1, CD29, ITGB2, CD18, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, or NKG2D.

In one embodiment of the invention, the chimeric receptor comprises at least one intracellular domain of a T cell costimulatory molecule selected from the group comprising 4-1BB, ICOS, CD27, OX40, CD28, CTLA4 and PD-1.

In one embodiment, the T cell costimulatory signaling domain comprises or consists in the amino acid sequence of a 4-1BB intracellular domain (SEQ ID NO: 29) or an amino acid sequence with 95-99% identity to SEQ ID NO: 29. In another embodiment, the T cell costimulatory signaling domain comprises or consists in an amino acid sequence having at least one, two or three modifications but not more than 20, 10 or 5 modifications of an amino acid sequence of SEQ ID NO: 29.

In one embodiment, the T cell costimulatory signaling domain comprises or consists in the amino acid sequence of a CD27 intracellular domain (SEQ ID NO: 30) or an amino acid sequence with 95-99% identity to SEQ ID NO: 30. In another embodiment, the T cell costimulatory signaling domain comprises or consists in an amino acid sequence having at least one, two or three modifications but not more than 20, 10 or 5 modifications of an amino acid sequence of SEQ ID NO: 30.

In one embodiment, the T cell costimulatory signaling domain comprises or consists in the amino acid sequence of a CD28 intracellular domain (SEQ ID NO: 31) or an amino acid sequence with 95-99% identity to SEQ ID NO: 31. In another embodiment, the T cell costimulatory signaling domain comprises or consists in an amino acid sequence having at least one, two or three modifications but not more than 20, 10 or 5 modifications of an amino acid sequence of SEQ ID NO: 31.

In one embodiment of the invention, the chimeric receptor comprises a combination of at least two intracellular domains of a T cell costimulatory molecule, preferably selected from an intracellular domain of CD28, an intracellular domain of CD27 and an intracellular domain of 4-1BB.

In one embodiment, the chimeric receptor comprises the amino acid sequence of a 4-1BB intracellular domain (SEQ ID NO: 29) or an amino acid sequence with 95-99% identity to SEQ ID NO: 29 and the amino acid sequence of a CD27 intracellular domain (SEQ ID NO: 30) or an amino acid sequence with 95-99% identity to SEQ ID NO: 30.

In another embodiment, the chimeric receptor comprises the amino acid sequence of a 4-1BB intracellular domain (SEQ ID NO: 29) or an amino acid sequence with 95-99% identity to SEQ ID NO: 29 and the amino acid sequence of a CD28 intracellular domain (SEQ ID NO: 31) or an amino acid sequence with 95-99% identity to SEQ ID NO: 31.

In yet another embodiment, the chimeric receptor comprises the amino acid sequence of a CD27 intracellular domain (SEQ ID NO: 30) or an amino acid sequence with 95-99% identity to SEQ ID NO: 30 and the amino acid sequence of a CD28 intracellular domain (SEQ ID NO: 31) or an amino acid sequence with 95-99% identity to SEQ ID NO: 31. In one embodiment, the chimeric receptor comprises the amino acid sequence of a 4-1BB intracellular domain (SEQ ID NO: 29) or an amino acid sequence with 95-99% identity to SEQ ID NO: 29 and the amino acid sequence of a CD27 intracellular domain (SEQ ID NO: 30) or an amino acid sequence with 95-99% identity to SEQ ID NO: 30 and the amino acid sequence of a CD28 intracellular domain (SEQ ID NO: 31) or an amino acid sequence with 95-99% identity to SEQ ID NO: 31.

Thus, in one embodiment, the nucleic acid sequence encoding the T cell costimulatory signaling domain comprises the nucleic acid sequence of a 4-1BB intracellular domain (SEQ ID NO: 32) or a nucleic acid sequence with 95-99% identity to SEQ ID NO: 32, and/or the nucleic acid sequence of a CD27 intracellular domain (SEQ ID NO: 33) or a nucleic acid sequence with 95-99% identity to SEQ ID NO: 33, and/or the nucleic acid sequence of a CD28 intracellular domain (SEQ ID NO: 34), or a nucleic acid sequence with 95-99% identity to SEQ ID NO: 34.

In embodiment, the intracellular signaling domain of the chimeric receptor of the invention comprises:
- the amino acid sequence of a 4-1BB intracellular domain of SEQ ID NO: 29 or an amino acid sequence with 95-99% identity to SEQ ID NO: 29, and/or the amino acid sequence of a CD27 intracellular domain of SEQ ID NO: 30 or an amino acid sequence with 95-99% identity to SEQ ID NO: 30, and/or the amino acid sequence of a CD28 intracellular domain of SEQ ID NO: 31 or an amino acid sequence with 95-99% identity to SEQ ID NO: 31; and
- the amino acid sequence of a CD3-zeta intracellular domain of SEQ ID NO: 25, 26, 66 or 67, or an amino acid sequence with 95-99% identity to SEQ ID NO: 25, 26, 66 or 67;
wherein the sequences comprised in the intracellular domain are expressed in the same frame and as a single polypeptide chain.

Thus, in one embodiment, the nucleic acid sequence encoding the intracellular signaling domain of the chimeric receptor of the invention comprises:
- the nucleic acid sequence of a 4-1BB intracellular domain of SEQ ID NO: 32 or a nucleic acid sequence with 95-99% identity to SEQ ID NO: 32, and/or the nucleic acid sequence of a CD27 intracellular domain of SEQ ID NO: 33 or a nucleic acid sequence with 95-99% identity to SEQ ID NO: 33, and/or the nucleic acid sequence of a CD28 intracellular domain of SEQ ID NO: 34 or a nucleic acid sequence with 95-99% identity to SEQ ID NO: 34; and
- the nucleic acid sequence of a CD3-zeta intracellular domain of SEQ ID NO: 27 or SEQ ID NO: 28, or a sequence with 95-99% identity to SEQ ID NO: 27 or SEQ ID NO: 28.

In one embodiment, the intracellular signaling domain of the chimeric receptor of the invention comprises at least two different domains (e.g. a primary signaling domain and at least one intracellular domain of a T cell costimulatory molecule) that may be linked to each other in a random or in a specified order.

Optionally, a short oligo- or polypeptide linker, for example, between 2 and 10 amino acids (e.g., 2, 3, 4, 5, 6, 7, 8, 9, or 10 amino acids) in length may form the linkage between distinct signaling domains. In one embodiment, a glycine-serine doublet (GS) is used as a suitable linker. In one embodiment, a single amino acid, e.g., an alanine (A), a glycine (G), is used as a suitable linker.

In another embodiment, the intracellular signaling domain of the chimeric receptor of the invention comprise two or more, e.g., 2, 3, 4, 5, or more, costimulatory signaling domains. In another embodiment, the two or more, e.g., 2, 3, 4, 5, or more, costimulatory signaling domains, are separated by a linker molecule, e.g., a linker molecule as described hereinabove.

In one embodiment, the intracellular signaling domain of the chimeric receptor of the invention comprises the primary signaling domain of CD3-zeta (preferably SEQ ID NO: 25, 26, 66 or 67) and the co-stimulatory signaling domain of CD28 (preferably SEQ ID NO: 31).

In another embodiment, the intracellular signaling domain of the chimeric receptor of the invention comprises the primary signaling domain of CD3-zeta (preferably SEQ ID NO: 25, 26, 66 or 67) and the co-stimulatory signaling domain of 4-1BB (preferably SEQ ID NO: 29).

In another embodiment, the intracellular signaling domain of the chimeric receptor of the invention comprises the signaling domain of CD3-zeta (preferably SEQ ID NO: 25, 26, 66 or 67) and the signaling domain of CD27 (preferably SEQ ID NO: 30).

According to the invention, the chimeric receptor of the invention recognizes a B cell surface marker expressed at the surface of a B cell. In one embodiment, the cells of the monospecific Treg cell population of the invention express a chimeric receptor recognizing a B cell surface marker expressed at the surface of a B cell and are cytotoxic for B cells expressing said B cell surface marker at their surface.

In one embodiment, the B cell surface marker binding domain is an antibody directed to a B cell surface marker or an antigen binding fragment thereof.

The portion of the chimeric receptor of the invention comprising an antibody or antigen binding fragment thereof may exist in a variety of forms where the antigen binding domain is expressed as part of a contiguous polypeptide chain including, for example, a single domain antibody fragment (sdAb), a single chain antibody (scFv), a humanized antibody or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the ligand binding domain of a chimeric receptor composition of the invention comprises an antibody fragment. In a further aspect, the chimeric receptor comprises an antibody fragment that comprises a scFv. The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof.

In one embodiment, the antibody is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

Examples of surface markers of B cells (preferably human B cells) include but are not limited to, CD19, CD20, BCMA, IgM, IgA, IgG, IgE, IgD, CD1, CD5, CD21, CD22, CD23, CD24, CD25, CD27, CD30, CD38, CD40, CD78, CD80, CD138, CD319, PDL-2, CXCR3, CXCR4, CXCR5, CXCR6, Notch2, TLR4, IL-6, IL-10 and TGFβ. Preferably, the at least one B cell surface marker is selected from CD19, CD20, BCMA, IgM, IgA, IgG, IgE, IgD, CD1, CD21, CD22, CD138. More preferably, the at least one B cell surface marker is selected from CD19 and CD20.

Examples of surface markers of proB cells (preferably human proB cells) include but are not limited to, CD10, CD19, CD24, CD34 and CD38.

Examples of surface markers of preB cells (preferably human preB cells) include but are not limited to, CD5, CD10, CD19, CD20 and CD34, CD38.

Examples of surface markers of immature (or transitional) B cells (preferably human immature B cells) include but are not limited to, CD5, CD10, CD19, CD20, CD22, CD24, CD38 and IgG.

Examples of surface markers of mature naive B cells (preferably human mature naive B cells) include but are not limited to, CD5, CD19, CD20, CD22, CD24, CD38 and IgG.

Examples of surface markers of marginal zone B cells (preferably human marginal zone B cells) include but are not limited to, CD1, CD19, CD20, CD21, CD22, CD23, CD27, IgG and Notch2.

Examples of surface markers of plasma cells (preferably human plasma cells) include but are not limited to, CD19, CD27, CD38, CD138, IgG, MHCII, and Il6.

Examples of surface markers of plasmablasts (preferably human plasmablasts) include but are not limited to, CD19, CD20, CD27, CD38, IgG, and MHCII.

Examples of surface markers of memory B cells (preferably human memory B cells) include but are not limited to, CD19, CD20, CD22, CD24, CD27, CD38, CD40, CD80, PD-L2, IgG, CXCR3, CXCR4, CXCR5, CXCR6, IgA, IgG and IgE.

Examples of surface markers of germinal center B cells (preferably human germinal center B cells) include but are not limited to, CD10, CD19, CD20, CD22, CD38, and IgG.

Examples of surface markers of activated B cells (preferably human activated B cells) include but are not limited to, CD19, CD25, and CD30.

Examples of surface markers of regulatory B cells (Breg cells) include but are not limited to, CD1, CD1d, CD5, CD19, CD21, CD23, CD24, CD40, Fas ligand, I110, TLR4, TGFβ, IgD, IgM, PD-L1, PD-L2, TIM-1, TNFSF18, TRAIL. Examples of surface markers of human Breg cells include, but are not limited to, CD1, CD1d, CD5, CD19, CD21, CD24, CD40, I110, TLR4, TGFβ, IgD and IgM.

Examples of surface markers of B cells (preferably human B cells) with non-secreted Ig include but are not limited to, CD138 and Notch2.

In one embodiment, the B cell surface marker is CD 19. In one embodiment, the CD 19 binding domain comprises an antibody directed to CD19 or an antigen binding fragment thereof. In one embodiment, the CD 19 binding domain comprises a scFv directed to CD 19. Examples of scFv directed to CD 19 include, but are not limited to, SEQ ID NO: 37 (encoded by SEQ ID NO: 36) and MFC63 (SEQ ID NO: 38).

In another embodiment, the B cell surface marker is CD20. In one embodiment, the CD20 binding domain comprises an antibody directed to CD20 or an antigen binding fragment thereof. Examples of CD20 antibodies include, but are not limited to, rituximab (comprising or consisting of SEQ ID NO: 56 or a fragment or variant thereof) and scFv comprising or consisting of a sequence selected from SEQ ID NO: 46, SEQ ID NO: 59 or SEQ ID NO: 62, or fragments or variants thereof.

As used herein, a variant of a peptide may refer to a modified peptide wherein 1, 2, 3, 4, 5, 6, 7, 8, 9 or 10 amino acids are deleted, added or substituted as compared to the original peptide.

In one embodiment, the chimeric receptor of the invention further comprises a leader sequence located N-terminally from the B cell surface marker binding domain. A non-limiting example of leader sequence is a leader sequence of CD8 that may comprise or consists in the sequence SEQ ID NO: 39.

In one embodiment, the chimeric receptor further comprises a tag, such as, for example, a tag for quality control, enrichment, tracking in vivo and the like. Said Tag may be localized N-terminally, C-terminally and/or internally. Examples of tags that may be used in the chimeric receptor of the invention are well known by the skilled artisan. For example, but without limitation, a tag used in the invention can be a tag selected from the group comprising or consisting of Hemagglutinin Tag, Poly Arginine Tag, Poly Histidine Tag, Myc Tag, Strep Tag, S-Tag, HAT Tag, 3x Flag Tag, Calmodulin-binding peptide Tag, SBP Tag, Chitin binding domain Tag, GST Tag, Maltose-Binding protein Tag, Fluorescent Protein Tag, T7 Tag, V5 Tag and Xpress Tag. Other examples of tag include, without limitation, NWSHPQFEK (SEQ ID NO: 64) or SAWSHPQFEK (SEQ ID NO: 65).

According to a first embodiment, the chimeric receptor of the invention comprises a B cell surface marker binding domain, optionally an extracellular hinge domain, a transmembrane domain, and a T cell primary signaling domain.

In one embodiment, the chimeric receptor of the invention comprises a B cell surface marker binding domain; a transmembrane domain of CD8 (preferably SEQ ID NO: 21); and a CD3-zeta primary signaling domain (preferably SEQ ID NO: 25, 26, 66 or 67).

In one embodiment, the chimeric receptor of the invention comprises a B cell surface marker binding domain; a transmembrane domain of CD28 (preferably SEQ ID NO: 23); and a CD3-zeta primary signaling domain (preferably SEQ ID NO: 25, 26, 66 or 67).

In another embodiment, the chimeric receptor of the invention comprises a B cell surface marker binding domain; a hinge domain of CD8 (preferably SEQ ID NO: 13); a transmembrane domain of CD8 (preferably SEQ ID NO: 21); and a CD3-zeta primary signaling domain (preferably SEQ ID NO: 25, 26, 66 or 67). In one embodiment, the chimeric receptor of the invention comprises a B cell surface marker binding domain; a hinge domain of CD8 (preferably SEQ ID NO: 13); a transmembrane domain of CD28 (preferably SEQ ID NO: 23); and a CD3-zeta primary signaling domain (preferably SEQ ID NO: 25, 26, 66 or 67).

In another embodiment, the chimeric receptor of the invention comprises a B cell surface marker binding domain; a hinge domain of IgG4 (preferably SEQ ID NO: 15); a transmembrane domain of CD8 (preferably SEQ ID NO: 21); and a CD3-zeta primary signaling domain (preferably SEQ ID NO: 25, 26, 66 or 67). In one embodiment, the chimeric receptor of the invention comprises a B cell surface marker binding domain; a hinge domain of IgG4 (preferably SEQ ID NO: 15); a transmembrane domain of CD28 (preferably SEQ ID NO: 23); and a CD3-zeta primary signaling domain (preferably SEQ ID NO: 25, 26, 66 or 67).

In another embodiment, the chimeric receptor of the invention comprises a B cell surface marker binding domain; a hinge domain of IgD (preferably SEQ ID NO: 17); a transmembrane domain of CD8 (preferably SEQ ID NO: 21); and a CD3-zeta primary signaling domain (preferably SEQ ID NO: 25, 26, 66 or 67). In one embodiment, the chimeric receptor of the invention comprises a B cell surface marker binding domain; a hinge domain of IgD (preferably SEQ ID NO: 17); a transmembrane domain of CD28 (preferably SEQ ID NO: 23); and a CD3-zeta primary signaling domain (preferably SEQ ID NO: 25, 26, 66 or 67).

In another embodiment, the chimeric receptor of the invention comprises a B cell surface marker binding domain; a hinge domain of CD28 (preferably SEQ ID NO: 19); a transmembrane domain of CD8 (preferably SEQ ID NO: 21); and a CD3-zeta primary signaling domain (preferably SEQ ID NO: 25, 26, 66 or 67). In one embodiment, the chimeric receptor of the invention comprises a B cell surface marker binding domain; a hinge domain of CD28 (preferably SEQ ID NO: 19); a transmembrane domain of CD28 (preferably SEQ ID NO: 23); and a CD3-zeta primary signaling domain (preferably SEQ ID NO: 25, 26, 66 or 67).

According to a second embodiment, the chimeric receptor of the invention comprises a B cell surface marker binding domain, optionally an extracellular hinge domain, a transmembrane domain, a single intracellular domain of a T cell costimulatory molecule and a T cell primary signaling domain.

In one embodiment, the chimeric receptor of the invention comprises a B cell surface marker binding domain; a transmembrane domain of CD8 (preferably SEQ ID NO: 21); an intracellular domain of 4-1BB (preferably SEQ ID NO: 29); and a CD3-zeta primary signaling domain (preferably SEQ ID NO: 25, 26, 66 or 67). In one embodiment, the chimeric receptor of the invention comprises a B cell surface marker binding domain; a transmembrane domain of CD8 (preferably SEQ ID NO: 21); an intracellular domain of CD27 (preferably SED ID NO: 30); and a CD3-zeta primary signaling domain (preferably SEQ ID NO: 25, 26, 66 or 67). In one embodiment, the chimeric receptor of the invention comprises a B cell surface marker binding domain; a transmembrane domain of CD8 (preferably SEQ ID NO: 21); an intracellular domain of CD28 (preferably SED ID NO: 31); and a CD3-zeta primary signaling domain (preferably SEQ ID NO: 25, 26, 66 or 67).

In one embodiment, the chimeric receptor of the invention comprises a B cell surface marker binding domain; a transmembrane domain of CD28 (preferably SEQ ID NO: 23); an intracellular domain of 4-1BB (preferably SED ID NO: 29); and a CD3-zeta primary signaling domain (preferably SEQ ID NO: 25, 26, 66 or 67). In one embodiment, the chimeric receptor of the invention comprises a B cell surface marker binding domain; a transmembrane domain of CD28 (preferably SEQ ID NO: 23); an intracellular domain of CD27 (preferably SED ID NO: 30); and a CD3-zeta primary signaling domain (preferably SEQ ID NO: 25, 26, 66 or 67). In one embodiment, the chimeric receptor of the invention comprises a B cell surface marker binding domain; a transmembrane domain of CD28 (preferably SEQ ID NO: 23); an intracellular domain of CD28 (preferably SED ID NO: 31); and a CD3-zeta primary signaling domain (preferably SEQ ID NO: 25, 26, 66 or 67).

In another embodiment, the chimeric receptor of the invention comprises a B cell surface marker binding domain; a hinge domain of CD8 (preferably SEQ ID NO: 13); a transmembrane domain of CD8 (preferably SEQ ID NO: 21); an intracellular domain of 4-1BB (preferably SED ID NO: 29); and a CD3-zeta primary signaling domain (preferably SEQ ID NO: 25, 26, 66 or 67). In one embodiment, the chimeric receptor of the invention comprises a B cell surface marker binding domain; a hinge domain of CD8 (preferably SEQ ID NO: 13); a transmembrane domain of CD8 (preferably SEQ ID NO: 21); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of CD8 (preferably SEQ ID NO: 13); a transmembrane domain of CD8 (preferably SEQ ID NO: 21); an intracellular domain of CD28 (preferably SEQ ID NO: 31); and a CD3-zeta primary signaling domain (preferably SEQ ID NO: 25, 26, 66 or 67).

In one embodiment, the chimeric receptor of the invention comprises a B cell surface marker binding domain; a hinge domain of CD8 (preferably SEQ ID NO: 13); a transmembrane domain of CD28 (preferably SEQ ID NO: 23); an intracellular domain of 4-1BB (preferably SEQ ID NO: 29); and a CD3-zeta primary signaling domain (preferably SEQ ID NO: 25, 26, 66 or 67). In one embodiment, the chimeric receptor of the invention comprises a B cell surface marker binding domain; a hinge domain of CD8 (preferably SEQ ID NO: 13); a transmembrane domain of CD28 (preferably SEQ ID NO: 23); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of CD8 (preferably SEQ ID NO: 13); a transmembrane domain of CD28 (preferably SEQ ID NO: 23); an intracellular domain of CD28 (preferably SEQ ID NO: 31); and a CD3-zeta primary signaling domain (preferably SEQ ID NO: 25, 26, 66 or 67).

In another embodiment, the chimeric receptor of the invention comprises a B cell surface marker binding domain; a hinge domain of IgG4 (preferably SEQ ID NO: 15); a transmembrane domain of CD8 (preferably SEQ ID NO: 21); an intracellular domain of 4-1BB (preferably SED ID NO: 29); and a CD3-zeta primary signaling domain (preferably SEQ ID NO: 25, 26, 66 or 67). In one embodiment, the chimeric receptor of the invention comprises a B cell surface marker binding domain; a hinge domain of IgG4 (preferably SEQ ID NO: 15); a transmembrane domain of CD8 (preferably SEQ ID NO: 21); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of IgG4 (preferably SEQ ID NO: 15); a transmembrane domain of CD8 (preferably SEQ ID NO: 21); an intracellular domain of CD28 (preferably SEQ ID NO: 31); and a CD3-zeta primary signaling domain (preferably SEQ ID NO: 25, 26, 66 or 67).

In one embodiment, the chimeric receptor of the invention comprises a B cell surface marker binding domain; a hinge domain of IgG4 (preferably SEQ ID NO: 15); a transmembrane domain of CD28 (preferably SEQ ID NO: 23); an intracellular domain of 4-1BB (preferably SED ID NO: 29); and a CD3-zeta primary signaling domain (preferably SEQ ID NO: 25, 26, 66 or 67). In one embodiment, the chimeric receptor of the invention comprises a B cell surface marker binding domain; a hinge domain of IgG4 (preferably SEQ ID NO: 15); a transmembrane domain of CD28 (preferably SEQ ID NO: 23); an intracellular domain of CD27 (preferably SED ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of IgG4 (preferably SEQ ID NO: 15); a transmembrane domain of CD28 (preferably SEQ ID NO: 23); an intracellular domain of CD28 (preferably SED ID NO: 31); and a CD3-zeta primary signaling domain (preferably SEQ ID NO: 25, 26, 66 or 67).

In another embodiment, the chimeric receptor of the invention comprises a B cell surface marker binding domain; a hinge domain of IgD (preferably SEQ ID NO: 17); a transmembrane domain of CD8 (preferably SEQ ID NO: 21); an intracellular domain of 4-1BB (preferably SED ID NO: 29); and a CD3-zeta primary signaling domain (preferably SEQ ID NO: 25, 26, 66 or 67). In one embodiment, the chimeric receptor of the invention comprises a B cell surface marker binding domain; a hinge domain of IgD (preferably SEQ ID NO: 17); a transmembrane domain of CD8 (preferably SEQ ID NO: 21); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of IgD (preferably SEQ ID NO: 17); a transmembrane domain of CD8 (preferably SEQ ID NO: 21); an intracellular domain of CD28 (preferably SEQ ID NO: 31); and a CD3-zeta primary signaling domain (preferably SEQ ID NO: 25, 26, 66 or 67).

In one embodiment, the chimeric receptor of the invention comprises a B cell surface marker binding domain; a hinge domain of IgD (preferably SEQ ID NO: 17); a transmembrane domain of CD28 (preferably SEQ ID NO: 23); an intracellular domain of 4-1BB (preferably SED ID NO: 29); and a CD3-zeta primary signaling domain (preferably SEQ ID NO: 25, 26, 66 or 67). In one embodiment, the chimeric receptor of the invention comprises a B cell surface marker binding domain; a hinge domain of IgD (preferably SEQ ID NO: 17); a transmembrane domain of CD28 (preferably SEQ ID NO: 23); an intracellular domain of CD27 (preferably SED ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of IgD (preferably SEQ ID NO: 17); a transmembrane domain of CD28 (preferably SEQ ID NO: 23); an intracellular domain of CD28 (preferably SEQ ID NO: 31); and a CD3-zeta primary signaling domain (preferably SEQ ID NO: 25, 26, 66 or 67).

In another embodiment, the chimeric receptor of the invention comprises a B cell surface marker binding domain; a hinge domain of CD28 (preferably SEQ ID NO: 19); a transmembrane domain of CD8 (preferably SEQ ID NO: 21); an intracellular domain of 4-1BB (preferably SED ID NO: 29); and a CD3-zeta primary signaling domain (preferably SEQ ID NO: 25, 26, 66 or 67). In one embodiment, the chimeric receptor of the invention comprises a B cell surface marker binding domain; a hinge domain of CD28 (preferably SEQ ID NO: 19); a transmembrane domain of CD8 (preferably SEQ ID NO: 21); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of CD28 (preferably SEQ ID NO: 19); a transmembrane domain of CD8 (preferably SEQ ID NO: 21); an intracellular domain of CD28 (preferably SEQ ID NO: 31); and a CD3-zeta primary signaling domain (preferably SEQ ID NO: 25, 26, 66 or 67).

In one embodiment, the chimeric receptor of the invention comprises a B cell surface marker binding domain; a hinge domain of CD28 (preferably SEQ ID NO: 19); a transmembrane domain of CD28 (preferably SEQ ID NO: 23); an intracellular domain of 4-1BB (preferably SED ID NO: 29); and a CD3-zeta primary signaling domain (preferably SEQ ID NO: 25, 26, 66 or 67). In one embodiment, the chimeric receptor of the invention comprises a B cell surface marker binding domain; a hinge domain of CD28 (preferably SEQ ID NO: 19); a transmembrane domain of CD28 (preferably SEQ ID NO: 23); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of CD28 (preferably SEQ ID NO: 19); a transmembrane domain of CD28 (preferably SEQ ID NO: 23); an intracellular domain of CD28 (preferably SED ID NO: 31); and a CD3-zeta primary signaling domain (preferably SEQ ID NO: 25, 26, 66 or 67).

According to a third embodiment, the chimeric receptor of the invention comprises a B cell surface marker binding domain, optionally an extracellular hinge domain, a transmembrane domain, two intracellular domains of a T cell costimulatory molecule and a T cell primary signaling domain.

In one embodiment, the chimeric receptor of the invention comprises a B cell surface marker binding domain; a transmembrane domain of CD8 (preferably SEQ ID NO: 21); an intracellular domain of 4-1BB (preferably SED ID NO: 29); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a transmembrane domain of CD8 (preferably SEQ ID NO: 21); an intracellular domain of 4-1BB (preferably SEQ ID NO: 29); an intracellular domain of CD28 (preferably SEQ ID NO: 31); and a CD3-zeta primary signaling domain (preferably SEQ ID NO: 25, 26, 66 or 67). In one embodiment, the chimeric receptor of the invention comprises a B cell surface marker binding domain; a transmembrane domain of CD8 (preferably SEQ ID NO: 21); an intracellular domain of CD27 (preferably SEQ ID NO: 30); an intracellular domain of CD28 (preferably SEQ ID NO: 31); and a CD3-zeta primary signaling domain (preferably SEQ ID NO: 25, 26, 66 or 67).

In one embodiment, the chimeric receptor of the invention comprises a B cell surface marker binding domain; a transmembrane domain of CD28 (preferably SEQ ID NO: 23); an intracellular domain of 4-1BB (preferably SED ID NO: 29); an intracellular domain of CD27 (preferably SED ID NO: 30); and a CD3-zeta primary signaling domain (preferably SEQ ID NO: 25, 26, 66 or 67). In one embodiment, the chimeric receptor of the invention comprises a B cell surface marker binding domain; a transmembrane domain of CD28 (preferably SEQ ID NO: 23); an intracellular domain of 4-1BB (preferably SED ID NO: 29); an intracellular domain of CD28 (preferably SEQ ID NO: 31); and a CD3-zeta primary signaling domain (preferably SEQ ID NO: 25, 26, 66 or 67). In one embodiment, the chimeric receptor of the invention comprises a B cell surface marker binding domain; a transmembrane domain of CD28 (preferably SEQ ID NO: 23); an intracellular domain of CD27 (preferably SED ID NO: 30); an intracellular domain of CD28 (preferably SED ID NO: 31); and a CD3-zeta primary signaling domain (preferably SEQ ID NO: 25, 26, 66 or 67).

In another embodiment, the chimeric receptor of the invention comprises a B cell surface marker binding domain; a hinge domain of CD8 (preferably SEQ ID NO: 13); a transmembrane domain of CD8 (preferably SEQ ID NO: 21); an intracellular domain of 4-1BB (preferably SED ID NO: 29); an intracellular domain of CD27 (preferably SED ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of CD8 (preferably SEQ ID NO: 13); a transmembrane domain of CD8 (preferably SEQ ID NO: 21); an intracellular domain of 4-1BB (preferably SED ID NO: 29); an intracellular domain of CD28 (preferably SED ID NO: 31); and a CD3-zeta primary signaling domain (preferably SEQ ID NO: 25, 26, 66 or 67). In one embodiment, the chimeric receptor of the invention comprises a B cell surface marker binding domain; a hinge domain of CD8 (preferably SEQ ID NO: 13); a transmembrane domain of CD8 (preferably SEQ ID NO: 21); an intracellular domain of CD27 (preferably SEQ ID NO: 30); an intracellular domain of CD28 (preferably SEQ ID NO: 31); and a CD3-zeta primary signaling domain (preferably SEQ ID NO: 25, 26, 66 or 67).

In one embodiment, the chimeric receptor of the invention comprises a B cell surface marker binding domain; a hinge domain of CD8 (preferably SEQ ID NO: 13); a transmembrane domain of CD28 (preferably SEQ ID NO: 23); an intracellular domain of 4-1BB (preferably SED ID NO: 29); an intracellular domain of CD27 (preferably SED ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of CD8 (preferably SEQ ID NO: 13); a transmembrane domain of CD28 (preferably SEQ ID NO: 23); an intracellular domain of 4-1BB (preferably SEQ ID NO: 29); an intracellular domain of CD28 (preferably SEQ ID NO: 31); and a CD3-zeta primary signaling domain (preferably SEQ ID NO: 25, 26, 66 or 67). In one embodiment, the chimeric receptor of the invention comprises a B cell surface marker binding domain; a hinge domain of CD8 (preferably SEQ ID NO: 13); a transmembrane domain of CD28 (preferably SEQ ID NO: 23); an intracellular domain of CD27 (preferably SED ID NO: 30); an intracellular domain of CD28 (preferably SEQ ID NO: 31); and a CD3-zeta primary signaling domain (preferably SEQ ID NO: 25, 26, 66 or 67).

In another embodiment, the chimeric receptor of the invention comprises a B cell surface marker binding domain; a hinge domain of IgG4 (preferably SEQ ID NO: 15); a transmembrane domain of CD8 (preferably SEQ ID NO: 21); an intracellular domain of 4-1BB (preferably SED ID NO: 29); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of IgG4 (preferably SEQ ID NO: 15); a transmembrane domain of CD8 (preferably SEQ ID NO: 21); an intracellular domain of 4-1BB (preferably SEQ ID NO: 29); an intracellular domain of CD28 (preferably SEQ ID NO: 31); and a CD3-zeta primary signaling domain (preferably SEQ ID NO: 25, 26, 66 or 67). In one embodiment, the chimeric receptor of the invention comprises a B cell surface marker binding domain; a hinge domain of IgG4 (preferably SEQ ID NO: 15); a transmembrane domain of CD8 (preferably SEQ ID NO: 21); an intracellular domain of CD27 (preferably SEQ ID NO: 30); an intracellular domain of CD28 (preferably SED ID NO: 31); and a CD3-zeta primary signaling domain (preferably SEQ ID NO: 25, 26, 66 or 67).

In one embodiment, the chimeric receptor of the invention comprises a B cell surface marker binding domain; a hinge domain of IgG4 (preferably SEQ ID NO: 15); a transmembrane domain of CD28 (preferably SEQ ID NO: 23); an intracellular domain of 4-1BB (preferably SED ID NO: 29); an intracellular domain of CD27 (preferably SED ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of IgG4 (preferably SEQ ID NO: 15); a transmembrane domain of CD28 (preferably SEQ ID NO: 23); an intracellular domain of 4-1BB (preferably SEQ ID NO: 29); an intracellular domain of CD28 (preferably SEQ ID NO: 31); and a CD3-zeta primary signaling domain (preferably SEQ ID NO: 25, 26, 66 or 67). In one embodiment, the chimeric receptor of the invention comprises a B cell surface marker binding domain; a hinge domain of IgG4 (preferably SEQ ID NO: 15); a transmembrane domain of CD28 (preferably SEQ ID NO: 23); an intracellular domain of CD27 (preferably SED ID NO: 30); an intracellular domain of CD28 (preferably SEQ ID NO: 31); and a CD3-zeta primary signaling domain (preferably SEQ ID NO: 25, 26, 66 or 67).

In another embodiment, the chimeric receptor of the invention comprises a B cell surface marker binding domain; a hinge domain of IgD, preferably comprising the amino acid sequence SEQ ID NO: 17; a transmembrane domain of CD8 (preferably SEQ ID NO: 21); an intracellular domain of 4-1BB (preferably SEQ ID NO: 29); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of IgD (preferably SEQ ID NO: 17); a transmembrane domain of CD8 (preferably SEQ ID NO: 21); an intracellular domain of 4-1BB (preferably SED ID NO: 29); an intracellular domain of CD28 (preferably SEQ ID NO: 31); and a CD3-zeta primary signaling domain (preferably SEQ ID NO: 25, 26, 66 or 67). In one embodiment, the chimeric receptor of the invention comprises a B cell surface marker binding domain; a hinge domain of IgD (preferably SEQ ID NO: 17); a transmembrane domain of CD8 (preferably SEQ ID NO: 21); an intracellular domain of CD27 (preferably SEQ ID NO: 30); an intracellular domain of CD28 (preferably SEQ ID NO: 31); and a CD3-zeta primary signaling domain (preferably SEQ ID NO: 25, 26, 66 or 67).

In one embodiment, the chimeric receptor of the invention comprises a B cell surface marker binding domain; a hinge domain of IgD (preferably SEQ ID NO: 17); a transmembrane domain of CD28 (preferably SEQ ID NO: 23); an intracellular domain of 4-1BB (preferably SED ID NO: 29); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of IgD (preferably SEQ ID NO: 17); a transmembrane domain of CD28 (preferably SEQ ID NO: 23); an intracellular domain of 4-1BB (preferably SED ID NO: 29); an intracellular domain of CD28 (preferably SED ID NO: 31); and a CD3-zeta primary signaling domain (preferably SEQ ID NO: 25, 26, 66 or 67). In one embodiment, the chimeric receptor of the invention comprises a B cell surface marker binding domain; a hinge domain of IgD (preferably SEQ ID NO: 17); a transmembrane domain of CD28 (preferably SEQ ID NO: 23); an intracellular domain of CD27 (preferably SED ID NO: 30); an intracellular domain of CD28 (preferably SED ID NO: 31); and a CD3-zeta primary signaling domain (preferably SEQ ID NO: 25, 26, 66 or 67).

In another embodiment, the chimeric receptor of the invention comprises a B cell surface marker binding domain; a hinge domain of CD28 (preferably SEQ ID NO: 19); a transmembrane domain of CD8 (preferably SEQ ID NO: 21); an intracellular domain of 4-1BB (preferably SED ID NO: 29); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of CD28 (preferably SEQ ID NO: 19); a transmembrane domain of CD8 (preferably SEQ ID NO: 21); an intracellular domain of 4-1BB (preferably SED ID NO: 29); an intracellular domain of CD28 (preferably SEQ ID NO: 31); and a CD3-zeta primary signaling domain (preferably SEQ ID NO: 25, 26, 66 or 67). In one embodiment, the chimeric receptor of the invention comprises a B cell surface marker binding domain; a hinge domain of CD28 (preferably SEQ ID NO: 19); a transmembrane domain of CD8 (preferably SEQ ID NO: 21); an intracellular domain of CD27 (preferably SEQ ID NO: 30); an intracellular domain of CD28 (preferably SEQ ID NO: 31); and a CD3-zeta primary signaling domain (preferably SEQ ID NO: 25, 26, 66 or 67).

In one embodiment, the chimeric receptor of the invention comprises a B cell surface marker binding domain; a hinge domain of CD28 (preferably SEQ ID NO: 19); a transmembrane domain of CD28 (preferably SEQ ID NO: 23); an intracellular domain of 4-1BB (preferably SEQ ID NO: 29); an intracellular domain of CD27 (preferably SEQ ID NO: 30); and a CD3-zeta primary signaling domain (preferably SEQ ID NO: 25, 26, 66 or 67). In one embodiment, the chimeric receptor of the invention comprises a B cell surface marker binding domain; a hinge domain of CD28 (preferably SEQ ID NO: 19); a transmembrane domain of CD28 (preferably SEQ ID NO: 23); an intracellular domain of 4-1BB (preferably SEQ ID NO: 29); an intracellular domain of CD28 (preferably SEQ ID NO: 31); and a CD3-zeta primary signaling domain (preferably SEQ ID NO: 25, 26, 66 or 67). In one embodiment, the chimeric receptor of the invention comprises a B cell surface marker binding domain; a hinge domain of CD28 (preferably SEQ ID NO: 19); a transmembrane domain of CD28 (preferably SEQ ID NO: 23); an intracellular domain of CD27 (preferably SED ID NO: 30); an intracellular domain of CD28 (preferably SED ID NO: 31); and a CD3-zeta primary signaling domain (preferably SEQ ID NO: 25, 26, 66 or 67).

In one embodiment, the chimeric receptor of the invention comprises (i) a B cell surface marker binding domain, (ii) a hinge region of human CD28, (iii) a transmembrane domain of human CD28, (iv) an intracellular domain of human CD28 and (v) an intracellular domain of human CD3ζ chain.

In one embodiment, the part of the chimeric receptor comprising a hinge region of human CD28, a transmembrane domain of human CD28, an intracellular domain of human CD28 and an intracellular domain of human CD3ζ chain corresponds to the amino acid sequence of SEQ ID NO: 35 or 68 or an amino acid sequence with 95-99% identity to SEQ ID NO: 35 or 68.

In one embodiment, the chimeric receptor of the invention comprises a B cell surface marker binding domain, linked to an amino acid sequence of SEQ ID NO: 35 or 68 or a sequence or an amino acid sequence with 95-99% identity to SEQ ID NO: 35 or 68.

In another embodiment, the chimeric receptor of the invention comprises (i) a B cell surface marker binding domain, (ii) a hinge region of human CD8, (iii) a transmembrane domain of human CD8, (iv) an intracellular domain of human 4-1BB and (v) an intracellular domain of human CD3ζ. In one embodiment, the part of the chimeric receptor comprising a hinge region of human CD8, a transmembrane domain of human CD8, an intracellular domain of human 4-1BB and an intracellular domain of human CD3ζ comprises or consists in the amino acid sequence SEQ ID NO: 49, 50, 77 or 78, or any amino acid sequence with 95-99% identity with SEQ ID NO: 49, 50, 77 or 78.

In another embodiment, the chimeric receptor of the invention comprises (i) a B cell surface marker binding domain, (ii) a hinge region of human CD8, (iii) a transmembrane domain of human CD8, (iv) an intracellular domain of human CD28 and (v) an intracellular domain of human CD3ζ. In one embodiment, the part of the chimeric receptor comprising a hinge region of human CD8, a transmembrane domain of human CD8, an intracellular domain of human CD28 and an intracellular domain of human CD3ζ comprises or consists in the amino acid sequence SEQ ID NO: 51 or 79, or any amino acid sequence with 95-99% identity with SEQ ID NO: 51 or 79.

In one embodiment, the chimeric receptor of the invention comprises an anti-CD 19 scFv (e.g. FMC63), a hinge region of CD8, a transmembrane domain of human CD8, an intracellular domain of human 4-1BB and an intracellular domain of human CD3ζ. In one embodiment, said chimeric receptor comprises or consists in SEQ ID NO: 41, 43, 70 or 72 or an amino acid sequence with 95-99% identity to SEQ ID NO: 41, 43, 70 or 72.

In one embodiment, the chimeric receptor of the invention comprises a leader sequence of CD8, an anti-CD19 scFv (e.g. FMC63), a hinge region of CD8, a transmembrane domain of human CD8, an intracellular domain of human 4-1BB and an intracellular domain of human CD3ζ. In one embodiment, said chimeric receptor comprises or consists in SEQ ID NO: 40, 42, 69 or 71 or an amino acid sequence with 95-99% identity to SEQ ID NO: 40, 42, 69 or 71.

In one embodiment, the chimeric receptor of the invention comprises an anti-CD 19 scFv (e.g. FMC63), a hinge region of CD8, a transmembrane domain of human CD8, an intracellular domain of human CD28 and an intracellular domain of human CD3ζ. In one embodiment, said chimeric receptor comprises or consists in SEQ ID NO: 48, 53, 76 or 81 or an amino acid sequence with 95-99% identity to SEQ ID NO: 48, 53, 76 or 81.

In one embodiment, the chimeric receptor of the invention comprises a leader sequence of CD8, an anti-CD19 scFv (e.g. FMC63), a hinge region of CD8, a transmembrane domain of human CD8, an intracellular domain of human CD28 and an intracellular domain of human CD3ζ. In one embodiment, said chimeric receptor comprises or consists in SEQ ID NO: 47, 52, 75 or 80 or an amino acid sequence with 95-99% identity to SEQ ID NO: 47, 52, 75 or 80.

In one embodiment, the chimeric receptor of the invention comprises an anti-CD20 scFv, a hinge region of CD8, a transmembrane domain of human CD8, an intracellular domain of human 4-1BB and an intracellular domain of human CD3ζ. In one embodiment, said chimeric receptor comprises or consists in SEQ ID NO: 45, 55, 58, 61, 74, 83, 85 or 87 or an amino acid sequence with 95-99% identity to SEQ ID NO: 45, 55, 58, 61, 74, 83, 85 or 87.

In one embodiment, the chimeric receptor of the invention comprises a leader sequence of CD8, an anti-CD20 scFv, a hinge region of CD8, a transmembrane domain of human CD8, an intracellular domain of human 4-1BB and an intracellular domain of human CD3ζ. In one embodiment, said chimeric receptor comprises or consists in SEQ ID NO: 44, 54, 57, 60, 73, 82, 84 or 86 or an amino acid sequence with 95-99% identity to SEQ ID NO: 44, 54, 57, 60, 73, 82, 84 or 86.

In one embodiment, the cells of the monospecific Treg cell population of the invention express at their cell surface a chimeric receptor of the invention, and another receptor (herein referred to as "second receptor"), that binds to another ligand than the B cell surface marker recognized by the chimeric receptor of the invention. According to the invention, this other receptor comprises an extracellular ligand binding domain, optionally a hinge, optionally a transmembrane domain, and an intracellular signaling domain, as previously described.

In one embodiment, the second receptor is endogenous (such as, for example, the endogenous TCR). In another embodiment, the second receptor is exogenous, and its expression is induced in the cells of the monospecific Treg cell population of the invention by transformation or transduction of a nucleic acid encoding it. Said exogenous receptor may be an exogenous TCR or a chimeric receptor. Therefore, in one embodiment, the cells of the monospecific Treg cell population of the invention express two chimeric receptors, wherein the first one recognizes a B cell surface marker on a B cell, and the second one recognizes a distinct ligand.

In one embodiment, the chimeric receptor of the invention comprises a first intracellular signaling domain, and the second receptor comprises a distinct second intracellular signaling domain. In a first embodiment, the chimeric receptor of the invention comprises a T cell primary signaling domain (such as, for example, CD3zeta), and the second receptor comprises a costimulatory signaling domain (such as, for example, of 4-1BB, CD28 or a combination of costimulatory signaling domain of 4-1BB and CD28). In a second embodiment, the chimeric receptor of the invention comprises a costimulatory signaling domain (such as, for example, of 4-1BB, CD28 or a combination of costimulatory signaling domain of 4-1BB and CD28), and the second receptor comprises a T cell primary signaling domain (such as, for example, CD3zeta).

Consequently, according to these embodiments, the complete activation of the monospecific Treg cell population of the invention requires both the binding of the chimeric receptor of the invention to a B cell surface marker on B cells, and the binding of the second receptor to the ligand to which it is directed.

In one embodiment, the ligand recognized by the second receptor is expressed or present at the diseased tissue or organ, or at the site of the autoimmune response. Consequently, cytotoxicity for B cells will be induced only at the diseased tissue or organ or at the site of the autoimmune response, when said ligand will be present and recognized by the second receptor on the cells of the monospecific Treg cell population.

In one embodiment, the chimeric receptor of the invention further comprises an extracellular ligand binding domain recognizing a ligand distinct from the B cell surface marker recognized by the chimeric receptor. In one embodiment, said ligand binding domain is an antibody or an antigen binding fragment thereof.

In one embodiment, the chimeric receptor of the invention comprises an extracellular ligand binding domain comprising a B cell surface marker binding domain and another ligand binding domain recognizing a ligand distinct from said B cell surface marker. In one embodiment, said ligand binding domain is a bifunctional antibody recognizing both the B cell surface marker and the distinct ligand.

The invention also relates to an isolated and/or substantially purified monospecific Treg cell population as defined hereinabove.

Thus, one object of the invention is an isolated and/or substantially purified monospecific Treg cell population, wherein the cells of the population comprise a chimeric receptor, wherein said chimeric receptor recognizes a B cell surface marker on a B cell, and wherein said cells are cytotoxic for B cells, preferably are cytotoxic for B cells expressing at their surface the B cell surface marker recognized by the chimeric receptor.

As used herein, "isolated population" refers to a cell population that is removed from its natural environment (such as the peripheral blood) and that is isolated, purified or separated, and is at least about 75% free, 80% free, 85% free and preferably about 90%, 95%, 96%, 97%, 98%, 99% free, from other cells with which it is naturally present, but which lack the cell surface markers based on which the cells were isolated.

In one embodiment, the isolated monospecific Treg cell population of the invention has been frozen and thawed.

Another object of the invention is a vector comprising the nucleic acid sequence encoding a chimeric receptor (CR) as described hereinabove.

Examples of vectors that may be used in the present invention include, but are not limited to, a DNA vector, a RNA vector, a plasmid, a phagemid, a phage derivative, an animal virus and a cosmid.

Viral vector technology is well known in the art and is described, for example, in Sambrook et al. (2001, Molecular Cloning: A Laboratory Manual, Cold Spring Harbor Laboratory, New York), and in other virology and molecular biology manuals. Viruses, which are useful as vectors include, but are not limited to, retroviruses, adenoviruses, adeno- associated viruses, herpes viruses, and lentiviruses.

In general, a suitable vector contains an origin of replication functional in at least one organism, a promoter sequence, convenient restriction endonuclease sites, and one or more selectable markers, (e.g., WO01/96584; WO01/29058; and US6,326,193 incorporated herein by reference).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor - la (EF-la). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the invention should not be limited to the use of constitutive promoters. Inducible promoters are also contemplated as part of the invention. The use of an inducible promoter provides a molecular switch capable of turning on expression of the polynucleotide sequence which it is operatively linked to when such expression is desired, or turning off the expression when expression is not desired. Examples of inducible promoters include, but are not limited to a metallothionine promoter, a glucocorticoid promoter, a progesterone promoter, and a tetracycline promoter. In addition, bi-directional promoters allowing efficient and coordinate expression of two or more genes may also be of interest in the present invention. Examples of bi-directional promoters include but are not limited to the promoters described by Luigi Naldini in US2006200869, incorporated herein by reference, disclosing a bi-directional promoter comprising i) a first minimal promoter sequence derived from cytomegalovirus (CMV) or mouse mammary tumor virus (MMTV) genomes and ii) a full efficient promoter sequence derived from an animal gene.

In order to assess the expression of a CR polypeptide or portions thereof, the expression vector to be introduced into a T cell can also contain either a selectable marker gene such as CD34 or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

In some embodiments of the invention, suicide gene technology may be used. Different suicide gene technologies are described in the art depending on their mechanism of action (Jones et al. Frontiers in Pharmacology, 2014 (5): 254). Examples of gene-directed enzyme prodrug therapy (GDEPT) converting a nontoxic drug to a toxic drug include herpes simplex virus thymidine kinase (HSV-TK) and cytosine deaminase (CD). Other examples are chimeric proteins composed of a drug binding domain linked to apoptotic components such as for example the inducible Fas (iFas) or the inducible Caspase 9 (iCasp9) systems. Other examples include systems mediated by therapeutic antibodies such as inducing overexpression of c-myc at the surface of the engineered cell to induce their deletion by administration of an anti-c-myc antibody. The use of EGFR is described as a similar system compared to the c-myc system.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, US5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and in vivo is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL). Stock solutions of lipids in chloroform or chloroform/methanol can be stored at about -20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the invention.

In one embodiment, the genetically modified T cells of the invention are modified through the introduction of RNA. In one embodiment, an *in vitro* transcribed RNA CR can be introduced to a cell as a form of transient transfection. The RNA is produced by *in vitro* transcription using a polymerase chain reaction (PCR)-generated template. DNA of interest from any source can be directly converted by PCR into a template for *in vitro* mRNA synthesis using appropriate primers and RNA polymerase. The source of the DNA can be, for example, genomic DNA, plasmid DNA, phage DNA, cDNA, synthetic DNA sequence or any other appropriate source of DNA. The desired template for *in vitro* transcription is the CR of the present invention.

In one embodiment, the DNA to be used for PCR contains an open reading frame. The DNA can be from a naturally occurring DNA sequence from the genome of an organism. In one embodiment, the DNA is a full length gene of interest or a portion of a gene. The gene can include some or all of the 5' and/or 3' untranslated regions (UTRs). The gene can include exons and introns. In one embodiment, the DNA to be used for PCR is a human gene. In another embodiment, the DNA to be used for PCR is a human gene including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for *in vitro* transcription of mRNA which is used for transfection. Methods for performing PCR are well known in the art. Primers for use in PCR are designed to have regions that are substantially complementary to regions of the DNA to be used as a template for the PCR. "Substantially complementary", as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a gene that is normally transcribed in cells (the open reading frame), including 5' and 3' UTRs. The primers can also be designed to amplify a portion of a gene that encodes a particular domain of interest. In one embodiment, the primers are designed to amplify the coding region of a human cDNA, including all or portions of the 5' and 3' UTRs. Primers useful for PCR are generated by synthetic methods that are well known in the art.

"Forward primers" are primers that contain a region of nucleotides that are substantially complementary to nucleotides on the DNA template that are upstream of the DNA sequence that is to be amplified. "Upstream" is used herein to refer to a location 5', to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA preferably has 5' and 3' UTRs. In one embodiment, the 5' UTR is between zero and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA. The 5' and 3' UTRs can be the naturally occurring, endogenous 5' and 3' UTRs for the gene of interest. Alternatively, UTR sequences that are not endogenous to the gene of interest can be added by incorporating the UTR sequences into the forward and reverse primers or by any other modifications of the template. The use of UTR sequences that are not endogenous to the gene of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous gene. Alternatively, when a 5' UTR that is not endogenous to the gene of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments, the 5' UTR can be derived from an RNA virus whose RNA genome is stable in cells. In other embodiments, various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In a preferred embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatemeric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270: 1485-65 (2003).

The conventional method of integration of polyA/T stretches into a DNA template is molecular cloning. However, polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (size can be 50-5000 T), or after PCR by any other method, including, but not limited to, DNA ligation or *in vitro* recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines.

Poly(A) tails of RNAs can be further extended following *in vitro* transcription with the use of a poly(A) polymerase, such as E. coli polyA polymerase (E-PAP). In one embodiment, increasing the length of a poly(A) tail from 100 nucleotides to between 300 and 400 nucleotides results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on RNAs also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7: 1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

RNA can be introduced into target cells using any of a number of different methods, for instance, commercially available methods which include, but are not limited to, electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany), cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001).

In one embodiment, the CR sequences are delivered into cells using a retroviral or lentiviral vector. CR-expressing retroviral and lentiviral vectors can be delivered into different types of eukaryotic cells as well as into tissues and whole organisms using transduced cells as carriers or cell-free local or systemic delivery of encapsulated, bound or naked vectors. The method used can be for any purpose where stable expression is required or sufficient.

In another embodiment, the CR sequences are delivered into cells using *in vitro* transcribed mRNA. *In vitro* transcribed mRNA CR can be delivered into different types of eukaryotic cells as well as into tissues and whole organisms using transfected cells as carriers or cell-free local or systemic delivery of encapsulated, bound or naked mRNA. The method used can be for any purpose where transient expression is required or sufficient.

In another embodiment, the desired CR can be expressed in the cells by way of transposons.

Prior to expansion and genetic modification of the T cells of the invention, a source of T cells is obtained from a subject. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present invention, any number of T cell lines available in the art, may be used. In certain embodiments of the present invention, T cells can be obtained from a unit of blood collected from a subject using any number of techniques known to the skilled artisan, such as Ficoll™ separation. In one preferred embodiment, cells from the circulating blood of an individual are obtained by apheresis. The apheresis product typically contains lymphocytes, including T cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing steps. In one embodiment of the invention, the cells are washed with phosphate buffered saline (PBS). In an alternative embodiment, the wash solution lacks calcium and may lack magnesium or may lack many if not all divalent cations. After washing, the cells may be resuspended in a variety of biocompatible buffers, such as, for example, Ca²⁺-free, Mg²⁺-free PBS, PlasmaLyte A, or other saline solution with or without buffer. Alternatively, the undesirable components of the apheresis sample may be removed and the cells directly resuspended in culture media.

In another embodiment, T cells are isolated from peripheral blood lymphocytes by lysing the red blood cells and depleting the monocytes, for example, by centrifugation through a PERCOLL™ gradient or by counterflow centrifugal elutriation. A specific subpopulation of T cells can be further isolated by positive or negative selection techniques. For example, in one embodiment, T cells are isolated by incubation with anti-CD3/anti-CD28 (i.e., 3x28)-conjugated beads, such as DYNABEADS® M-450 CD3/CD28 T, for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another preferred embodiment, the time period is 10 to 24 hours. In one preferred embodiment, the incubation time period is 24 hours. For isolation of T cells from patients with leukemia, use of longer incubation times, such as 24 hours, can increase cell yield. Longer incubation times may be used to isolate T cells in any situation where there are few T cells as compared to other cell types, such in isolating tumor infiltrating lymphocytes (TIL) from tumor tissue or from immune-compromised individuals. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points. The skilled artisan would recognize that multiple rounds of selection can also be used in the context of this invention.

In another embodiment, it may be desirable to perform the selection procedure and use the "unselected" cells in the activation and expansion process. "Unselected" cells can also be subjected to further rounds of selection. Enrichment of a T cell population by negative selection can be accomplished with a combination of antibodies directed to surface markers unique to the negatively selected cells. One method is cell sorting and/or selection via negative magnetic immuno-adherence or flow cytometry that uses a cocktail of monoclonal antibodies directed to cell surface markers present on the cells negatively selected. For example, to enrich for CD4+ cells by negative selection, a monoclonal antibody cocktail typically includes antibodies to CD 14, CD20, CD11b, CD16, HLA-DR, and CD8. In certain embodiments, T regulatory cells are depleted by anti-C25 conjugated beads or other similar method of selection.

For isolation of a desired population of cells by positive or negative selection, the concentration of cells and surface (e.g., particles such as beads) can be varied. In certain embodiments, it may be desirable to significantly decrease the volume in which beads and cells are mixed together (i.e., increase the concentration of cells), to ensure maximum contact of cells and beads. For example, in one embodiment, a concentration of 2 billion cells/ml is used. In one embodiment, a concentration of 1 billion cells/ml is used. In a further embodiment, greater than 100 million cells/ml is used. In a further embodiment, a concentration of cells of 10, 15, 20, 25, 30, 35, 40, 45, or 50 million cells/ml is used. In yet another embodiment, a concentration of cells from 75, 80, 85, 90, 95, or 100 million cells/ml is used. In further embodiments, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (i.e., leukemic blood, tumor tissue, etc.). Such populations of cells may have therapeutic value and would be desirable to obtain.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20°C or in liquid nitrogen.

In certain embodiments, cryopreserved cells are thawed and washed as described herein and allowed to rest for one hour at room temperature prior to activation.

Also contemplated in the context of the invention is the collection of blood samples or apheresis product from a subject at a time period prior to when the expanded cells as described herein might be needed. As such, the source of the cells to be expanded can be collected at any time point necessary, and desired cells, such as T cells, isolated and frozen for later use in T cell therapy for any number of diseases or conditions that would benefit from T cell therapy, such as those described herein. In one embodiment, a blood sample or an apheresis is taken from a generally healthy subject. In certain embodiments, a blood sample or an apheresis is taken from a generally healthy subject who is at risk of developing a disease, but who has not yet developed a disease, and the cells of interest are isolated and frozen for later use. In certain embodiments, the T cells may be expanded, frozen, and used at a later time.

Whether prior to or after genetic modification of the Treg cells to express a desirable CR, the T cells can be activated and expanded generally using methods as described, for example, in US6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and US20060121005, incorporated herein by reference. Generally, the monospecific Treg cell population of the invention is expanded by contact with a surface having attached thereto an agent that stimulates a CD3/TCR complex associated signal and a ligand that stimulates a co-stimulatory molecule on the surface of the cells of the monospecific Treg cell population. In particular, the monospecific Treg cell population may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4⁺ T cells, an anti-CD3 antibody and an anti-CD28 antibody may be used. Examples of an anti-CD28 antibody include, without being limited to, 9.3, B-T3, XR-CD28 (Diaclone, Besancon, France). Other expansion methods commonly known in the art can be used (Berg et al., Transplant Proc. 30(8):3975-3977, 1998; Haanen et al., J. Exp. Med. 190(9): 13191328, 1999; Garland et al., J. Immunol Meth. 227(1-2):53-63, 1999).

In certain embodiments, the primary stimulatory signal and the co-stimulatory signal for the Treg cells of the monospecific Treg cell population may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one embodiment, the agent providing the co-stimulatory signal is bound to a cell surface and the agent providing the primary activation signal is in solution or coupled to a surface. In certain embodiments, both agents can be in solution. In another embodiment, the agents may be in soluble form, and then cross-linked to a surface, such as a cell expressing Fc receptors or an antibody or other binding agent which will bind to the agents. In this regard, see for example, US20040101519 and 20060034810, incorporated herein by reference, for artificial antigen presenting cells (aAPCs) that are contemplated for use in activating and expanding T cells in the present invention.

In one embodiment, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the co-stimulatory signal is an anti-CD28 antibody or antigen-binding fragment thereof; and both agents are co-immobilized to the same bead in equivalent molecular amounts. In one embodiment, a 1:1 ratio of each antibody bound to the beads for CD4+ T cell expansion and T cell growth is used. In certain aspects of the present invention, a ratio of anti CD3:CD28 antibodies bound to the beads is used such that an increase in T cell expansion is observed as compared to the expansion observed using a ratio of 1:1. In one particular embodiment an increase of from about 1 to about 3 fold is observed as compared to the expansion observed using a ratio of 1:1. In one embodiment, the ratio of CD3:CD28 antibody bound to the beads ranges from 100:1 to 1:100 and all integer values there between. In one aspect of the present invention, more anti-CD28 antibody is bound to the particles than anti-CD3 antibody, i.e., the ratio of CD3:CD28 is less than one. In certain embodiments of the invention, the ratio of anti-CD28 antibody to anti CD3 antibody bound to the beads is greater than 2:1. In one particular embodiment, a 1:100 CD3:CD28 ratio of antibody bound to beads is used. In another embodiment, a 1:75 CD3:CD28 ratio of antibody bound to beads is used. In a further embodiment, a 1:50 CD3:CD28 ratio of antibody bound to beads is used. In another embodiment, a 1:30 CD3:CD28 ratio of antibody bound to beads is used. In one preferred embodiment, a 1:10 CD3:CD28 ratio of antibody bound to beads is used. In another embodiment, a 1:3 CD3:CD28 ratio of antibody bound to the beads is used. In yet another embodiment, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain embodiments, the ratio of cells to particles ranges from 1:100 to 100:1 and any integer values in-between and in further embodiments the ratio comprises 1:9 to 9:1 and any integer values in between, can also be used to stimulate T cells. The ratio of anti-CD3- and anti-CD28-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one embodiment, a ratio of particles to cells of 1:1 or less is used. In one particular embodiment, a preferred particle: cell ratio is 1:5. In further embodiments, the ratio of particles to cells can be varied depending on the day of stimulation. For example, in one embodiment, the ratio of particles to cells is from 1:1 to 10: 1 on the first day and additional particles are added to the cells every day or every other day thereafter for up to 10 days, at final ratios of from 1:1 to 1:10 (based on cell counts on the day of addition). In one particular embodiment, the ratio of particles to cells is 1:1 on the first day of stimulation and adjusted to 1:5 on the third and fifth days of stimulation. In another embodiment, particles are added on a daily or every other day basis to a final ratio of 1:1 on the first day, and 1:5 on the third and fifth days of stimulation. In another embodiment, the ratio of particles to cells is 2:1 on the first day of stimulation and adjusted to 1:10 on the third and fifth days of stimulation. In another embodiment, particles are added on a daily or every other day basis to a final ratio of 1:1 on the first day, and 1:10 on the third and fifth days of stimulation. One of skill in the art will appreciate that a variety of other ratios may be suitable for use in the present invention. In particular, ratios will vary depending on particle size and on cell size and type.

In further embodiments of the present invention, the Treg cells are combined with agent-coated beads, the beads and the cells are subsequently separated, and then the cells are cultured. In an alternative embodiment, prior to culture, the agent-coated beads and cells are not separated but are cultured together. In a further embodiment, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the Treg cells of the monospecific Treg cell population. In one embodiment, the cells (for example, 10⁴ to 10⁹ T cells) and beads (for example, DYNABEADS® M-450 CD3/CD28 T paramagnetic beads at a ratio of 1: 1) are combined in a buffer, preferably PBS (without divalent cations such as, calcium and magnesium). Again, those of ordinary skill in the art can readily appreciate that any cell concentration may be used. For example, the target cell may be very rare in the sample and comprise only 0.01% of the sample or the entire sample (i.e., 100%) may comprise the target cell of interest. Accordingly, any cell number is within the context of the present invention. In certain embodiments, it may be desirable to significantly decrease the volume in which particles and cells are mixed together (i.e., increase the concentration of cells), to ensure maximum contact of cells and particles. For example, in one embodiment, a concentration of about 2 billion cells/ml is used. In another embodiment, greater than 100 million cells/ml is used. In a further embodiment, a concentration of cells of 10, 15, 20, 25, 30, 35, 40, 45, or 50 million cells/ml is used. In yet another embodiment, a concentration of cells from 75, 80, 85, 90, 95, or 100 million cells/ml is used. In further embodiments, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain embodiments.

In one embodiment of the present invention, the mixture may be cultured for several hours (about 3 hours) to about 14 days or any hourly integer value in between. In another embodiment, the mixture may be cultured for 21 days. In one embodiment of the invention the beads and the T cells are cultured together for about eight days. In another embodiment, the beads and T cells are cultured together for 2-3 days. Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, a-MEM, F- 12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37°C) and atmosphere (e.g., air plus 5% CO₂).

T cells that have been exposed to varied stimulation times may exhibit different characteristics. For example, typical blood or apheresed peripheral blood mononuclear cell products have a helper T cell population (Th, CD4⁺) that is greater than the cytotoxic or suppressor T cell population (Tc, CD8⁺). Ex vivo expansion of T cells by stimulating CD3 and CD28 receptors produces a population of T cells that prior to about days 8-9 consists predominately of Th cells, while after about days 8-9, the population of T cells comprises an increasingly greater population of Tc cells. Accordingly, depending on the purpose of treatment, infusing a subject with a T cell population comprising predominately of Th cells may be advantageous. Similarly, if an antigen- specific subset of Tc cells has been isolated it may be beneficial to expand this subset to a greater degree. Further, in addition to CD4 and CD8 markers, other phenotypic markers vary significantly, but in large part, reproducibly during the course of the cell expansion process. Thus, such reproducibility enables the ability to tailor an activated T cell product for specific purposes.

In one embodiment of the invention, the T cells may be cultured in the presence of rapamycin in order to obtain regulatory T cells, as described for example in WO2007110785 incorporated herein by reference. Another method to generate regulatory T cells is described in US2016024470 incorporated herein by reference, where T cells are cultured with a T cell receptor (TCR)/CD3 activator such as for example TCR/CD3 antibodies, a TCR co-stimulator activator such as for example CD28, CD137 (4-1 BB), GITR, B7-1/2, CD5, ICOS, OX40, CD40 or CD137 antibodies, and rapamycin.

In one embodiment of the invention, the T cells genetically modified by expression of the CR may also have been genetically modified by expression of at least one intracellular factor such as ROR-C, Foxp3, Foxo1, T-bet, or Gata 3, c-Maf, AhR. In one embodiment, the genetically modified immune cell of the invention expresses Foxp3. In one embodiment, the genetically modified immune cell of the invention expresses Foxo1.

In one embodiment, the genetically modified Treg cell of the monospecific Treg cell population can be an allogeneic Treg cell. For example, the allogeneic Treg cell can be a T cell lacking expression of a functional human leukocyte antigen (HLA), e.g., HLA class I and/or HLA class II.

In one embodiment, the cells of the monospecific Treg cell population as described herein can be engineered such that they do not express a functional HLA on its surface. For example, a Treg cell of the monospecific Treg cell population as described herein can be engineered such that cell surface expression HLA, e.g., HLA class 1 and/or HLA class II or non-classical HLA molecules is downregulated.

Modified Treg cells that lack expression of a functional HLA can be obtained by any suitable means, including a knock out or knock down of one or more subunit of HLA. For example, the Treg cell can include a knock down of HLA using siRNA, shRNA, clustered regularly interspaced short palindromic repeats (CRISPR) transcription-activator like effector nuclease (TALEN), zinc finger endonuclease (ZFN), meganuclease (mn, also known as homing endonuclease), or megaTAL (combining a TAL effector with a mn cleavage domain).

In one embodiment, HLA expression can be inhibited using siRNA or shRNA that targets a nucleic acid encoding a HLA in a T cell. Expression of siRNA and shRNAs in T cells can be achieved using any conventional expression system, e.g., such as a lentiviral expression system. Exemplary siRNA and shRNA that downregulate expression of HLA class I and/or HLA class II genes are described, e.g., in US2007/0036773.

"CRISPR" or "CRISPR to HLA" or "CRISPR to inhibit HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a HLA gene.

Naturally-occurring CRISPR/Cas systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. (2008) Science 322: 1843-1845. The CRISPR/Cas system has been modified for use in gene editing (silencing, enhancing or changing specific genes) in eukaryotes such as mice or primates. Wiedenheft et al. (2012) Nature 482: 331-8. This is accomplished by introducing into the eukaryotic cell a plasmid containing a specifically designed CRISPR and one or more appropriate Cas. The CRISPR sequence, sometimes called a CRISPR locus, comprises alternating repeats and spacers. In a naturally-occurring CRISPR, the spacers usually comprise sequences foreign to the bacterium such as a plasmid or phage sequence; in the HLA CRISPR/Cas system, the spacers are derived from the HLA gene sequence. RNA from the CRISPR locus is constitutively expressed and processed by Cas proteins into small RNAs. These comprise a spacer flanked by a repeat sequence. The RNAs guide other Cas proteins to silence exogenous genetic elements at the RNA or DNA level. Horvath et al. (2010) Science 327: 167-170; Makarova et al. (2006) Biology Direct 1: 7. The spacers thus serve as templates for RNA molecules, analogously to siRNAs. Pennisi (2013) Science 341: 833-836. The CRISPR/Cas system can thus be used to edit a HLA gene (adding or deleting a basepair), or introducing a premature stop which thus decreases expression of a HLA. The CRISPR/Cas system can alternatively be used like RNA interference, turning off HLA gene in a reversible fashion. In a mammalian cell, for example, the RNA can guide the Cas protein to a HLA promoter, sterically blocking RNA polymerases.

Artificial CRISPR/Cas systems can be generated which inhibit HLA, using technology known in the art, e.g., that described in US20140068797, and Cong (2013) Science 339: 819-823. Other artificial CRISPR/Cas systems that are known in the art may also be generated which inhibit HLA, e.g., that described in Tsai (2014) Nature Biotechnol., 32:6 569-576, US8,871,445; 8,865,406; 8,795,965; 8,771,945; and 8,697,359.

"TALEN" or "TALEN to HLA" or "TALEN to inhibit HLA" refers to a transcription activator- like effector nuclease, an artificial nuclease which can be used to edit the HLA gene. TALENs are produced artificially by fusing a TAL effector DNA binding domain to a DNA cleavage domain. Transcription activator-like effectors (TALEs) can be engineered to bind any desired DNA sequence, including a portion of the HLA gene. By combining an engineered TALE with a DNA cleavage domain, a restriction enzyme can be produced which is specific to any desired DNA sequence, including a HLA sequence. These can then be introduced into a cell, wherein they can be used for genome editing. Boch (2011) Nature Biotech. 29: 135-6; and Boch et al. (2009) Science 326: 1509-12; Moscou et al. (2009) Science 326: 3501.

TALEs are proteins secreted by Xanthomonas bacteria. The DNA binding domain contains a repeated, highly conserved 33-34 amino acid sequence, with the exception of the 12th and 13th amino acids. These two positions are highly variable, showing a strong correlation with specific nucleotide recognition. They can thus be engineered to bind to a desired DNA sequence. To produce a TALEN, a TALE protein is fused to a nuclease (N), which is a wild-type or mutated Fokl endonuclease. Several mutations to Fokl have been made for its use in TALENs; these, for example, improve cleavage specificity or activity. Cermak et al. (2011) Nucl. Acids Res. 39: e82; Miller et al. (2011) Nature Biotech. 29: 143-8; Hockemeyer et al. (2011) Nature Biotech. 29: 731-734; Wood et al. (2011) Science 333: 307; Doyon et al. (2010) Nature Methods 8: 74-79; Szczepek et al. (2007) Nature Biotech. 25: 786-793; and Guo et al. (2010) /. Mol. Biol. 200: 96. The Fokl domain functions as a dimer, requiring two constructs with unique DNA binding domains for sites in the target genome with proper orientation and spacing. Both the number of amino acid residues between the TALE DNA binding domain and the Fokl cleavage domain and the number of bases between the two individual TALEN binding sites appear to be important parameters for achieving high levels of activity. Miller et al. (2011) Nature Biotech. 29: 143-8. A HLA TALEN can be used inside a cell to produce a double- stranded break (DSB). A mutation can be introduced at the break site if the repair mechanisms improperly repair the break via non-homologous end joining. For example, improper repair may introduce a frame shift mutation. Alternatively, foreign DNA can be introduced into the cell along with the TALEN; depending on the sequences of the foreign DNA and chromosomal sequence, this process can be used to correct a defect in the HLA gene or introduce such a defect into a wt HLA gene, thus decreasing expression of HLA. TALENs specific to sequences in HLA can be constructed using any method known in the art, including various schemes using modular components. Zhang et al. (2011) Nature Biotech. 29: 149-53; Geibler et al. (2011) PLoS ONE 6: el9509.

"ZFN" or "Zinc Finger Nuclease" or "ZFN to HLA" or "ZFN to inhibit HLA" refer to a zinc finger nuclease, an artificial nuclease which can be used to edit the HLA gene. Like a TALEN, a ZFN comprises a Fokl nuclease domain (or derivative thereof) fused to a DNA-binding domain. In the case of a ZFN, the DNA-binding domain comprises one or more zinc fingers. Carroll et al. (2011) Genetics Society of America 188: 773-782; and Kim et al. (1996) Proc. Natl. Acad. Sci. USA 93: 1156-1160. A zinc finger is a small protein structural motif stabilized by one or more zinc ions. A zinc finger can comprise, for example, Cys₂His₂, and can recognize an approximately 3-bp sequence. Various zinc fingers of known specificity can be combined to produce multi-finger polypeptides which recognize about 6, 9, 12, 15 or 18-bp sequences. Various selection and modular assembly techniques are available to generate zinc fingers (and combinations thereof) recognizing specific sequences, including phage display, yeast one -hybrid systems, bacterial one -hybrid and two-hybrid systems, and mammalian cells.

Like a TALEN, a ZFN must dimerize to cleave DNA. Thus, a pair of ZFNs are required to target non-palindromic DNA sites. The two individual ZFNs must bind opposite strands of the DNA with their nucleases properly spaced apart. Bitinaite et al. (1998) Proc. Natl. Acad. Sci. USA 95: 10570-5. Also like a TALEN, a ZFN can create a double-stranded break in the DNA, which can create a frame-shift mutation if improperly repaired, leading to a decrease in the expression and amount of HLA in a cell. ZFNs can also be used with homologous recombination to mutate in the HLA gene. ZFNs specific to sequences in HLA can be constructed using any method known in the art. See, e.g., Provasi (2011) Nature Med. 18: 807-815; Torikai (2013) Blood 122: 1341-1349; Cathomen et al. (2008) Mol. Ther. 16: 1200-7; Quo et al. (2010) /. Mol. Biol. 400: 96; US2011/0158957; and US2012/0060230.

"Meganuclease" or "meganuclease to HLA" or "meganuclease to inhibit HLA" refers to a monomeric endonuclease with large (>14 base pairs) recognition sites, which can be used to edit the HLA gene. Meganucleases (mn) are monomeric proteins with innate nuclease activity that are derived from bacterial homing endonucleases and engineered for a unique target site. Homing endonucleases are DNA-cleaving enzymes that can generate double strand breaks at individual loci in their host genomes, and thereby drive site-specific gene conversion events. (Stoddard, Structure. 2011 Jan 12;19(1):7-15). Despite their small size, homing endonucleases recognize long DNA sequences (typically 20 to 30 base pairs). Homing endonucleases are extremely widespread and are found in microbes, as well as in phages and viruses. The LAGLIDADG and His-Cys box enzymes (which are the most sequence-specific of these enzymes) rely upon antiparallel β-sheets that dock into the major grooves of their DNA target sites (Flick et al., 1998; Jurica et al., 1998). There they establish a collection of sequence-specific and non-specific contacts that are distributed nonuniformly across multiple consecutive basepairs (Chevalier et al., 2003; Scalley-Kim et al., 2007).

The LAGLIDADG homing endonuclease (LHE) family is the primary source of the engineered enzymes used for gene targeting applications. The LHE family is primarily encoded within archaea and in the chloroplast and mitochondrial genomes of algae and fungi (Chevalier et al., 2005; Dalgaard et al., 1997; Sethuraman et al., 2009). Meganucleases that possess a single conserved LAGLIDADG motif (SEQ ID NO: 63) per protein chain form homodimeric proteins that cleave palindromic and nearly palindromic DNA target sequences, while those that contain two such motifs per protein chain form larger, pseudo-symmetric monomers that can target completely asymmetric DNA sequences.

Meganucleases can be engineered to target HLA and thus create a double-stranded break in the DNA, which can create a frame-shift mutation if improperly repaired, leading to a decrease in the expression and amount of HLA in a cell.

"MegaTAL" or "megaTAL to HLA" or " megaTAL to inhibit HLA" refers to an artificial nuclease, which can be used to edit the HLA gene. MegaTALs are hybrid monomeric nucleases obtained through the fusion of minimal TAL (transcription activator-like) effector domains to the N-terminus of meganucleases derived from the LAGLIDADG homing endonuclease family (Nucleic Acids Res. 2014 Feb;42(4):2591-601; Takeuchi et al, Methods Mol Biol. 2015;1239:105-32. doi: 10.1007/978-1-4939-1862-1_6). MegaTALs thus consist of a site-specific meganuclease cleavage head with additional affinity and specificity provided by a TAL effector DNA binding domain.

MegaTALs can be engineered to target HLA and thus create a double-stranded break in the DNA, which can create a frame-shift mutation if improperly repaired, leading to a decrease in the expression and amount of HLA in a cell.

While not wishing to be bound by any particular theory, in some embodiments, a therapeutic T cell has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117: 1466-1476 (2007). Thus, in an embodiment, the genetically modified Treg cell, ectopically expresses a telomerase subunit, e.g., the catalytic subunit of telomerase, e.g., TERT, e.g., hTERT. In some aspects, this disclosure provides a method of producing a CR-expressing cell, comprising contacting a cell with a nucleic acid encoding a telomerase subunit, e.g., the catalytic subunit of telomerase, e.g., TERT, e.g., hTERT. The cell may be contacted with the nucleic acid before, simultaneous with, or after being contacted with a construct encoding a CR.

Another object of the invention is a composition comprising, consisting or consisting essentially of at least one monospecific Treg cell population of the invention.

In one embodiment, said composition comprises, consist or consists essentially of at least one isolated and/or substantially purified monospecific Treg cell population of the invention.

In one embodiment, said composition has been frozen and thawed.

Another object of the invention is a pharmaceutical composition comprising, consisting or consisting essentially of at least one monospecific Treg cell population as described hereinabove and at least one pharmaceutically acceptable excipient.

Another object of the invention is a medicament comprising, consisting or consisting essentially of at least one monospecific Treg cell population as described hereinabove.

In one embodiment, the pharmaceutical composition or medicament comprises at least one isolated and/or substantially purified monospecific Treg cell population of the invention.

In one embodiment, the pharmaceutical composition or medicament comprises a combination of monospecific Treg cell populations as described hereinabove (i.e. at least two distinct monospecific Treg cell populations of the invention).

As used herein, the term "consisting essentially of", with reference to a pharmaceutical composition or medicament, means that the at least one monospecific Treg cell population of the invention is the only one therapeutic agent or agent with a biologic activity within said pharmaceutical composition or medicament.

Such compositions and medicaments may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

The administration of the compositions may be carried out in any convenient manner, including by aerosol inhalation, injection, ingestion, transfusion, implantation or transplantation. The compositions described herein may be administered to a patient trans arterially, subcutaneously, intradermally, intratumorally, intranodally, intramedullary, intramuscularly, by intravenous (i.v.) injection, or intraperitoneally. In one aspect, the T cell compositions of the present invention are administered to a patient by intradermal or subcutaneous injection.

In one embodiment, the monospecific Treg cell populations of the present invention are administered by i.v. injection.

The compositions of the present invention are in one embodiment formulated for intravenous administration.

In another embodiment, the monospecific Treg cell populations of the present invention may be injected directly into the site of the autoimmune disease.

Another object of the invention is a method for treating an autoimmune disease in a subject in need thereof, preferably an autoantibody-mediated autoimmune disease, wherein said method comprises administering a therapeutically effective amount of at least one monospecific Treg cell population as described hereinabove.

The present invention thus relates to at least one monospecific Treg cell population as described hereinabove (preferably in a composition, pharmaceutical composition or medicament as described hereinabove), for treating or for use in the treatment of an autoimmune disease, preferably an autoantibody-mediated autoimmune disease.

Examples of autoantibody-mediated autoimmune diseases include but are not limited to, bullous pemphigoid, lupus (including systemic lupus erythematosus (SLE), lupus nephritis (LN), discoid lupus, lupus erythematosus profundus, Chilbrain lupus erythematosus, tumidus lupus erythematosus, severe systemic lupus erythematosus, acute cutaneous lupus, chronic cutaneous lupus), multiple sclerosis, neuromyelitis optica (NMO), autoimmune limbic encephalitis (LE), Hashimoto's disease, N-methyl-D-aspartate receptor (NMDAR) encephalitis, autoimmune hemolytic anemia, pemphigus vulgaris, myasthenia gravis, Graves' disease, idiopathic thrombocytopenic purpura, Goodpasture's syndrome, rheumatoid arthritis, celiac disease, pernicious anemia, vitiligo, scleroderma, psoriasis, ulcerative colitis (UC), Crohn's disease, Sjogren's syndrome, Wegener granulomatosis, polymyositis, dermatomyositis, primary biliary cirrhosis, antiphospholipid syndrome, mixed connective tissue disease, Miller Fisher syndrome, Guillain-Barré syndrome, acute motor axonal neuropathy, autoimmune hepatitis, dermatitis herpetiformis, Churg-Strauss disease, microscopic polyangiitis, IgA nephropathy, vasculitis caused by ANCA and other ANCA associated diseases, acute rheumatic fever, pernicious anemia, type 1 diabetes (T1D), reactive arthritis (Reiter syndrome), membranous nephropathy, chronic inflammatory demyelinating polyneuropathy, thrombotic thrombocytopenic purpura, hyperviscosity in monoclonal gammopathies, hemolytic uremic syndrome (atypical, due to antibody to factor H), Wilson disease, fulminant, Lambert-Eaton myasthenic syndrome, RBC alloimmunization in pregnancy, mushroom poisoning, acute disseminated encephalomyelitis, hemolytic uremic syndrome (atypical, due to complement factor mutations), autoimmune hemolytic anemia (life-threatening cold agglutinin disease), myeloma cast nephropathy, post-transfusion purpura, autoimmune hemolytic anemia (warm autoimmune hemolytic anemia), hypertriglyceridemic pancreatitis, thyroid storm, stiff person syndrome, Hemolytic uremic syndrome (typical diarrhea-associated), immune thrombocytopenia, ABO-incompatible solid organ transplantation (SOT), cryoglobulinemia, heparin-induced thrombocytopenia, thyroid storm, chronic inflammatory demyelinating polyradiculoneuropathy, focal segmental glomerulosclerosis and fulminant hepatic failure. Preferably, said autoantibody-mediated autoimmune disease is selected from the group comprising bullous pemphigoid, lupus (including systemic lupus erythematosus (SLE), lupus nephritis (LN), discoid lupus, lupus erythematosus profundus, Chilbrain lupus erythematosus, tumidus lupus erythematosus, severe systemic lupus erythematosus, acute cutaneous lupus, chronic cutaneous lupus) and multiple sclerosis.

In one embodiment, the autoantibody-mediated autoimmune disease may be treated by apheresis. Examples of diseases that may be treated by apheresis include but are not limited to, systemic lupus erythematosus (severe, nephritis), multiple sclerosis, Guillain-Barre syndrome, Myasthenia gravis, chronic inflammatory demyelinating polyneuropathy, thrombotic thrombocytopenic purpura, hyperviscosity in monoclonal gammopathies, goodpasture syndrome, hemolytic uremic syndrome (atypical, due to antibody to factor H), Wilson disease, fulminant, Lambert-Eaton myasthenic syndrome, RBC alloimmunization in pregnancy, mushroom poisoning, acute disseminated encephalomyelitis, hemolytic uremic syndrome (atypical, due to complement factor mutations), autoimmune hemolytic anemia (life-threatening cold agglutinin disease), myeloma cast nephropathy, post-transfusion purpura, autoimmune hemolytic anemia (warm autoimmune hemolytic anemia), hypertriglyceridemic pancreatitis, thyroid storm, stiff person syndrome, Hemolytic uremic syndrome (typical diarrhea-associated), and immune thrombocytopenia.

In one embodiment, the autoantibody-mediated autoimmune disease may be treated by plasmapheresis. Examples of diseases that may be treated by plasmapheresis include but are not limited to, systemic lupus erythematosus, ABO-incompatible solid organ transplantation (SOT), thrombotic thrombocytopenic purpura, cryoglobulinemia, heparin-induced thrombocytopenia, thyroid storm, chronic inflammatory demyelinating polyradiculoneuropathy, ANCA associated diseases, focal segmental glomerulosclerosis, fulminant hepatic failure, myasthenia gravis, Goodpasture's syndrome, Guillain-Barré Syndrome, autoimmune hemolytic anemia, IgA nephropathy, hemolytic uremic syndrome.

In one embodiment, the autoantibody-mediated autoimmune disease may be treated by rituximab. Examples of autoantibody-mediated autoimmune diseases that may be treated by rituximab include, but are not limited to, lupus nephritis, systemic lupus erythematosus, rheumatoid arthritis, Wegener granulomatosis, microscopic polyangiitis, immune thrombocytopenic purpura (ITP), pemphigus vulgaris, sicca syndrome (Sjogren), glomerulonephritis, myasthenia gravis, liver transplant rejection, idiopathic thrombocytopenic purpura (immune thrombocytopenic purpura), kidney transplant rejection, hemophilia A, neuromyelitis optica (Devic's syndrome), pemphigus vulgaris, and thrombotic thrombocytopenic purpura.

In one embodiment, the subject (e.g., human) receives an initial administration of the monospecific Treg cell population of the invention, and one or more subsequent administrations, wherein the one or more subsequent administrations are administered less than 15 days, e.g., 14, 13, 12, 11, 10, 9, 8, 7, 6, 5, 4, 3, or 2 days after the previous administration.

In one embodiment, the amount of Treg cells of the at least one monospecific Treg cell population of the invention administered to the subject ranges from about 10² to about 10⁹, from about 10³ to about 10⁸, from about 10⁴ to about 10⁷, or from about 10⁵ to about 10⁶ cells.

In one embodiment, more than one administration of the at least one monospecific Treg cell population of the invention are administered to the subject (e.g., human) per week, e.g., 2, 3, or 4 administrations of the genetically modified CR monospecific Treg cell population of the invention are administered per week.

### BRIEF DESCRIPTION OF THE DRAWINGS

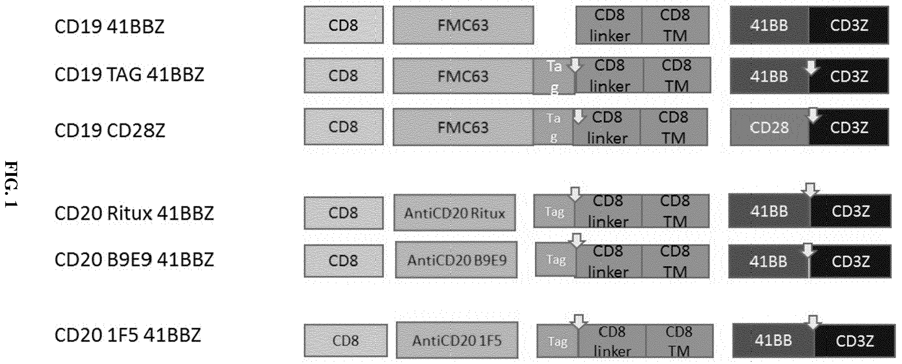

**Figure 1** is a scheme depicting the series of CR constructs designed and built. The CD 19 TAG 41BBZ construct was used as the template to build all the other CR constructs comprising a tag.

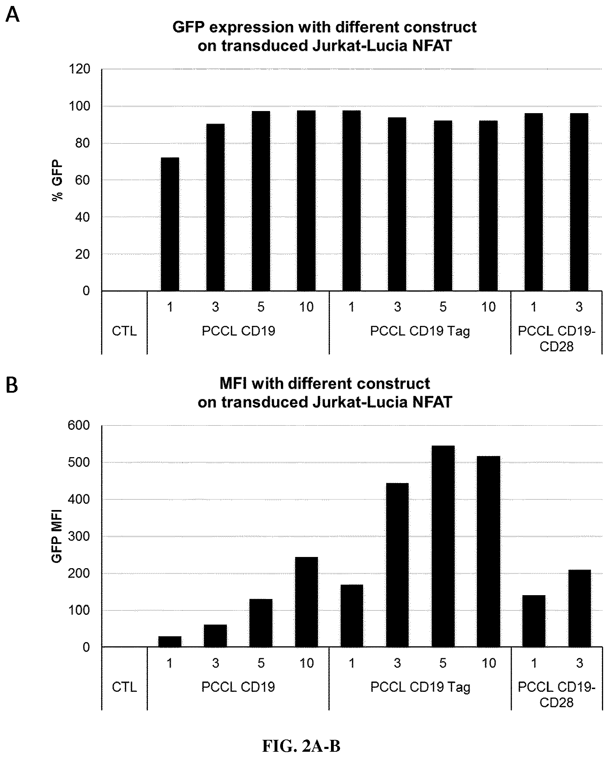

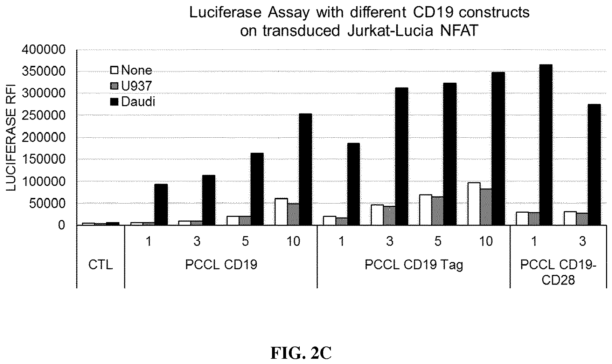

**Figure 2** is a combination of graph showing the expression of three CD19-CRs in Jurkat-Lucia NFAT cells transduced with the CD19-CR and the NFAT assay carried out with the transduced cells. Transduction efficiency was assessed by determining the percentage of GFP positive cells (**A**) and by determining the GFP mean fluorescence intensity or MFI (**B**). The number under each column indicates the MOI used for the transduction. NFAT assay on Jurkat-Lucia NFAT cells transduced with the CD19-CR constructs (**C**). U937 CD19⁻ cells were used as the control. Daudi B cells were used as the CD19⁺ target cells. The ratio of E (effector cells):T (target cells) is 1:1.

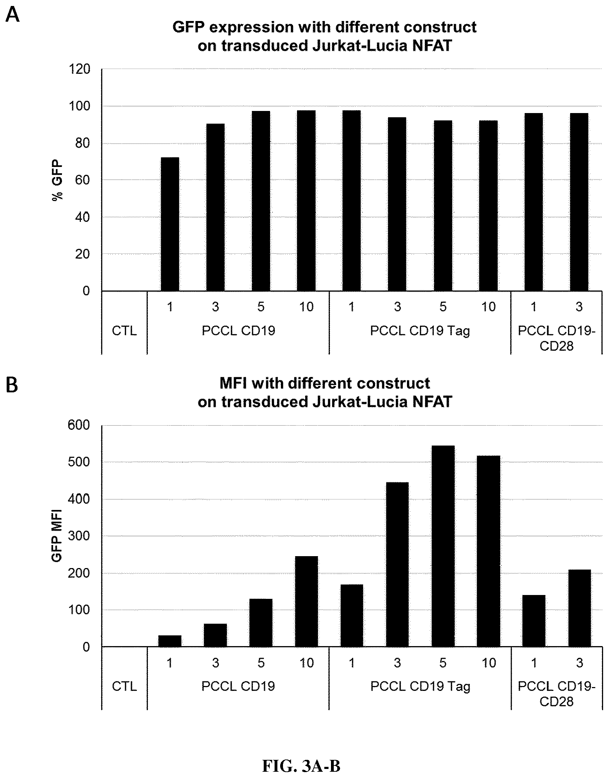

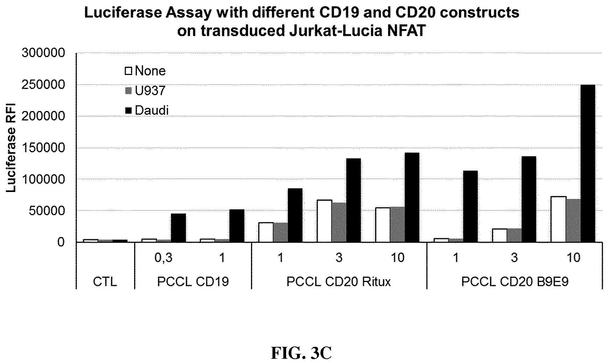

**Figure 3** is a combination of graphs showing the expression of two CD20-CRs in Jurkat-Lucia NFAT cells transduced with the CD20-CR and the NFAT assay carried out with the transduced cells. Transduction efficiency was assessed by determining the percentage of GFP positive cells (**A**) and by determining the GFP mean fluorescence intensity or MFI (**B**). The number under each column indicates the MOI used for the transduction. NFAT assay on Jurkat-Lucia NFAT cells transduced with the CD20-CR constructs (C). U937 CD19⁻ cells were used as the control. Daudi B cells were used as the CD19⁺ target cells. The ratio of E (effector cells):T (target cells) is 1:1.

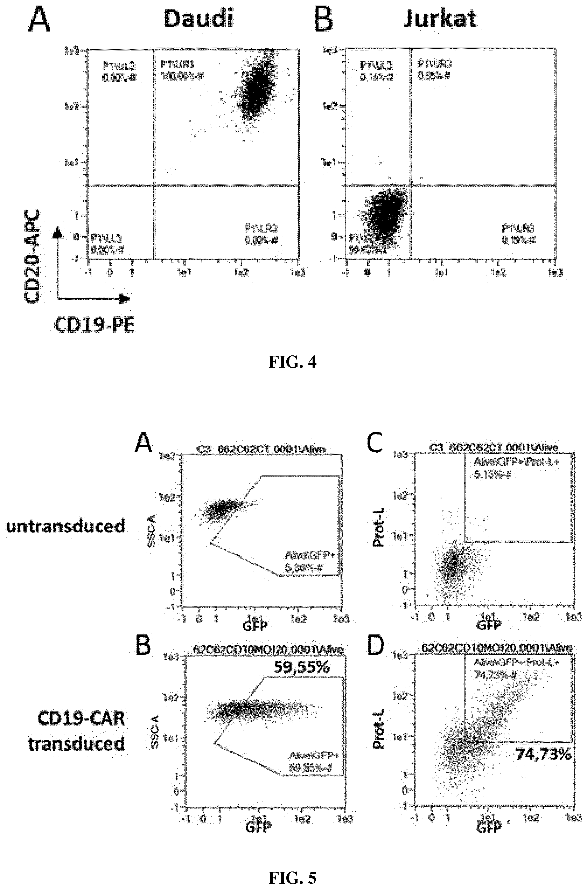

**Figure 4** is a combination of graphs showing the expression of CD20 and CD 19 in Daudi cells (**A**) and Jurkat cells (**B**).
**Figure 5** is a combination of graphs showing the OVA-specific Treg transduction efficacy and the expression of the transduced CR at the cell surface. The GFP expression was analyzed by flow cytometry in untransduced OVA-specific Tregs (**A**) and in CD19-CR transduced OVA-specific Tregs (**B**). The expression of the transduced CR at the cell surface was assessed by protein-L staining in untransduced OVA-specific Tregs (**C**) and in CD19-CR transduced OVA-specific Tregs (**D**).

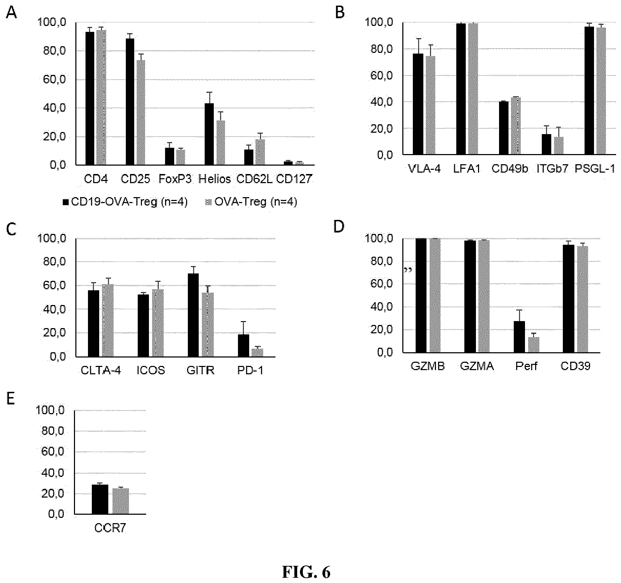

**Figure 6** is a combination of graphs showing the phenotypic characterization of untransduced and transduced OVA-specific Tregs. Markers associated with regulatory phenotype (**A**, CD4, CD25, FoxP3, Helios, CD62L, CD127), adhesion / transmigration (**B**, VLA-4, LFA1, CD49b, ITGβ7, PSGL-1), co-signalization (C, CTLA-4, ICOS, GITR, PD-1), cytolysis (**D**, Granzyme B (GZMB), Granzyme A (GZMA), Perforin (Perf)), metabolism disruption (**D**, CD39) and chemotaxis (**E**, CCR7) were evaluated by flow cytometry. Results are expressed as percentage of positive cells.

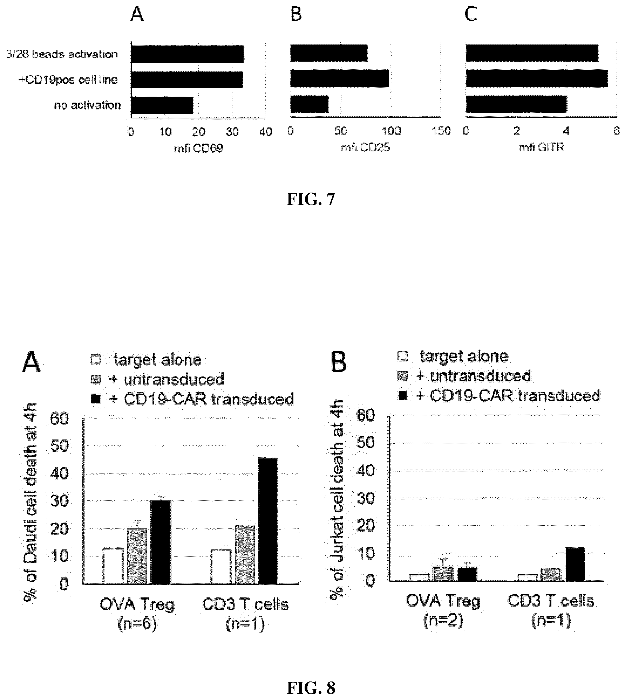

**Figure 7** is a combination of graphs showing the CR-mediated OVA-specific Treg activation. One day after activation with CD3/CD28 coated beads (at a 1:5 bead-to-cell ratio) or with the CD19⁺ cell line (CD19^{pos} cell line), the expression of CD69 (**A**), CD25 (**B**) and GITR (**C**) was measured on CD19-CR transduced OVA-specific Tregs by flow cytometry. As a control, the expression of CD69, CD25 and GITR in inactivated CD19-CR transduced OVA-specific Tregs is also shown. Results are expressed as mean fluorescence intensity (mfi).
**Figure 8** is a combination of graphs showing the CR-mediated redirected cytolytic potential of OVA-specific Tregs. The percentage of CD19⁺ Daudi cell death (**A)** and the percentage of CD 19- Jurkat cell death (**B**) was evaluated after 4 hours of co-culture with untransduced or transduced OVA-specific Tregs at a 5:1 effector:target ratio. As a control, both the percentage of CD19⁺ Daudi cell death (**A**) and the percentage of CD19- Jurkat cell death (**B**) were also assessed after co-culture with untransduced or transduced CD3 T cells. The percentage of Daudi cell death and Jurkat cell death was also assessed among Daudi cells and Jurkat cells not co-cultured with OVA-specific Tregs (target alone).

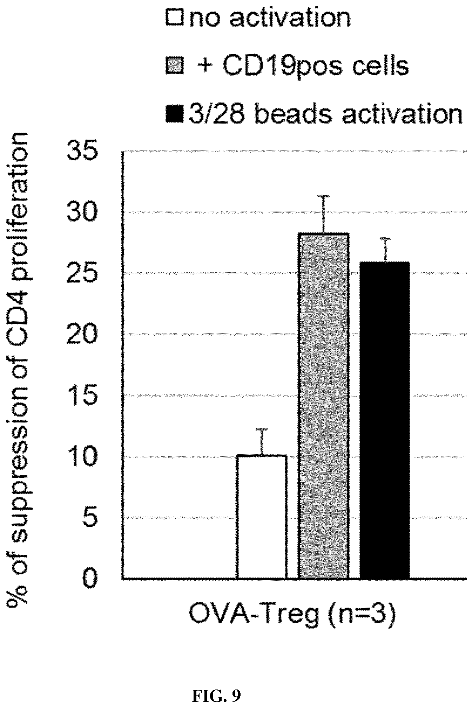

**Figure 9** is a graph showing the CR-mediated triggering of OVA-specific Tregs contact dependent suppression of CD4 T cell proliferation. Proliferation of CD4 T cells was assessed after co-culture with inactivated transduced OVA-specific Tregs (write bar), with transduced OVA-specific Tregs activated via the TCR (anti-CD3/anti-CD28 beads, black bar) or with transduced OVA-specific Tregs activated via the CR (CD19pos cells, grey bar).

### EXAMPLES

The present invention is further illustrated by the following examples.

### Example 1

### Material & Methods

### Cell line culture

The CD19⁺ human B-lineage (Daudi) cell line and the CD19⁻ U937 cell line were obtained from the American Type Culture Collection (ATCC) and were cultured in complete RPMI -1640 supplemented with 10% fetal bovine serum.

Jurkat-Lucia NFAT cells (InvivoGen) were derived from the human T lymphocyte-based Jurkat cell line by stable integration of an NFAT-inducible Lucia reporter construct. The Lucia gene, which encodes a secreted coelenterazine-utilizing luciferase, is driven by an ISG54 minimal promoter fused to six copies of the NFAT consensus transcriptional response element. Upon activation of NFAT (nuclear factor of activated T-cells), the Lucia gene is transcribed and the Jurkat-Lucia NFAT cells express luciferase. Jurkat-Lucia NFAT cells were maintained in growth medium (RPMI + 10% FBS) containing Zeocin (100µg/mL).

### Lentiviral transduction

The day before the transduction, fresh medium was applied to Jurkat-Lucia NFAT cells to improve cell growth. On the day of the transduction, cells were seeded in a 24-well plate at 0.5x10⁶ cells/ml per well. The lentivirus comprising the CR construct was thawed and added to the cells at different multiplicity of infection (MOI). The spinoculation was carried out at 1000g for 90 minutes at 32°C. The cells were then incubated for 48 hours (37°C, 5% CO2). The cells were aliquoted for GFP expression (to assess transduction efficacy), for protein-L staining (to assess CR expression at the cell surface), and for co-culture (to conduct the NFAT assay).

### Protein-L staining

The media was changed once. Cells were incubated with 200 nM protein-L for 30 min.

### NFAT Assay

Co-cultures were carried out by mixing the transduced Jurkat-Lucia cells with the target cells (Daudi cell line) or the control cells (U937 cell line) at a 1:1 ratio. The cells were seeded at 0.2x10⁶ cells/ml per well in a 24-well plate. The cells were then incubated for 24 hours (37°C, 5% CO2) before the expression of luciferase was assessed. 20 µL of cells were transferred in triplicate into a 96-well plate (3912 - Costar) and 50 µL of Quanti-Luc were added in each well. The plate was read in the Glomax plate reader (Promega) to detect the luciferase signal.

### Results

A series of 6 CR constructs were designed and built, either with the scFv recognizing the CD19 antigen or with the scFv recognizing the CD20 antigen (**Figure 1**): CD19 41BBZ (SEQ ID NO: 40 or 69), CD19 TAG 41BBZ (SEQ ID NO: 42 or 71), CD19 CD28Z (SEQ ID NO: 52 or 80), CD20 Ritux 41BBZ (SEQ ID NO: 54 or 82), CD20 B9E9 41BBZ (SEQ ID NO: 57 or 84), CD20 1F5 41BBZ (SEQ ID NO: 60 or 86).The scFv for CD19 antigen was taken from the murine hybridoma FMC63. The scFv for CD20 was taken either from Rituximab or from B9E9. The CR constructs were built with a CD8 leader sequence, a CD8 hinge, a CD8 transmembrane domain, a 41BB (CD137) intracellular domain or a CD28 intracellular domain, and a CD3 zeta intracellular domain. Two constructs, CD19 41BBZ and CD19 TAG 41BBZ, were built with the scFv recognizing the CD19 antigen (FMC63) and a 41BB (CD137) intracellular domain. CD19 41BBZ comprises neither cloning sites nor the STII tag as shown in **Figure 1**. CD19 TAG 41BBZ was built with a STII tag between the C terminal of the scFv and the CD8 hinge, and two enzyme sites to facilitate cloning and domain swapping. The expression and the activity data for these two CD19-CRs were comparable (see **Figure 2**), therefore CD19 TAG 41BBZ was used as the template to obtain other CR constructs, including anti-CD20 CR constructs.

Next, lentiviruses comprising each of the three CD19-CRS constructs were generated. Jurkat Lucia NFAT cells were transduced with the different lentiviruses at the indicated MOI and the transduction efficiency was evaluated by assessing the percentage of GFP positive cells and the GFP mean fluorescence intensity (MFI) 48 hours after transduction (**Figures 2A & 2B**). As shown in **Figure 2A****,** the transduction efficiency for the CD19-CRs was around 70-97%, with MOI of 1 to 10. There was an increase in GFP mean fluorescent intensity with increasing MOI, indicating that there could be an increased copy number per cell with increasing MOI (**Figure 2B**). Finally, CR expression at the surface of the cells was detected by protein-L staining (data not shown). The majority of the transduced cells (around 85-100%, depending on the MOI) expressed the CR at the cell surface.

Then, the activity of the transduced Jurkat Lucia NFAT cells was assessed through a NFAT assay (**Figure 2C**). NFAT-induced luciferase expression was detected after co-culture of the transduced cells either with CD19⁻ control cells (U937 cell line) or CD19⁺ target cells (Daudi cell line). Whereas the transduced Jurkat Lucia NFAT cells alone or in co-culture with the control U937 cells expressed little luciferase, the transduced Jurkat Lucia NFAT cells in co-culture with the target Daudi B cells strongly expressed luciferase. This result showed that the cells transduced with the CD19-CR constructs (CD19 41BBZ, CD19 TAG 41BBZ or CD19 CD28Z) all showed specific activity against the target Daudi B cells. Jurkat Lucia NFAT cells transduced with the CD19 41BBZ construct also showed a NFAT activation increasing with the MOI (see **Figure 2C**).

Two CR constructs were built for CD20 antigen, CD20 Ritux 41BBZ and CD20 B9E9 41BBZ. Lentiviruses comprising either the CD20 Ritux 41BBZ or the CD20 B9E9 41BBZ construct were generated. Jurkat Lucia NFAT cells were transduced with the different lentiviruses at the indicated MOI and the transduction efficiency was evaluated by assessing the percentage of GFP positive cells and the GFP mean fluorescence intensity (MFI) 48 hours after transduction (**Figures 3A & 3B**). As shown in **Figure 3A**, the transduction efficiency for the CD19-CRs was around 66-94%, with MOI of 1 to 10. There also was an increase in GFP mean fluorescent intensity with increasing MOI, indicating that there could be an increased copy number per cell with increasing MOI (**Figure 3B**). Finally, CR expression at the surface of the cells was detected by protein-L staining (data not shown). The majority of the transduced cells (around 86-94%) expressed the CR at the cell surface.

The activity of the Jurkat Lucia NFAT cells transduced with the CD20-CRs was similarly assessed through a NFAT assay (**Figure 3C**). NFAT-induced luciferase expression was detected after co-culture of the transduced cells either with control cells (U937 cell line) or target cells (Daudi cell line). Whereas the transduced Jurkat Lucia NFAT cells alone or in co-culture with the control U937 cells expressed little luciferase, the transduced Jurkat Lucia NFAT cells in co-culture with the target Daudi B cells strongly expressed luciferase. This result showed that the cells transduced with the CD20-CR constructs (CD20 Ritux 41BBZ or CD20 B9E9 41BBZ) all showed specific activity against the target Daudi B cells.

### Example 2

### Material & Methods

### Cell line culture

The CD19⁺ human B-lineage (Daudi) cell line and the CD19⁻ lymphoblastic cell line (Jurkat) were obtained from the American Type Culture Collection (ATCC) and were cultured in complete RPMI -1640 supplemented with 10% fetal bovine serum. Daudi cells express both CD19 and CD20 while Jurkat cells express neither CD19 nor CD20 (see **Figure 4**).

### OVA-specific Treg obtention

Peripheral blood mononuclear cells (PBMCs) were isolated by Ficoll (GE Healthcare, Uppsala, Sweden) gradient centrifugation from human blood. To expand OVA-specific Treg cells, PBMCs were cultured at 37°C and 5% CO2 in the presence of ovalbumin in X-Vivo15 supplemented with recombinant human IL-2 and recombinant human IL-4.

### Lentiviral transduction

OVA-specific Treg seeded at 0.5x10⁶ cells/ml in a 24-well plate were activated with anti-CD3/anti-CD28 coated beads for two days before transduction with lentiviral supernatant comprising the CR construct expressed and released by the lentivirus. Briefly, transduction was carried out by the addition of lentiviral supernatant at different multiplicity of infection (MOI) to each well, followed by centrifugation at 32°C, 1000 g for 90 minutes. The cells were then incubated at 37°C with 5% CO2. The efficacy of transduction (assessed by GFP expression) and the CR expression at the cell surface (assessed by protein-L staining) were checked 3 and 5 days after transduction, respectively.

### Protein-L staining

The cells were washed and resuspended in 0.2 ml of ice-cold wash buffer (PBS with 4% BSA) and incubated with 5 µg of protein-L at 4°C for 20 minutes. Cells were washed with 0.2 ml of ice-cold wash buffer three times, and then incubated (in the dark) with 1 µl of allophycocyanin conjugated streptavidin (APC streptavidin) in 0.2 ml of wash buffer. Immunofluorescence staining was analyzed on a MACQUANT (Miltenyi) using MacsQuantify software (Miltenyi).

### Phenotypic characterization of Tregs

Antibodies listed in Table 1 were used for immuno-phenotypic characterization of OVA-specific Treg cells. Briefly, 2x10⁵ OVA-specific Treg cells were washed in PBS and incubated at 4°C for 20 minutes with the specified mAb conjugates. After incubation and washing, samples were analyzed on a MACQUANT (Miltenyi) using MacsQuantify software (Miltenyi). When necessary, cells were permeabilized and fixed for intracellular staining using the BD kit.

| Table 1 | | | |
|---|---|---|---|
| Panels | Markers | Fluorochromes | Manufacturer |
| Regulatory status | CD4 | VioGreen | Miltenyi |
| | CD25 | PE | Miltenyi |
| | CD127 | APC-Vio770 | Miltenyi |
| Co-signalization | ICOS | APC-Vio770 | Miltenyi |
| | GITR | eF450 | eBioscience |
| | CTLA-4 | PECy7 | eBioscience |
| | PD-1 | PerCP-Cy5.5 | BD |
| Contact dependent functions | Granzyme A | PE | Miltenyi |
| | Granzyme B | APC | Miltenyi |
| | Perform | VioBlue | Miltenyi |
| | CD39 | PEVio770 | Miltenyi |
| Adhesion / Transmigration | CD11a | VioBlue | Miltenyi |
| | CD18 | PE | Miltenyi |
| | CD49b | PE-Vio770 | Miltenyi |
| | Integrinβ7 | APC | Miltenyi |
| Adhesion / Transmigration | PSGL-1 | APC | Miltenyi |
| | CD29 | APC-Vio770 | Miltenyi |
| | CD49d | PE | Miltenyi |

### In vitro cytolysis assay

Cytolysis was assessed by a simple non-radioactive cell killing assay that is based on FACS analysis combined with dye 450-labeled target cells. Briefly, dye450 pre-labeled target cells (5x10⁴ cells in 100 µl) were co-cultured with OVA-specific Treg cells at various Effector:Target ratios in 96-well U-bottom tissue culture plate (Costar, Cambridge, MA). After 4h-co-incubation, the percentage of dying target cells was assessed by propidium iodide (PI) staining, as propidium iodide can freely enter cells with compromised cell membranes but cannot pass through intact cell membranes.

### In vitro suppression assay

Suppression mediated by contact was assessed using co-culture of CD4 T cells pre-labeled with dye 450 and (i) unlabeled untransduced or (ii) unlabeled transduced OVA-specific Treg cells at various ratios of OVA-specific Tregs to responder cells. CD4 T cells were first stimulated with antiCD3/anti-CD28 coated beads at 1:3 ratio. Co-cultures of CD4 T cells and OVA-specific Treg cells were then incubated for 3 days at 37°C in a 5% CO2 incubator. At day 3, cells were harvested, and proliferation of CD4 T cells was assessed by flow cytometry (Miltenyi, MACSQUANT) through the determination of dye 450 dilution.

### Results

OVA-specific Tregs cells were efficiently transduced with CD19-CR. As shown in **Figure 5**, around 60% of OVA-specific Treg cells were transduced, and 74.73% of the transduced cells expressed the CR at the cell surface as revealed by protein-L staining.

However, a major issue with engineered T cells, and particularly with Tregs of a monospecific Treg cell population, is to ensure the maintenance of the desired phenotype. The expression of markers associated with OVA-specific Treg phenotype was thus analyzed in untransduced and transduced OVA-specific Tregs (**Figure 6**). All the markers tested, associated with regulatory phenotype (CD4, CD25, FoxP3, Helios, CD62L, CD127, **Figure 6A**), adhesion and transmigration (VLA-4 (CD49d/CD29), LFA1 (CD11a/CD18) CD49b, ITGβ7, PSGL-1, **Figure 6B**), co-signalization (CTLA-4, ICOS, GITR, PD-1, **Figure 6C**), cytolysis (Granzyme B, Granzyme A, Perforin, **Figure 6D**), metabolism disruption (CD39, **Figure 6D**) or chemotaxis (CCR7, **Figure 6E**) exhibited a similar expression on engineered OVA-specific Tregs as compared to unmodified OVA -specific Tregs. The transduction of the OVA-specific Tregs with the CD19-CR did not alter the phenotype of the OVA-specific Tregs thus genetically modified.

The ability of the engineered OVA-specific Treg cells to be activated via the CR was then examined. OVA-specific Tregs were stimulated with CD3/CD28 coated beads or by a CD 19 positive (CD19⁺) cell line. One day after stimulation, the status of activation of the OVA-specific Tregs was analyzed through GITR, CD69 and CD25 expression by flow cytometry (**Figure 7**). As a control, the expression of GITR, CD69 and CD25 was also analyzed in inactivated transduced OVA-specific Tregs. Following stimulation with either the CD3/CD28 coated beads or the CD19⁺ cell line, GITR, CD69 and CD25 were up-regulated to a similar level as compared to the control. GITR, CD69 and CD25 up-regulation after stimulation by a CD 19 positive cell line demonstrated that engineered OVA-specific Tregs could be activated through CR triggering.

Next, the cytolytic activity of CD19-CR transduced OVA-specific Tregs was compared to that of unmodified OVA-specific Tregs (**Figure 8**). As a control, the cytolytic activity of CD19-CR transduced CD3 T cells was also assessed and compared to that of untransduced CD3 T cells. CD19-CR transduced CD3 T cells were able to lyse CD19⁺ B cells. Similarly, whereas unmodified OVA-specific Treg cells were unable to kill B cells in co-culture conditions, CD19-CR transduced OVA-specific Treg cells could specifically lyse CD19⁺ B cells at a low Effector:Target ratio (5:1). By contrast, neither CD19-CR transduced CD3 T cells nor CD19-CR transduced OVA-specific Tregs were able to kill the control CD19- Jurkat cells. These results showed that activation of engineered OVA-specific Tregs through a CR specific for a B cell membrane antigen redirected the cytolytic potential of the OVA-specific Treg cells against B cells.

Finally, the contact dependent suppression of inactivated CD19-CR transduced OVA-specific Tregs (white bar) on CD4 T cell proliferation was compared to that of CD19-CR transduced OVA-specific Tregs activated either via the TCR (with anti-CD3/anti-CD28 beads, black bar) or via the CR (with a CD19⁺ cell line, grey bar) (**Figure 9**). Whereas inactivated OVA-specific Treg cells had little effect on the proliferation of CD4 T cells, OVA-specific Treg cells activated either via the TCR or via the CD19-CR suppressed the proliferation of CD4 T cells. This result showed that OVA-specific Treg cells transduced with a CD19-CR maintained their contact dependent suppression on CD4 T cell proliferation through the CR triggering.

## Claims

1. A monospecific Treg cell population, wherein the Treg cells of the population comprise a chimeric receptor, wherein said chimeric receptor recognizes at least one B cell surface marker on B cells and wherein said Treg cells are cytotoxic for B cells.

2. The monospecific Treg cell population according to claim 1, wherein the chimeric receptor comprises (i) an extracellular B cell surface marker binding domain, (ii) optionally an extracellular hinge domain, (iii) optionally a transmembrane domain, and (iv) an intracellular signaling domain, and optionally the chimeric receptor further comprises a tag and/or a leader sequence.

3. The monospecific Treg cell population according to any one of claims **1** to **2,** being cytotoxic for B cells in the conditions of Test A.

4. The monospecific Treg cell population according to any one of claims **1** to **3**, wherein the cytotoxicity for B cells is chimeric receptor dependent and TCR independent.

5. The monospecific Treg cell population according to any one of claims **1** to **4**, wherein the B cell is a mature activated B cell.

6. The monospecific Treg cell population according to any one of claims **1** to **5**, wherein the at least one B cell surface marker is selected from CD19, CD20, BCMA, IgM, IgA, IgG, IgE, IgD, CD1, CD5, CD21, CD22, CD23, CD24, CD25, CD27, CD30, CD38, CD40, CD78, CD80, CD138, CD319, PDL-2, CXCR3, CXCR4, CXCR5, CXCR6, Notch2, TLR4, IL-6, IL-10 and TGFβ, preferably CD 19 or CD20.

7. The monospecific Treg cell population according to any one of claims **1** to **6**, wherein the at least one B cell surface marker is CD 19 and wherein the chimeric receptor comprises an antibody directed to CD 19, or an antigen binding fragment thereof.

8. The monospecific Treg cell population according to any one of claims **1** to **6**, wherein the at least one B cell surface marker is CD20 and wherein the chimeric receptor comprises an antibody directed to CD20, or an antigen binding fragment thereof.

9. A pharmaceutical composition comprising at least one monospecific Treg cell population according to any one of claims **1** to **8** and at least one pharmaceutically acceptable excipient or carrier.

10. A medicament comprising at least one monospecific Treg cell population according to any one of claims **1** to **8**.

11. A monospecific Treg cell population according to any one of claims **1** to **8**, a pharmaceutical composition according to claim **9** or a medicament according to claim **10** for treating an autoimmune disease in a subject in need thereof.

12. The monospecific Treg cell population, pharmaceutical composition or medicament for use according to claim **11**, wherein said autoimmune disease is an autoantibody-mediated autoimmune disease.

13. The monospecific Treg cell population, pharmaceutical composition or medicament for use according to claim **11** or **12**, wherein said autoimmune disease is selected from the group comprising bullous pemphigoid, lupus (including systemic lupus erythematosus (SLE), lupus nephritis (LN), discoid lupus, lupus erythematosus profundus, Chilbrain lupus erythematosus, tumidus lupus erythematosus, severe systemic lupus erythematosus, acute cutaneous lupus, chronic cutaneous lupus), multiple sclerosis, neuromyelitis optica (NMO), autoimmune limbic encephalitis (LE), Hashimoto's disease, N-methyl-D-aspartate receptor (NMDAR) encephalitis, autoimmune hemolytic anemia, pemphigus vulgaris, myasthenia gravis, Graves' disease, idiopathic thrombocytopenic purpura, Goodpasture's syndrome, rheumatoid arthritis, celiac disease, pernicious anemia, vitiligo, scleroderma, psoriasis, ulcerative colitis (UC), Crohn's disease, Sjogren's syndrome, Wegener granulomatosis, polymyositis, dermatomyositis, primary biliary cirrhosis, antiphospholipid syndrome, mixed connective tissue disease, Miller Fisher syndrome, Guillain-Barré syndrome, acute motor axonal neuropathy, autoimmune hepatitis, dermatitis herpetiformis, Churg-Strauss disease, microscopic polyangiitis, IgA nephropathy, vasculitis caused by ANCA and other ANCA associated diseases, acute rheumatic fever, pernicious anemia, type 1 diabetes (T1D), reactive arthritis (Reiter syndrome), membranous nephropathy, chronic inflammatory demyelinating polyneuropathy, thrombotic thrombocytopenic purpura, hyperviscosity in monoclonal gammopathies, hemolytic uremic syndrome (atypical, due to antibody to factor H), Wilson disease, fulminant, Lambert-Eaton myasthenic syndrome, RBC alloimmunization in pregnancy, mushroom poisoning, acute disseminated encephalomyelitis, hemolytic uremic syndrome (atypical, due to complement factor mutations), autoimmune hemolytic anemia (life-threatening cold agglutinin disease), myeloma cast nephropathy, post-transfusion purpura, autoimmune hemolytic anemia (warm autoimmune hemolytic anemia), hypertriglyceridemic pancreatitis, thyroid storm, stiff person syndrome, Hemolytic uremic syndrome (typical diarrhea-associated), immune thrombocytopenia, ABO-incompatible solid organ transplantation (SOT), cryoglobulinemia, heparin-induced thrombocytopenia, thyroid storm, chronic inflammatory demyelinating polyradiculoneuropathy, focal segmental glomerulosclerosis and fulminant hepatic failure.

14. The monospecific Treg cell population, pharmaceutical composition or medicament for use according to any one of claims **11** to **13**, wherein said autoimmune disease is selected from the group comprising bullous pemphigoid, lupus (including systemic lupus erythematosus (SLE), lupus nephritis (LN), discoid lupus, lupus erythematosus profundus, Chilbrain lupus erythematosus, tumidus lupus erythematosus, severe systemic lupus erythematosus, acute cutaneous lupus, chronic cutaneous lupus) and multiple sclerosis.
